# EUROPEAN PATENT SPECIFICATION

(11) **EP 0 483 271 B1**
(45) Date of publication and mention of the grant of the patent: **25.08.1993**
(21) Application number: 90911838.2
(22) Date of filing: 09.07.1990
(51) Int. Cl.: C07K 5/02, A61K 37/64

(54) **PEPTIDES CONTAINING DIAMINO-GLYCOLS AS TRANSITION-STATE MIMICS**
PEPTIDE MIT DIAMINOGLYCOLEN ALS ÜBERGANGSZUSTANDMIMIK
PEPTIDES COMPRENANT DES DIAMINOGLYCOLS EN TANT QU'IMITATEURS DE L'ETAT DE TRANSITION

(30) Priority: 19.07.1989 US 382806
(43) Date of publication of application: 06.05.1992
(73) Proprietor: THE UPJOHN COMPANY, Kalamazoo, Michigan 49001 (US)
(72) Inventor: THAISRIVONGS, Suvit, Portage, MI 49002 (US)
(74) Representative: Perry, Robert Edward
(86) International application number: US9003754
(87) International publication number: WO9101327

(56) References cited:
- EP-A- 0 230 266
- J Med Chem vol. 31, 1988, American Chemical Society, J R Luly et al.: "Renin inhibitors. Dipeptide analogues of angiotensinogen utilizing a dihydroxyethylene transitio-state mimic at the scissile bond to impart greater inhibitory potency", pages 2264-2267

## Description

The present invention provides novel compounds. More particularly, the present invention provides novel peptide analogs. The peptides of the present invention contain a diamino-glycol moiety as a novel non-cleavable transition state insert corresponding to to 10,11-position of the renin substrate (angiotensinogen). These peptides are useful as renin inhibitors and as inhibitors of retroviral proteases. Renin inhibitors are useful for the diagnosis and control of renin-dependent hypertension, congestive heart failure, renin-dependent hyperaldosterism, and other renin-dependent cardiovascular disorders. Inhibitors of retroviral proteases, such as the HIV-I protease, are useful for treating diseases caused by retroviruses, such as human acquired immunodeficiency disease syndrome (AIDS).

Renin is an endopeptidase which specifically cleaves a particular peptide bond of its substrate (angiotensinogen), of which the N-terminal sequence in equine substrate is for example: 
Renin
↓
Asp-Arg-Val-Tyr-Ile-His-Pro-Phe-His-Leu-Leu-Val-Tyr-Ser- IA
1 2 3 4 5 6 7 8 9 10 11 12 13 14
as found by L.T. Skeggs, et al., J. Exper. Med. 106, 439 (1957). Human renin substrate has a different sequence as recently discovered by D.A. Tewkesbury, et al., Biochem. Biophys. Res. Comm. 99, 1311 (1981). It may be represented as follows: 
Renin
↓
-Val-Ile-His-
11 12 13 IB
and having the sequence to the left of the arrow (↓) being as designated in formula IA above.

Renin cleaves angiotensinogen to produce angiotensin I, which is converted to the potent pressor angiotensin II. A number of angiotensin I converting enzyme inhibitors are known to be useful in the treatment of hypertension. Inhibitors of renin are also useful in the treatment of hypertension.

A number of renin-inhibitory peptides have been disclosed. Thus, U.S. patent 4,424,207; European published applications 45,665; 104,041; and 156,322; and U.S. patent application, Serial No. 825,250, filed 3 February 1986; disclose certain peptides with the dipeptide at the 10,11-position containing an isostere bond. A number of statine derivatives stated to be renin inhibitors haves been disclosed, see, e.g., European published applications 77,028; 81,783; 114,993; 156,319; and 156,321; and U.S. patents 4,478,826; 4,470,971; 4,479,941; and 4,485,099. Terminal disulfide cycles have also been disclosed in renin inhibiting peptides; see, e.g. U.S. patents 4,477,440 and 4,477,441. Aromatic and aliphatic amino acid residues at the 10,11 position of the renin substrate are disclosed in U.S. patents 4,478,827 and 4,455,303. C-terminal amide cycles are disclosed in U.S. patent 4,485,099 and European pusblished applications 156,320 and 156,318. Certain tetrapeptides are disclosed in European publications 111,266 and 77,027. Further, European published application No. 118,223 discloses certain renin inhibiting peptide analogs where the 10-11 peptide link is replaced by a one to four atom carbon or carbon-nitrogen link. Additionally, Holladay, et al., in "Synthesis of Hydroxyethylene and Ketomethylene Dipeptide Isosteres", Tetrahedron Letters, Vol. 24, No. 41, pp. 4401-4404, 1983 discloses various intermediates in an process to prepare stereo-directed "ketomethylene" and "hydroxyethylene" dipeptide isosteric functional groups disclosed in the above U.S. Patent No. 4,424,207.

Additionally, published European Applications 45,161 and 53,017 disclose amide derivatives useful as inhibitors of angiotensin converting enzymes.

Certain dipeptide and tripeptides are disclosed in U.S. patents 4,514,332; 4,510,085; and 4,548,926 as well as in European published applications 128,762; 152,255; and 181,110. Pepstatin derived renin inhibitors have been disclosed in U.S. patents 4,481,192. Retro-inverso bond modifications at positions 10-11 have been disclosed in U.S. patents 4,560,505 and in European published applications 127,234 and 127,235. Derivatives of isosteric bond replacements at positions 10-11 have been disclosed in European published applications 143,746 and 144,209; and U.S. patent application, Serial No. 833,993, filed 27 February 1986. Isosteric bond modifications at positions 11-12 and 12-13 have been disclosed in European published application 179,352. Certain peptides containing 2-substituted statine analogues have been disclosed in European published applications 157,409. Certain peptides containing 3-aminodeoxystatine have been disclosed in European published application 161,588. Certain peptides containing 1-amino-2-hydroxybutane derivatives at positions 10-11 have been disclosed in European published application 172,346. Certain peptides containing 1-amino-2-hydroxypropane derivatives at positions 10-11 have been disclosed in European published application 172,347. Certain peptides containing N-terminal amide cycles have been disclosed in U.S. patent application, Serial No. 844,716, filed 27 March 1986. Certain peptides containing dihalostatine have been disclosed in PCT application, Serial No. 000,713, filed 7 April 1986.

European published applications 156,322; 114,993; and 118,223; and U.S. patent application, Serial No. 798,459, filed 15 November 1985; U.S. patent application, Serial No. 825,250, filed 3 February 1986; U.S. patent application, Serial No. 833,993, filed 27 February 1986; and U.S. patent application, Serial No. 844,716, filed 27 March 1986; disclose hydroxamic acids or esters at the C-terminus.

### INFORMATION DISCLOSURE

European patent application 189,203, published 30 July 1986, and European patent application 230,266, published 24 July 1987, disclose N-dihydroxyalkyl peptide derivatives which are useful as inhibitors of renin for treating hypertension. They specifically disclose the intermediate compound, (2S,3R,4S)-1-Amino-2,3-dihydroxy-4-tert-butoxy-carbonylamino-5-cyclohexylpentane, and the final compound Boc-Phe-His Amide of (2S,3R,4S)-1-(3-Methylbutylcarbonylamino)-2,3-dihydroxy-4-amino-5-cyclohexylpentane.

European patent application 184,855, published 18 June 1986, discloses new hydroxy carbonyl substituted-statine peptide derivatives which are useful as inhibitors of renin for treating hypertension.

Derivatives of isosteric bond replacements at positions 10,11 as dihydroxy ethylene isosteres are disclosed in International Patent Applications, PCT/US87/00291, published 11 September 1987 (Publication No. WO87/05302).

International Patent Application, PCT/US 87/03007, published 30 June 1988 (Publication No. WO88/04664) discloses renin inhibitory peptides having an epoxide or glycol moiety at the 10,11-position. Derwent Abstracts, Accession Nos. 89-101394, 89-101395, 89-101396 and 89-101397, disclose compounds having an amino:diol alkyl moiety as the transition state insert which are useful as renin inhibitors in the treatment of hypertension.

### SUMMARY OF THE INVENTION

The present invention particularly provides:

As peptide having a non-cleavable transition state insert corresponding to the 10,11-position of a renin substrate (angiotensinogen) of the formula XL₈, wherein X₁ is
(a) hydrogen,
(b) Y₁-(CH₂)ₐ-C(O)-,
(c) (HOCH₂)₃C-NH-C(O)-,
(d) (HOCH₂)₂CH-NH-C(O)-,
(e) the moiety of formula II,
(f) HOH₂C(CHOH)ₐCH(CH₂OH)-NH-C(O)-,
(g) NaO₃S(CH₂)₂N(CH₃)C(O)(CH₂)₆C(O)-,
(h) R₂C(O)-,
(i) R₂-O-C(O)- or
(j) Z₁-C(O)-;

wherein Y₁ is
(a) -NH₂,
(b) -CO₂H,
(c) -C(NH₂)(CH₃)₂,
(d) -CH(NH₂)(CO₂H), or
(e) -OP(O)(OH)₂;

wherein Z₁ is -Het bonded through a nitrogen;
wherein R₁ is
(a) hydrogen,
(b) C₁-C₈ alkyl,
(c) aryl,
(d) C₃-C₇ cycloalkyl,
(e) Het,
(f) C₁-C₃ alkoxy, or
(g) C₁-C₃ alkylthio;

wherein R₂ is
(a) C₁-C₁₀ alkyl,
(b) C₃-C₇ cycloalkyl,
(c) aryl,
(d) Het,
(e) aryl C₁-C₅ alkyl, or
(f) Het C₁-C₅ alkyl;

wherein R₃ is
(a) C₁-C₁₀ alkyl,
(b) -(CH₂)ₚ-aryl,
(c) -(CH₂)ₚ-Het,
(d) -(CH₂)ₚ-C₃-C₇ cycloalkyl, or
(e) -1- or 2-adamantyl;

wherein n is 0 to five inclusive;
wherein p is 0 to two inclusive;
wherein aryl is phenyl or naphthyl substituted by zero to three of the following:
(a) C₁-C₃ alkyl,
(b) hydroxy,
(c) C₁-C₃ alkoxy,
(d) halo,
(e) amino,
(f) mono- or di-C₁-C₃ alkylamino,
(g) -CHO,
(h) -COOH,
(i) -CONH₂,
(j) -CONH(C₁-C₅ alkyl),
(k) -nitro,
(l) mercapto,
(m) C₁-C₃ alkylthio,
(n) C₁-C₃ alkylsulfinyl,
(o) C₁-C₃ alkylsulfonyl,
(p) -N(R₆)-C₁-C₃ alkylsulfonyl,
(q) SO₃H,
(r) SO₂NH₂,
(s) -CN, or
(t) -CH₂NH₂;

wherein -Het is a 5- or 6-membered saturated or unsaturated ring containing from one to three heteroatoms selected from the group consisting of nitrogen, oxygen, and sulfur; and including any bicyclic group in which any of the above heterocyclic rings is rused to a benzene ring, which heterocyclic moiety is substituted with zero to three of the following:
(i) C₁-C₆ alkyl,
(ii) hydroxy,
(iii) trifluoromethyl,
(iv) C₁-C₄ alkoxy,
(v) halo,
(vi) aryl,
(vii) aryl C₁-C₄ alkyl-,
(viii) amino,
(ix) mono- or di-(C₁-C₄ alkyl) amino, or
(x) C₁-C₅ alkanoyl;

wherein R₆ is
(a) hydrogen,
(b) C₁-C₅ alkyl,
(c) -(CH₂)ₚ-aryl, or
(d) -(CH₂)ₚ-Het.

These compounds are shown in relation to the human renin substrate as follows:

6 7 8 9 10 11 12 13
-His Pro Phe His Leu Val Ile His-
X A6 B7 C8 D9 E10 F11 G12 H13 I14 Z,

The present invention provides peptide inhibitors of renin and retroviral proteases which contain a diamino-glycol moiety as a novel non-cleavable transition state insert corresponding to the 10,11-position of the renin substrate (angiotensinogen).

By "renin inhibitory peptide" is meant a compound capable of inhibiting the renin enzyme in mammalian metabolism and having three or more amino acid residues linked by peptide or pseudo-peptidic bonds.

By "a non-cleavable transition state insert" is meant a transition state insert which is not cleavable by a hydrolytic enzyme in mammalian metabolism. A variety of such transition state inserts, corresponding to the 10,11-position of the renin substrate, are known in the art, including those disclosed in the following references, which are hereby incorporated by reference:

US-A-4,424,207; EP-A-0 104041; EP-A-0 144,290; EP-A-0,156,322; EP-A-0 161588; EP-A-0 172,347; EP-A-0 172346; EP-A-156318; EP-A-157409; EP-A-0 152255; US-A-4,548,926; WO-A-8705302 (US. patent application, Serial No. 904,149, filed 5 September 1986); WO-A-8705909 (U.S. patent application, Serial No. 844,716 filed 27 March 1986); WO-A-8606379 (PCT application, Serial No. 000,713, filed 7 April 1986); WO-8804664 (U.S. patent application, Serial No. 945,340, filed 22 December 1986); EP-A-0173481 (U.S. patent application, Serial No. 825,250, filed 3 February 1986); A. Spalenstein, P. Carpino, F. Miyake and P.B. Hyskins, Tetrahedron Letters, 27:2095 (1986); D.H. Rich and M.S. Bernatowicz, J. Med. Chem., 25:791 (1982); Roger, J. Med. Chem., 28:1062 (1985); D.M. Glick, et al., Biochemistry, 21:3746 (1982); D.H. Rich, Biochemistry, 24:3165 (1985); R.L. Johnson, J. Med. Chem., 25:605 (1982); R.L. Johnson and K. Verschovor, J. Med. Chem., 26:1457 (1983); R.L. Johnson, J. Med. Chem., 27:1351 (1984); P.A. Bartlett, et al., J. Am. Chem. Soc., 106:4282 (1984); and Peptides: Synthesis, Structure and Function (V.J. Hruby; D.H. Rich, eds.) Proc. 8th American Peptide Sym., Pierce Chemical Company, Rockford, Ill., pp. 511-20; 587-590 (1983).

By "derivatives" of amino acids is meant the well known amino acid derivatives commonly employed in renin inhibitors as set forth in the references above.

Examples of the peptides of the present invention are represented by formula I. In formula I, the non-cleavable transition state insert, corresponding to the 10,11-position of the renin substrate, is designated E₁₀.

The present invention provides novel peptides having a diamino-glycol as a novel non-cleavable transition state insert.

As is apparent to those of ordinary skill in the art, the renin inhibitory peptides of the present invention can occur in several diastereomeric forms, depending on the configuration around the asymmetric carbon atoms. All such diastereomeric forms are included within the scope of the present invention. Preferably, the stereochemistry of the amino acids corresponds to that of the naturally-occuring amino acids.

Renin inhibitory peptides commonly have protecting groups at the N-terminus and the C-terminus. These protecting groups are known in the polypeptide art. Examples of these protecting groups are given below. Any of these protecting groups are suitable for the renin inhibitory peptides of the present invention.

Examples of pharmaceutically acceptable acid addition salts include; acetate, adipate, alginate, aspartate, benzoate, benzenesulfonate, bisulfate, butyrate, citrate, camphorate, camphorsulfonate, cyclopentanepropionate, digluconate, dodecylsulfate, ethane-sulfonate, fumarate, glucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalensulfonate, nicotinate, oxalate, palmoate, pectinate, persulfate, 3-phenylpropionate, picrate, pivalate, propionate, succinate, tartrate, thiocyanate, tosylate, and undecanoate.

The carbon atom content of various hydrocarbon-containing moieties is indicated by a prefix designating the minimum and maximum number of carbon atoms in the moiety, i.e., the prefix (Cᵢ-Cⱼ) indicates a moiety of the integer "i" to the integer "j" carbon atoms, inclusive. Thus (C₁-C₄)alkyl refers to alkyl of one to 4 carbon atoms, inclusive, or methyl, ethyl, propyl, butyl, and isomeric forms thereof. C₄-C₇cyclic amino indicates a monocyclic group containing one nitrogen and 4 to 7 carbon atoms.

Examples of (C₃-C₁₀)cycloalkyl, which include alkyl-substituted cycloalkyl containing a total of up to 10 total carbon atoms, are cyclopropyl, 2-methylcyclopropyl, 2,2-dimethylcyclopropyl, 2,3-diethylcyclopropyl, 2-butylcyclopropyl, cyclobutyl, 2-methylcyclobutyl, 3-propylcyclobutyl, cyclopentyl, 2,2-dimethylcyclopentyl, cyclohexyl, cycloheptyl, cyclooctyl, cyclononyl, cyclodecyl and isomeric forms thereof.

Examples of aryl include phenyl, naphthyl, (o-, m-, or p-)tolyl, (o-, m-, or p-)ethylphenyl, 2-ethyl-tolyl, 4-ethyl-o-tolyl, 5-ethyl-m-tolyl, (o-, m-, or p-)propylphenyl, 2-propyl-(o-, m-, or p-)tolyl, 4-isopropyl-2,6-xylyl, 3-propyl-4-ethylphenyl, (2,3,4-2,3,6-, or 2,4,5-)trimethylphenyl, (o-, m-, or p-)fluorophenyl, (o-, m-, or p-trifluoromethyl)phenyl, 4-fluoro-2,5-xylyl, (2,4-, 2,5- 2,6-, 3,4-, or 3,5-)difluorophenyl, (o-, m-, or p-)chlorophenyl, 2-chloro-p-tolyl, (3-, 4-, 5- or 6-)chloro-o-tolyl, 4-chloro-2-propylphenyl, 2-isopropyl-4-chlorophenyl, 4-chloro-3-fluorophenyl, (3- or 4-)chloro-2-fluorophenyl, (o-, m-, or p-)trifluoro-methylphenyl, (o-, m-, or p-)ethoxyphenyl, (4- or 5-)chloro-2-methoxy-phenyl, and 2,4-dichloro(5- or 6-)methylphenyl, and the like.

Examples of -Het include: 2-, 3-, or 4-pyridyl, imidazolyl, indolyl, N^{m}-formylindolyl, N^{m}-C₁-C₅alkyl-C(0)-indolyl, 1,2,4-triazolyl, 2-, 4-, or 5-pyrimidinyl, 2- or 3-thienyl, piperidinyl, pyrryl, pyrrolinyl, pyrrolidinyl, pyrazolyl, pyrazolinyl, pyrazolidinyl, imidazolinyl, imidazolidinyl, pyrazinyl, piperazinyl, pyridazinyl, oxazolyl, oxazolidinyl, isoxazolyl, isoxazolidinyl, morpholinyl, thiazolyl, thiazolidinyl, isothiazolyl, isothiazolidinyl, quinolinyl, isoquinolinyl, benzimidazolyl, benzothiazolyl, benzoxazolyl, furyl, thienyl, and benzothienyl. Each of these moieties may be substituted as noted above.

As would be generally recognized by those skilled in the art of organic chemistry, a heterocycle as defined herein for -Het would not be bonded through oxygen or sulfur or through nitrogen which is within a ring and part of a double bond.

Halo is halogen (fluoro, chloro, bromo, or iodo) or trifluoromethyl.

Examples of pharmaceutically acceptable cations include: pharmacologically acceptable metal cations, ammonium, amine cations, or quaternary ammonium cations. Especially preferred metal cations are those derived from the alkali metals, e.g., lithium, sodium, and potassium, and from the alkaline earth metals, e.g., magnesium and calcium, although cationic forms of other metals, e.g., aluminum, zinc, and iron are also within the scope of this invention. Pharmacologically acceptable amine cations are those derived from primary, secondary, or tertiary amines.

The novel peptides herein contain both natural and synthetic amino acid residues. These residues are depicted using standard amino acid abbreviations (see, e.g., IUPAC-IUB Joint Commission on Biochemical Nomenclature (JCBN), "Nomenclature and Symbolism for Amino Acids and Peptides", Eur. J. Biochem. 138:9-37 (1984) unless otherwise indicated.

The peptides of this invention are useful for treating any medical condition for which it is beneficial to reduce the levels of active circulating renin. Examples of such conditions include renin-dependent hypertension, hypertension, hypertension under treatment with another antihypertensive and/or a diuretic agent, congestive heart failure, renin-dependent hyperaldosterism, angina, post-myocardial infarction, other renin-dependent cardiovascular disorders and ocular disorders. The renin-angiotension system may play a role in maintenance of intracellular homeostatis: see Clinical and Experimental Hypertension, 86, 1739-1742 (1984) at page 1740 under Discussion.

The peptides of the present invention are preferably orally administered to humans to effect renin inhibition for the purpose of favorably affecting blood pressure. For this purpose, the compounds are administered from 0.1 mg to 100 mg per kg per dose, administered from 1 to 4 times daily. Equivalent dosages for other routes of administration are also employed. For example, renin-associated hypertension and hyperaldosteronism are effectively treated by the administration of from 0.5 to 50 milligrams of the compound per kilogram of body weight per day. The exact dose depends on the age, weight, and condition of the patient and on the frequency and route of administration. Such variations are within the skill of the practitioner or can readily be determined.

The peptides of the present invention to effect renin inhibition may be in the form of pharmaceutically acceptable salts both those which can be produced from the free bases by methods well known in the art and those with which acids have pharmacologically acceptable conjugate bases.

Conventional forms and means for administering renin-inhibiting compounds may be employed and are described, e.g., in U.S. Patent No. 4,424,207 which is incorporated by reference herein. Likewise, the amounts disclosed in the U.S. Patent No. 4,424,207 are examples applicable to the compounds of the present invention.

The peptides of the present invention to effect renin inhibition are preferably orally administered in the form of pharmacologically acceptable acid addition salts. Preferred pharmacologically acceptable salts for oral administration include the citrate and aspartate salts, although any pharmacologically acceptable salt is useful in this invention, including those listed above. These salts may be in hydrated or solvated form.

For renin inhibition, the peptides of the present invention may be administered topically, parenterally, by inhalation spray, or rectally in dosage unit formulations containing conventional non-toxic pharmaceutically acceptable carriers, adjuvants are vehicles. The term parenteral as used herein includes subcutaneous injections, intravenous, intramuscular, intrasternal injection or infusion techniques. In addition to the treatment of warm-blooded animals such as mice, rats, horses, dogs, cats, etc., the compounds of the invention are effective in the treatment of humans.

The pharmaceutical compositions of the peptides of the present invention for renin inhibition may be in the form of a sterile injectable preparation, for example as a sterile injectable aqueous or oleagenous suspension. This suspension may be formulated according to the known art using suitable dispersing or wetting agents and suspending agents. The sterile injectable preparation may also be a sterile injectable solution or suspension in a non-toxic parenterally-acceptable diluent or solvent, for example as a solution in 1,3-butanediol. Among the acceptable vehicles and solvents that may be employed are water, Ringer's solution and isotonic sodium chloride solution. In addition, sterile, fixed oils, are conventionally employed as a solvent or suspending medium. For this purpose any bland fixed oil may be employed including synthetic mono- or digly-cerides. In addition, fatty acids such as oleic acid find use in the preparation of injectables.

The peptides of this invention may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials are cocoa butter and polyethylene glycols.

The peptides of this invention may be administered in combination with other agents used in antihypertensive therapy such as diuretics, α and/or β-adrenergic blocking agents, CNS-acting agents, adrenergic neuron blocking agents, vasodilators, angiotensin I converting enzyme inhibitors, and the like as described for example in published European patent application 156,318.

The present invention is also directed to combinations of the nobel renin-inhibitory peptides of Formula I with one or more antihypertensive agents selected from the group consisting of diuretics, α and/or β-adrenergic blocking agents, CNS-acting agents, adrenergic neuron blocking agents, vasodilators, angiotensin I converting enzyme inhibitors, and other antihypertensive agents.

For example, the compounds of this invention can be given in combination with such compounds or salt or other derivative forms thereof as:
Diuretics: acetazolamide; amiloride; bendroflumethiazide; benzthia-zide; bumetanide; chlorothiazide; chlorthalidone; cyclothiazide; ethacrynic acid; furosemide; hydrochlorothiazide; hydroflumethiazide; indacrinone (racemic mixture, or as either the (+) or (-) enantiomer alone, or a manipulated ratio, e.g., 9:1 of said enatiomers, respecitvely); metolazone; methyclothiazide; muzolimine; polythiazide; quinethazone; sodium ethacrynate; sodium nitroprusside; spironolactone; ticrynaten; trimaterene; trichlormethiazide;
α-Adrenergic Blocking Agents: dibenamine; phentolamine; phenoxybenzamine; prazosin; tolazoline;
β-Adrenergic Blocking Agents: atenotol; metoprolol; nadolol; propranolol; timolol;
((±)-2-[3-(tert-butylamino)-2-hydroxypropoxy]-2-furananilide) (ancarolol);
(2-acetyl-7-(2-hydroxy-3-isopropylaminopropoxy)benzofuran HCl)(befunolol);
((±)-1-(isopropylamino)-3-(p-(2-cyclopropylmethoxyethyl)-phenoxy)-2-propranol HCl) (betaxolol);
(1-[(3,4-dimethoxyphenethyl)amino]-3-(m-tolyloxy)-2-propanol HCl)(bevantolol);
(((±)-1-(4-((2-isopropoxyethoxy)methyl)phenoxy)-3-isopropylamino-2-propanol) fumarate) (bisoprolol);
(4-(2-hydroxy-3-[4-(phenoxymethyl)-piperidino]-propoxy)-indole);
(carbazolyl-4-oxy-5,2-(2-methoxyphenoxy)-ethylamino-2-propanol);
(1-((1,1-dimethylethyl)amino)-3-((2-methyl 'H-indol-4-yl)oxy)-2-pro-panol benzoate) (bopindolol);
(1-(2-exobicyclo[2.2.1]-hept-2-ylphenoxy)-3-[(1-methylethyl)-amino]-2-propanol HCl) (bornaprolol);
(o-[2-hydroxy-3-[(2-indol-3-yl-1,1-dimethylethyl)-amino]propoxy]benzonitrile HCl) (bucindolol);
(α-[(tert.butylamino)methyl]-7-ethyl-2-benzofuranmethanol) (bufur-alol);
(3-[3-acetyl-4-[3-(tert.butylamino)-2-hydroxypropyl]-phenyl]-1,1-diethylurea HCl) (celiprolol);
((±)-2-[2-[3-[(1,1-dimethylethyl)amino]-2-hydroxypropoxy]phenoxy]-N-methylacetamide HCl) (cetamolol);
(2-benzimidazolyl-phenyl(2-isopropylaminopropanol));
((±)-3'-acetyl-4'-(2-hydroxy-3-isopropylaminopropoxy)-acetanilide HCl) (diacetolol);
(methyl-4-[2-hydroxy-3-[(1-methylethyl)aminopropoxyl]]-benzene-propanoate HCl) (esmolol);
(erythro-DL-1-(7-methylindan-4-yloxy)-3-isopropylaminobutan-2-ol);
(1-(tert.butylamino)-3-[0-(2-propynyloxy)phenoxy]-2-propanol (pargo-lol);
(1-(tert.butylamino)-3-[o-(6-hydrazino-3-pyridazinyl)phenoxy]-2-propanol diHCl) (prizidilol);
((-)-2-hydroxy-5-[(R)-1-hydroxy-2-[(R)-(1-methyl-3-phenylpropyl)-amino]ethyl]benzamide);
(4-hydroxy-9-[2-hydroxy-3-(isopropylamino)-propoxy]-7-methyl-5H-furo[3,2-g][1]-benzopyran-5-one) (iprocrolol);
((-)-5-(tert.butylamino)-2-hydroxypropoxy]-3,4-dihydro-1-(2H)-naphthalenone HCl) (levobunolol);
(4-(2-hydroxy-3-isopropylamino-propoxy)-1,2-benzisothiazole HCl);
(4-[3-(tert.butylamino)-2-hydroxypropoxy]-N-methylisocarbostyril HCl);
((±)-N-2-[4-(2-hydroxy-3-isopropylaminopropoxy)phenyl]ethyl-N'-isopropylurea) (pafenolol);
(3-[[(2-trifluoroacetamido)ethyl]amino]-1-phenoxypropan-2-ol);
(N-(3-(o-chlorophenoxy)-2-hydroxypropyl)-N'-(4'-chloro-2,3-dihydro-3-oxo-5-pyridazinyl)ethylenediamine);
((±)-N-[3-acetyl-4-[2-hydroxy-3-[(1-methylethyl)amino]propoxyphenyl]-butanamide)(acebutolol);
((±)-4'-[3-(tert-butylamino)-2-hydroxypropoxy]spiro[cyclohexane-1,2'-indan]-1'-one) (spirendolol);
(7-[3-[[2-hydroxy-3-[(2-methylindol-4-yl)oxylpropyl]amino]butyl]thio-phylline) (teoprolol);
((±)-1-tert.butylamino-3-(thiochroman-8-yloxy)-2-propanol) (tertato-lol);
((±)-1-tert.butylamino-3-(2,3-xylyloxy)-2-propanol HCl) (xibenolol);
(8-[3-(tert.butylamino)-2-hydroxypropoxy]-5-methylcoumarin) (bucumo-lol);
(2-(3-(tert.butylamino)-2-hydroxy-propoxy)benzonitrile HCl) (bunitro-lol); ((±)-2'-[3-(tert-butylamino)-2-hydroxypropoxy-5'-fluorobutyrophenone) (butofilolol);
(1-(carbazol-4-yloxy)-3-(isopropylamino)-2-propanol) (carazolol);
(5-(3-tert.butylamino-2-hydroxy)propoxy-3,4-dihydrocarbotyril HCl) (carteolol);
(1-(tert.butylamino)-3-(2,5-dichlorophenoxy)-2-propanol) (clorano-lol);
(1-(inden-4(or 7)-yloxy)-3-(isopropylamino)-2-propanol HCl) (indeno-lol);
(1-ispropylamino-3-[(2-methylindol-4-yl)oxy]-2-propanol) (mepindo-lol);
(1-(4-acetoxy-2,3,5-trimethylphenoxy)-3-isopropylaminopropan-2-ol) (metipranolol);
(1-(ispropylamino)-3-(o-methoxyphenoxy)-3-[(1-methylethyl)amino]-2-propanol) (moprolol);
((1-tert.butylamino)-3-[(5,6,7,8-tetrahydro-cis-6,7-dihydroxy-1-naphthyl)oxy]-2-propanol) (nadolol);
((S)-1-(2-cyclopentylphenoxy)-3-[(1,1-dimethylethyl)amino]-2-propanol sulfate (2:1)) (penbutolol);
(4'-[1-hydroxy-2-(amino)ethyl]methanesulfonanilide) (sotalol);
(2-methyl-3-[4-(2-hydroxy-3-tert.butylaminopropoxy)phenyl]-7-methoxy-isoquinolin-1-(2H)-one);
(1-(4-(2-(4-fluorophenyloxy)ethoxy)phenoxy)-3-isopropylamino-2-propanol HCl);
((-)-p-[3-[(3,4-dimethoxyphenethyl)amino]-2-hydroxypropoxy]-β-methyl-cinnamonitrile) (pacrinolol);
((±)-2-(3'-tert.butylamino-2'-hydroxypropylthio)-4-(5'-carbamoyl-2'-thienyl)thiazole HCl) (arotinolol);
((±)-1-[p-[2-(cyclopropylmethoxy)ethoxy]phenoxy]-3-(isopropylamino)-2-propanol) (cicloprolol);
((±)-1-[(3-chloro-2-methylindol-4-yl)oxy]-3-[(2-phenoxyethyl)amino]-2-propanol) (indopanolol);
((±)-6-[[2-[[3-(p-butoxyphenoxy)-2-hydroxypropyl]amino]ethyl]amino]-1,3-dimethyluracil) (pirepolol);
(4-(cyclohexylamino)-1(1-naphtolenyloxy)-2-butanol);
(1-phenyl-3-[2-[3-2-cyanophenoxy)-2-hydroxypropyl]aminoethyl]hydan-toin HCl);
(3,4-dihydro-8-(2-hydroxy-3-isopropylaminopropoxy)-3-nitroxy-2H-1-benzopyran) (nipradolol);
Angiotensin I Converting Enzyme Inhibitors:
1-(3-mercapto-2-methyl-1-oxopropyl-L-proline (captopril);
(1-(4-ethoxycarbonyl-2,4(R,R)-dimethylbutanoyl)indoline-2-(S)-car-boxylic acid);
(2-[2-[(1-(ethoxycarbonyl)-3-phenyl-propyl]amino]-1-oxopropyl]-1,2,3,4-tetrahydro-3-isoquinoline carboxylic acid);
((S)-1-[2-[(1-(ethoxycarbonyl)-3-phenylpropyl]amino]-1-oxopropyl]octahydro-1H-indole-2-carboxylic acid HCl);
(N-cyclopentyl-N-(3-(2,2-dimethyl-1-oxopropyl)thiol-2-methyl-1-oxo-propyl)glycine) (pivalopril);
((2R,4R)-2-(2-hydroxyphenyl)-3-(3-mercaptopropionyl)-4-thiazolidine-carboxylic acid);
(1-(N-[1(S)-ethoxycarbonyl-3-phenylpropyl]-(S)-alanyl)-cis,syn-octa-hydroindol-2(S)-carboxylic acid HCl);
((-)-(S)-1-[(S)-3-mercapto-2-methyl-1-oxopropyl]indoline-2-carboxylic acid);
([1(S),4S]-1-[3-(benzoylthio)-2-methyl-1-oxopropyl]-4-phenylthio-L-proline;
(3-([1-ethoxycarbonyl-3-phenyl-(1S)-propyl]amino)-2,3,4,5-tetrahydro-2-one-1-(3S)-benzazepine-1-acetic acid HCl);
(N-(2-benzyl-3-mercaptopropanoyl)-S-ethyl-L-cysteine) and the S-methyl analogue;
(N-(1(S)-ethoxycarbonyl-3-phenylpropyl)-L-alanyl-L-proline maleate) (enalapril);
N-[1-(S)-carboxy-3-phenylpropyl]-L-alanyl-1-proline;
N²-[1-(S)-carboxy-3-phenylpropyl]-L-lysyl-L-proline (lysinopril);
Other Antihypertensive Agents: aminophylline; cryptenamine acetates and tannates; deserpidine; meremethoxylline procaine; pargyline; tri-methaphan camsylate; and the like, as well as admixtures and combinations thereof.

Typically, the individual daily dosages for these combinations can range from about one-fifth of the minimally recommended clinical dosages to the maximum recommended levels for the entities when they are given singly. Coadministration is most readily accomplished by combining the active ingredients into a suitable unit dosage form containing the proper dosages of each. Other methods of coadministration are, of course, possible.

Thus, the novel peptide of the present invention possess an excellent degree of activity in treating renin-associated hypertension and hyperaldosteronism.

Renin inhibitors have also been disclosed to control the rise in intraocular pressure associated with the use of steroidal anti-inflammatory drugs as described in International Application PCT/US86/02291 (International Publication Number WO 87/02581 dated 7 May 1987).

The peptides of the present invention are also useful as novel human retroviral protease inhibitory peptide analogs. Therefore, the peptides of the present invention inhibit retroviral proteases and thus inhibit the replication of the virus. They are useful for treating human patients infected with a human retrovirus, such as human immunodeficiency virus (strains of HIV-1 or HIV-2) or human T-cell leukemia viruses (HTLV-I or HTLV-II) which results in acquired immunodeficiency syndrome (AIDS) and/or related diseases.

The capsid and replicative enzymes (i.e. protease, reverse transcriptase, integrase) of retroviruses are translated from the viral gag and pol genes as polyproteins that are further processed by the viral protease (PR) to the mature proteins found in the viral capsid and necessary for viral functions and replication. If the PR is absent or nonfunctional, the virus cannot replicate. The retroviral PR, such as HIV-1 PR, has been found to be an aspartic protease with active site characteristics similar to those exhibited by the more complex aspartic protease, renin.

The term human retrovirus (HRV) includes human immunodeficiency virus type I, human immunodeficiency virus type II, or strains thereof, as well as human T cell leukemia virus 1 and 2 (HTLV-1 and HTLV-2) or strains apparent to one skilled in the art, which belong to the same or related viral families and which create similar physiological effects in humans as various human retroviruses.

Patients to be treated would be those individuals: 1) infected with one or more strains of a human retrovirus as determined by the presence of either measurable viral antibody or antigen in the serum and 2) in the case of HIV, having either a symptomatic AIDS defining infection such as i) disseminated histoplasmosis, ii) isopsoriasis, iii) bronchial and pulmonary candidiasis including pneumocystic pneumonia iv) non-Hodgkin's lymphoma or v) Kaposi's sarcoma and being less than sixty years old; or having an absolute CD4 lymphocyte count of les than 200/m³ in the peripheral blood. Treatment would consist of maintaining an inhibitory level of the peptide used according to this invention in the patient at all times and would continue until the occurrence of a second symptomatic AIDS defining infection indicates alternate therapy is needed.

More specifically, an example of one such human retrovirus is the human immunodeficiency virus (HIV, also known as HTLV-III or LAV) which has been recognized as the causative agent in human acquired immunodificiency disease syndrome (AIDS), P. Duesberg, Proc. Natl. Acad. Sci. USA, 86:755 (1989). HIV contains a retro viral encoded protease, HIV-I protease, that cleaves the fusion polypeptides into the functional proteins of the mature virus particle, E.P. Lillehoj, et al., J. Virology, 62:3053 (1988); C. Debuck, *et al*., Proc. Natl. Acad. Sci., 84:8903 (1987). This enzyme, HIV-I protease, has been classified as an aspartyl protease and has a demonstrated homology to other aspartyl proteases such as renin, L.H. Pearl, *et al*., Nature 329:351 (1987); I. Katoh, *et al*., Nature 329:654 (1987). Inhibition of HIV-I protease blocks the replication of HIV and thus is useful in the treatment of human AIDS, E.D. Clerq, J. Med. Chem. 29:1561 (1986). Inhibitors of HIV-I protease are useful in the treatment of AIDS.

Pepstatin A, a general inhibitor of aspartyl proteases, has been disclosed as an inhibitor of HIV-I protease, S. Seelmeier, *et al*., Proc. Natl. Acad. Sci. USA, 85:6612 (1986). Other substrate derived inhibitors containing reduced bond isosteres or statine at the scissle position have also been disclosed, M.L. Moore, *et al*., Biochem. Biophys, Res. Commun. 159:420 (1989); S. Billich, *et al*., J. Biol. Chem. 263;17905 (1988); Sandoz, D.E. 3812-576-A.

Thus, the peptides of the present invention are useful for treating diseases caused by retroviruses, such as human acquired immunodeficiency disease syndrome (AIDS), using dosages, forms and modes of administration equivalent to those described above for renin inhibition. Exact dosages, forms and mode of administration would be apparent to one of ordinary skill in the art such as a physician or pharmacologist.

The peptides of the present invention are also useful for treating non-human animals infected with a retrovirus, such as cats infected with feline leukemia virus. Other viruses that infect cats include, for example, feline infectious peritonitis virus, calicivirus, rabies virus, feline immunodeficiency virus, feline parvovirus (panleukopenia virus), and feline chlamydia. Exact dosages, forms and modes of administration of the peptides of the present invention to non-human animals would be apparent to one of ordinary skill in the art, such as a veterinarian.

The compounds of the present invention are prepared as depicted in the charts and as described more fully in the Preparations and Examples. In these charts, the variables are as defined above.

### CHART A

Chart A describes the preparation of the novel transition state analog insert, 1-cyclohexyl-2S,6-diamino-3R,4S-dihydroxy-6-methylheptane. Thisappropriatelyprotected building block is utilized in the construction of some of the peptides of the present invention.

The preparation of the allylic alcohol of formula A-1 is described in WO-A-9002317 (U.S. patent application, Serial No. 234,413, filed 19 August 1988). This alcohol is oxidized to the corresponding enone of formula A-2 with either manganese dioxide in hexane or 2,3-dichloro-5,6-dicyano-1,4-benzoquinone in dioxane. 1,4-Addition of gaseous ammonia in ethanol leads to the amino ketone of formula A-3 which is then protected under appropriate conditions (CbzCl, PrNEt₂) on the amino group with a Z₁ group as in compound A-4 wherein Z₁ is carbobenzyloxy. The ketone is then reduced with sodium borohydride in the presence of cerium (III) chloride, as described in G. Gajewski, *et al*., Syn. Commun. 10, 623 (1980), to give an 85:15 mixture (¹³C-NMR) of epimeric alcohols of formula A-5. The acid labile protecting groups are then removed using hydrochloric acid in methanol to give the free amine of formula A-6.

### CHART B

Chart B illustrates the preparation of the novel peptides N-tert-Butyloxycarbonyl-L-phenylalanyl-L-histidyl-2S,6-diamino-1-cyclohexyl-3R,4R-dihydroxy-6-methylheptane and N-tert-Butyloxycarbonyl-L-phenylalanyl-L-histidyl-2S,6-diamino-1-cyclohexyl-3R,4S-dihydroxy-6-methylheptane of the present invention.

The epimeric mixture of formula B-1 (A-6), prepared as the compound of formula A-6 in Chart A, is coupled to Boc-His(Ts)-OH with diethylphosphoryl cyanide as the condensing agent to give the two epimeric compounds of formula B-2a, (R₁=H, R₂=OH) and B-2b (R₁=OH, R₂=H) which are separated by column chromatography on silica gel. Each epimer is then carried on through the syntheses separately. Removal of the tert-butyloxy-carbonyl group with trifluoroacetic acid and coupling of the resulting amines to Boc-Phe-OH with diethyphosphoryl cyanide as the condensing agent gives the two epimers of formula B-3a and B-3b. The tosyl protecting group is then removed with 1-hydroxybenzotriazole to give the two epimers of formula B-4a and B-4b. Finally, the benzyloxycarbonyl protecting group is hydrogenolyzed (5% Pd/C, H₂, ethanol) to give the final peptides of formula B-5a and B-5b.

### CHART C

Chart C describes the preparation of the peptide βVal-Phe-His-X of the present invention.

The peptide of formula C-1 (wherein X is 1-cyclohexyl-2S,6-diamino-3R,4S-dihydroxy-6-methylheptane; Cbz is carbobenzyloxy), prepared as the compound of formula B-3a or B-3b in Chart B, is deprotected with trifluoroacetic acid and the resulting amine is compound to Boc-β-Val-OH to give the peptide of formula C-2. The tosyl group is removed with 1-hydroxybenzotriazole to give the compound of formula C-3. The Cbz group is removed by hydrogenolysis with palladium on charcoal and ammonium formate to give the compound of formula C-4. The Boc group is removed with trifuloroacetic acid to give the desired final peptide of formula C-5, which is isolated as the tris trifluoroacetate salt.

### CHART D

Chart D describes the preparation of the peptide βVal-Phe-N-Me-His-X of the present invention.

The Boc-N-MeHis(Ts)-OH, obtained as described in US-A-4880781 (U.S. patent application, Serial No. 147,073, filed 20 January 1988), and in EP-A-0 173 481, published 5 March 1986, is coupled to the transition-state-insert building block of formula A-6, prepared as described in Chart A, with diethylphosphoryl cyanide (DEPC) to give the compound of formula D-1 (wherein X is 1-cyclohexyl-2S,6-diamino-3R,4S-dihydroxy-6-methylheptane; Cbz is carbobenzyloxy). The Boc group is removed with acid and the resulting amine is coupled to Boc-Phe-OH employing bis(2-oxo-3-oxazolidinyl)phosphinic chloride (BOPCl) to give the peptide of formula D-2. The Boc group is removed under acidic conditions and the amine is then coupled to Boc-βVal-OH to give the peptide of formula D-3. The tosyl group is removed with 1-hydroxybenzotriazole in methanol to give the compound of formula D-4. The Cbz group is removed with phase transfer hydrogenolysis to give the compound of formula D-5. Finally, the Boc group is removed with trifluoroacetic acid to give the desired final peptide of formula D-6 which is isolated as the tris trifluoroacetate salt.

### CHART E

Chart E describes the preparation of the renin inhibitory peptide γGlu-Pro-Phe-N-Me-His-X of the present invention.

Coupling of the amine from the peptide of formula E-1 (D-2), prepared as the compound of formula D-2 in Chart D, with Boc-Pro-OH gives the compound of formula E-2. The Boc group is removed with acid and the resulting amine is coupled to Boc-γGlu(Boc)-OH to give the peptide of formula E-3. The tosyl group is removed with 1-hydroxybenzotriazole to give the compound of formula E-4. The Cbz group is removed with catalytic hydrogenolysis to give the compound of formula E-5. Finally, the Boc groups are removed with trifluoroacetic acid to give the final peptide of formula E-6, which is isolated as the tris trifluoroacetate salt.

### CHART F

Chart F describes the preparation of the peptide Boc-O-MeTyr-His-X of the present invention.

Coupling of the amine from the peptide of formula F-1 (wherein X is 1-cyclohexyl-2S,6-diamino-3R,4S-dihydroxy-6-methylheptane; Cbz is carbobenzyloxy), prepared as the compound B-2a in Chart B, with Boc-O-Me-Tyr-OH, obtained from Boc protection of commercial O-Me-Tyr-OH, gives the compound of formula F-2. The tosyl group is removed with 1-hydroxybenzotriazole to give the compound of formula F-3. The Cbz group is removed with catalytic hydrogenolysis to give the final peptide of formula F-4.

### CHART G

Chart G describes the preparation of the peptide γGlu-Pro-O-MeTyr-His-X of the present invention.

The compound of formula G-1 (F-2) is deprotected with trifluoroacetic acid. The resulting amine is coupled with Boc-Pro-OH using diethylphosphoryl cyanide to give the compound of formula G-2. The Boc group is removed with trifluoroacetic acid and the resulting amine is then coupled to Boc-γGlu(Boc)-OH using diethylphosphoryl cyanide to give the compound of formula G-3. The tosyl group is removed with 1-hydroxybenzotriazole to give the compound of formula G-4. The Cbz group is hydrogenolyzed to give the compound of Formula G-5. The two Boc groups are removed with trifluoroacetic acid to give the compound of formula G-6 which is isolated as the tris(trifluoroacetate) salt.

### CHART H

Chart H describes the preparation of the peptide Glc-Pro-O-MeTyr-His-X of the present invention.

The compound of formula H-1 (F-2) is deprotected with trifluoroacetic acid. The resulting amine is coupled with the compound of formula I-4 using diethylphosphoryl cyanide to give the compound of formula H-2. The acetyl and the tosyl groups are removed with methanolic ammonia to give the compound of formula H-3. The Cbz group is hydrogenolyzed to give the compound of formula H-4.

### CHART I

Chart I describes the preparation of the compound of the formula I-4, abbreviated as Glc(per Ac)-Pro-OH, which is used in the preparation of the intermediate compound of formula H-2 in Chart H.

The compound of the formula I-1, prepared as described in WO-A-8907109 (International Patent Application, PCT/US 89/00247, filed 27 January 1989), is reacted with proline benzyl ester hydrochloride of formula I-2, which is commercially available, to give the compound of formula I-3. Catalytic hydrogenolysis removes the benzyl ester protecting group to give the acid of formula I-4.

Generally, the renin inhibiting polypeptides may be prepared by solution phase peptide synthetic procedures analogous to those described hereinafter or to those methods known in the art. Appropriate protecting groups, reagents, and solvents for the solution phase method can be found in "The Peptides: Analysis, Synthesis, and Biology," Vols. 1-5, eds. E. Gross and T. Meienhofer, Academic Press, NY, 1979-1983; "The Practice of Peptide Synthesis", M. Bodansky and A. Bodansky, Springer-Verlag, New York, 1984; "The Principles of Peptide Synthesis", M. Bodansky, Springer-Verlag, New York, 1984. Thus, for example, the carboxylic moiety of N^{α}-t-butyloxycarbonyl (BOC)-substituted amino acid derivatives having suitable side chain protecting groups, if necessary, may be condensed with the amino functionality of a suitably protected amino acid or peptide using a conventional coupling protocol such as dicyclohexylcarbodiimide (DCC) and 1-hydroxybenzotriazole (HOBT) or diethylphosphoryl cyanide (DEPC) and triethylamine (Et₃N) in methylene chloride or dimethylformamide.

Following coupling reaction completion, the N^{α}-BOC moiety may be selectively removed with 50% trifluoroacetic acid with or without 2% anisole (v/v) in methylene chloride. Neutralization of the resultant trifluoroacetate salt may be accomplished with 10% diisopropylethylamine or sodium bicarbonate in methylene chloride.

Variations in the above description for starting materials, reactants, reaction conditions and required protecting groups to obtain other such N-alkylated compounds are known to an ordinarily skilled chemist or are readily available in the literature.

The compounds of the present invention may be in either free form or in protected form at one or more of the remaining (not previously protected) peptide, carboxyl, amino, hydroxy, or other reactive groups. The protecting groups may be any of those known in the polypeptide art. Examples of nitrogen and oxygen protection groups are set forth in T.W. Greene, Protecting Groups in Organic Synthesis, Wiley, New York, (1981); J.F.W. McOmie, ed. Protective Groups in Organic Chemistry, Plenum Press (1973); and J. Fuhrhop and G. Benzlin, Organic Synthesis, Verlag Chemie (1983). Included among the nitrogen protective groups are t-butoxycarbonyl (BOC), benzyloxycarbonyl, acetyl, allyl, phthalyl, benzyl, benzoyl, trityl and the like.

The following compounds of the present invention are preferred:
βVal-Phe-N-Me-His-X;
γGlu-Pro-O-MeTyr-His-X;
N-tert-Butyloxycarbonyl-L-phenylalanyl-L-histidyl-2S,6-diamino-1-cyclohexyl-3R,4S-dihydroxy-6-methylheptane;
βVal-Phe-His-X;
γGlu-Pro-Phe-N-Me-His-X;
N-tert-Butyloxycarbonyl-L-phenylalanyl-L-histidyl-2S,6-diamino-1-cyclohexyl-3R,4R-dihydroxy-6-methylheptane; and
Boc-O-MeTyr-His-X.
The following compounds of the present invention are more preferred:
βVal-Phe-His-X;
γGlu-Pro-Phe-N-Me-His-X;
N-tert-Butyloxycarbonyl-L-phenylalanyl-L-histidyl-2S,6-diamino-1-cyclohexyl-3R,4R-dihydroxy-6-methylheptane; and
Boc-O-MeTyr-His-X.
The following compounds of the present invention are most preferred:
N-tert-Butyloxycarbonyl-L-phenylalanyl-L-histidyl-2S,6-diamino-1-cyclohexyl-3R,4R-dihydroxy-6-methylheptane; and
Boc-O-MeTyr-His-X.

### DESCRIPTION OF THE PREFERRED EMBODIMENTS

The following Preparations and Examples illustrate the present invention.

In the Preparations and Examples below and throughout this document:
¹H-NMR is nuclear magnetic resonance
BOC is t-butoxycarbonyl
Bz is benzyl
C is centrigade
Cbz is benzyloxycarbonyl
CDCl₃ is deuteriochloroform
Celite is a filter aid
CVA is Chaψ[CH(OH)CH₂]Val
DCC is dicyclohexylcarbodiimide
DEPC is diethylphosphoryl cyanide
Et0Ac is ethyl acetate
g is grams
γ-Glu is γ-glutamic acid
His is histidine
N-MeHis is Nα-methyl histidine
HOBT is 1-hydroxybenzotriazole
HPLC is high performance liquid chromatography
IR is infrared spectra
LVA is Leuφ(CH(0H)CH₂)Val with the S configuration at C4 (the hydroxylbearing carbon atom)
M or mol is mole
Me is methyl
min is minute
mL is milliliter
MPLC is medium pressure liquid chromatography
MS is mass spectroscopy
Ph is phenyl
Phe is phenylalanine
Pro is proline
RIP means a compound having the formula H-Pro-His-Phe-His-Phe-Phe-Val-Tyr-Lys-0H.2(CH₃C(0)0H).XH₂0 which is a known renin-inhibiting peptide
TEA is triethylamine
TFA is trifluoroacetic acid
THF is tetrahydrofuran
TLC is thin layer chromatography
Tos is p-toluenesulfonyl
Ts0H is p-toluenesulfonic acid
Tyr is tyrosine
(OCH₃)Tyr is O-methyl tyrosine
β-Val is β-Valine.

The wedge-shape line indicates a bond which extends above the plane of the paper relative to the plane of the compound thereon.

The dotted line indicates a bond which extends below the plane of the paper relative to the plane of the compound thereon.

In the examples below, the renin inhibitory activity of the compounds of the present invention (IC₅₀'s) are determined using the *in vitro* test described in US-A-4880 781 (U.S. patent application, Serial No. 07/147,073, filed 20 January 1988), and in EP-A-0 173 481, published 5 March 1986, pages 103-105, which are hereby incorporarted by reference. Compounds of the present invention have also exhibited renin-inhibitory acid during *in vivo* testing.

In the examples below, HPLC is high pressure liquid chromatography and k' is the partition ratio obtained. The solvent system used in indicated in parentheses after the partition ratio. HPLC is performed on a Perkin-Elmer Series IV liquid chromatograph operating at 1.5 mL/min through a Brownlee RP-18 10 micron column, with UV monitoring by Kratos Spectroflow 773 detector. A Perkin-Elmer LCI-100 integrator is used for peak data. Solvent A is 90% by volume Burdick & Jackson water, 10% acetonitrile, and 0.1% trifluoroacetic acid; solvent B is 10% by volume Burdick & Jackson water, 90% acetronitrile, and 0.1% trifluoroacetic acid.

### Preparation 1 1-[3-tert-Butyloxycarbonyl-4S-cyclohexyl-methyl-2,2-dimethyl-5R-oxazolidinyl]-3-methyl-1-oxo-trans-2-butene (Formula A-2) Refer to Chart A.

To a stirred solution of 5.51 g of the allylic alcohol of formula A-1 in 50 mL of hexane is added 24 g of manganese dioxide. After stirring for 2 days, the mixture is filtered through Celite with hexane and then dichloromethane washings. The filtrate is concentrated and the residue chromatographed on silica gel with 5%-10% ether in hexane to give 1.3 g of the title product. Continued elution with 15% ethyl acetate in hexane gives 1.88 g of the major, less polar allylic alcohol and 1.19 g of the minor, more polar allylic alcohol (A-1).

Alternatively, to a stirred solution of 2.0 g of the allylic alcohol of formula A-1 in 20 mL of dioxane is added 4.0 g of 2,3-dichloro-5,6-dicyano-1,4-benzoquinone. After stirring at room temperature for 4 days, the mixture is diluted with dichloromethane and then washed with aqueous sodium bicarbonate. The aqueous phase is extracted with several portions of dichloromethane and the organic phase is then dried (magnesium sulfate) and concentrated. The residue is chromatographed on silica gel with 10% ethyl acetate in hexane to give 1.076 g of the title product.

Physical characteristics are as follows:
¹H NMR (CDCl₃): δ 1.34, 1.36, 1.45, 1.83, 2.07, 4.0, 4.3, 6.4. IR (mull, cm⁻¹): 1702, 1625.
[α]_{D} = -8° (C=1.011, chloroform).
HRMS: Found 379.2749.
Anal.: Found C, 69.59; H, 9.36; N, 3.74.

### Preparation 2 3-Amino-1-[3-tert-butyloxycarbonyl-4S-cyclohexyl-methyl-2,2-dimethyl-5R-oxazolidinyl]-3-methyl-1-oxo-butane (Formula A-3) Refer to Chart A.

To a solution of 1.18 g of the title product of Preparation 1 in 10 mL of absolute ethanol at -15°C is passed gaseous ammonia until saturated. The container is sealed and the content allowed to warm to room temperature. After a few hours, the container is recooled to -15°C and a stream of nitrogen is passed through the content while the container is allowed to warm to room temperature. The content is concentrated and the residue flash-chromatographed on silica gel with 3% methanol (saturated with ammonia) in dichloromethane to give 1.24 g of the title product.

Physical characteristics are as follows:
¹H NMR (CDCl₃): δ 1.20, 1.21, 1.47, 1.48, 1.52, 2.68, 2.80, 4.09, 4.35.

### Preparation 3 3-Benzyloxycarbonylamino-1-[3-tert-butyloxycarbonyl-4S-cyclohexyl-methyl-2,2-dimethyl-5R-oxazolidinyl]-3-methyl-1-oxo-butane (Formula A-4: Z₁ is benzyloxycarbonyl) Refer to Chart A.

To a stirred solution of 1.11 g of the title product of Preparation 2 in 30 mL of dichloromethane is added 0.7 mL of diisopropylethylamine and then 0.55 mL of benzylchloroformate. After stirring at room temperature for 3 hours, the mixture is diluted with dichloromethane and then washed successively with aqueous citric acid, saturated aqueous sodium bicarbonate and saturated aqueous sodium chloride. The organic phase is dried (magnesium sulfate) and then concentrated. The residue is chromatographed on silica gel with 10%-20% ethyl acetate in hexane to give 1.24 g of the title product.

Physical characteristics are as follows:
¹H NMR (CDCl₃): δ 1.40, 1.42, 1.46, 1.51, 1.57, 3.1, 4.07, 4.32, 5.03, 7.34.
IR (neat, cm⁻¹): 1724, 1701.
[d]_{D}=+8° (C=0.391, chloroform).
HRMS: Found 531.3436.
Anal.: Found C, 67.45; H, 8.19; N, 5.67.

### Preparation 4 3-Benzyloxycarbonylamino-1-[3-tert-butyloxycarbonyl-4S-cyclohexylmethyl-2,2-dimethyl-5R-oxazolidinyl]-3-methyl-1R and 1S-butanol (Formula A-5: Z₁ is benzyloxycarbonyl) Refer to Chart A.

To a stirred solution of 712 mg of the title product of Preparation 3 and 550 mg of cerium (III) chloride heptahydrate in 15 mL of methanol at 0°C under argon is added 80 mg of sodium borohydride. After 1.5 hours, 10 mL of water is added and the mixture is partitioned between water and diethylether. The organic phase is dried (magnesium sulfate) and then concentrated. The residue is chromatographed on silica gel with 15% ethyl acetate in hexane to give 670 mg of the title product.

Physical characteristics are as follows:
¹H NMR (CDCl₃): δ 1.37, 1.42, 1.48, 1.51, 1.54, 3.55, 3.79, 4.10, 5.03, 7.33.
IR (mull, cm⁻¹): 3364, 1728, 1701, 1671.
HRMS: Found 533.3599.
Anal.: C, 67.69; H, 9.12; N, 5.25.

### Preparation 5 2S-Amino-6-benzyloxycarbonylamino-1-cyclohexyl-3R,4R and 4S-dihydroxy-6-methylheptane (Formula A-6: Z₁ is benzyloxycarbonyl) Refer to Chart A.

A solution of 0.14 mL of acetyl chloride in 2 mL of methanol is allowed to stir for 15 minutes; then 120 mg of the title product of Preparation 4 is dissolved and allowed to stir at room temperature. After 4 hours, 350 mg of solid sodium bicarbonate is slowly added. After stirring for 30 minutes, the mixture is filtered with dichloromethane washings. The concentrated residue is triturated with ether and then filtered through Celite. The filtrate is concentrated to give 92 mg of the title product.

Physical characteristics are as follows:
¹H NMR (CDCl₃): δ 1.37, 5.03, 7.32.

### Preparation 6 N-tert-Butyloxycarbonyl-N^{im}-tosyl-L-histidyl-2S-amino-6-benzyloxycarbonylamino-1-cyclohexyl-3R,4R and 4S-dihydroxy-6-methylheptane (Formula B-2a and B-2b: Z₁ is Cbz) Refer to Chart B.

To a stirred solution of 92 mg of the title product of Preparation 5 and 100 mg of N-tert-butyloxycarbonyl-N^{im}-tosyl-L-histidine in 2 mL of dichloromethane is added 0.07 mL of diisopropyldiethylamine and 0.04 mL of diethylphosphoryl cyanide. After stirring overnight, the concentrated mixture is chromatographed on silica gel with 30%-50% ethyl acetate in dichloromethane to give 47.8 mg of the less polar title product of formula B-2a and 35 mg of the more polar title product of formula B-2b.

Physical characteristics are as follows:
FAB-HRMS; Found 784.3963.
¹H NMR (CDCl₃) is consistent with structure.

### Preparation 7 N-tert-Butyloxycarbonyl-L-phenylalanyl-N^{im}-tosyl-L-histidyl-2S-amino-6-benzyloxycarbonylamino-1-cyclohexyl-3R,4R and 4S-dihydroxy-6-methylheptane (Formula B-3a and B-3b: Z₁ is Cbz) Refer to Chart B.

A solution of 47.8 mg of the less polar title product of formula B-2a of Preparation 6 in 1 mL of 1:1 = trifluoroacetic acid:dichloromethane is allowed to stir for 45 minutes. The mixture is then partitioned between dichloromethane and aqueous sodium bicarbonate. The organic phase is dried (magnesium sulfate) and then concentrated to give 41.5 mg of the corresponding amine. To a stirred solution of this amine and 19 mg of N-tert-butyloxycarbonyl-L-phenylalanine in 0.5 mL of dichloromethane is added 0.03 mL of diisopropylethylamine and 0.012 mL of diethyl-phosphoryl cyanide. After stirring overnight, concentrated mixture is then chromatographed on silica gel with 1:1 = ethylacetate:dichloromethane to give 49 mg of the title product of formula B-3a.

By the same procedure 35 mg of the more polar title product of formula B-2b of Preparation 6 is converted to 32 mg of the title product of formula B-3b.

Physical characteristics are as follows:
FAB-HRMS: Found 931.4641.
¹H NMR (CDCl₃) is consistent with structure.

### Preparation 8 N-tert-Butyloxycarbonyl-L-phenylalanyl-L-histidyl-2S-amino-6-benzyloxycarbonylamino-1-cyclohexyl-3R,4R and 4S-dihydroxy-6-methylheptane (Formula B-4a and B-4b: Z₁ is Cbz) Refer to Chart B.

A solution of 49 mg of the title product of formula B-3a of Preparation 7 and 30 mg of 1-hydroxybenzotriazole in 0.5 mL of methanol is allowed to stir at room temperature for 4 hours. The concentrated mixture is then chromatographed on silica gel with 4%-8% methanol (saturated with ammonia) in dichloromethane to give 40 mg of the title product of formula B-4a.

By the same procedure 32 mg of the title product of formula B-3b of Preparation 7 is converted to 25 mg of the title product of formula B-4b.

Physical characteristics are as follows:
¹H NMR (CDCl₃) is consistent with structure.

### Examples 1 and 2 L-Histidinamide, N-[(1,1-dimethylethoxy)carbonyl]-L-phenylalanyl-N-[5-amino-1-(cyclohexylmethyl)-2,3-dihydroxy-5-methylhexyl]-, or N-tert-Butyloxycarbonyl-L-phenylalanyl-L-histidyl-2S,6-diamino-1-cyclohexyl-3R,4R and 4S-dihydroxy-6-methylheptane (Formula B-5a and B-5b) Refer to Chart B.

A mixture of 40 mg of the title product of formula B-4a of Preparation 8 and 30 mg of 5% palladium on charcoal in 1 mL of absolute ethanol is shaken under 50 psi of hydrogen for 3 hours. The mixture is filtered through Celite with ethanol washings and the filtrate is then concentrated to give 28 mg of the title product of formula B-5a.

By the same procedure 25 mg of the title product of formula B-4b of Preparation 8 is converted to 18 mg of the title product of formula B-5b.

Physical characteristics are as follows:
Formula B-5a: FAB-HRMS: Found 643.4202.
IC₅₀(M): 3.2 x 10⁻⁹.
Formula B-5b: FAB-HRMS: Found 643.4183.
IC₅₀(M): 6.4 x 10⁻⁸.

### Preparation 9 Boc-βVal-Phe-His(Ts)-X(Cbz) (Formula C-2: X is 1-cyclohexyl-2S,6-diamino-3R,4S-dihydroxy-6-methylheptane;Cbz is benzyloxcarbonyl.) Refer to Chart C.

To a solution of 168 mg of Boc-Phe-His(Ts)-X(Cbz) of Preparation 7 in 1.5 mL of dichloromethane is added 1.5 mL of trifluoroacetic acid and then allowed to stir at room temperature. After 45 minutes, the reaction mixture is diluted with dichloromethane and then added slowly to a saturated aqueous solution of sodium bicarbonate. The resulting mixture is separated and the aqueous layer extracted with several portions of dichloromethane. The combined organic phase is dried (magnesium sulfate) and then concentrated to give the free amine.

To a stirred solution of 126 mg of H-Phe-His(Ts)-X(Cbz) and 50 mg of Boc-βVal-OH in 2 mL of dichloromethane is added 50 µl of diisopropylethylamine, followed by 35 µL of diethylphosphoryl cyanide. The reaction mixture is stirred for 15 hours and then flashed chromatographed on silica gel with 3% to 4% methanol in dichloromethane to afford 143 mg of the title compound as a glassy white solid.

Physical characteristics are as follows:
FAB-HRMS: (m+H)=1030.535.

### Preparation 10 Boc-βVal-Phe-His-X(Cbz) (Formula C-3: X is 1-cyclohexyl-2S,6 diamino-3R,4S-dihydroxy-6-methyheptane; Cbz is benzyloxycarbonyl.) Refer to Chart C.

To a flask containing 143 mg of the title product of Preparation 9 and 85 mg of 1-hydroxybenzotriazole hydrate is added 1.5 mL of methanol. After dissolution, the flask is capped and left to stir at room temperature. After 2.5 days, the reaction mixture is then concentrated under reduced pressure. Chromatography of the residue on silica with 3% to 5% methanol (saturated with ammonia) in dichloromethane affords 120 mg of the title product as a glassy white solid.

Physical characteristics are as follows:
FAB-HRMS: (m+H)=876.5247.

### Preparation 11 Boc-βVal-Phe-His-X (Formula C-4: X is 1-cyclohexyl-2S,6-diamino-3R,4S-dihydroxy-6-methylheptane; Cbz is benzyloxycarbonyl.) Refer to Chart C.

To a mixture of 120 mg of the title product of Preparation 10, 75 mg of 5% Pd/C, and 195 mg of ammonium formate is added 1.5 mL of diemthylformamide. The resulting back slurry is back-filled with argon gas and rapidly stirred at room temperature. After 40 hours, the reaction mixture is diluted with dichloromethane and then filtered through Celite with methanol/methylene chloride washings. The filtrate is concentrated and dimethylformamide removed under high vacuum. The residue is chromatographed on silica gel with 10% to 20% methanol (saturated with ammonia) in dichloromethane to afford 79 mg of the title compound as a white solid.

Physical characteristics are as follows:
FAB-HRMS: (m+H)=742.4851.

### Example 3 L-Arabino-heptitol, 6-amino-2-[[N-[N-(3-amino-3-methl-1-oxobutyl)-L-phenylalanyl]-L-histidyl]amino]-1-cyclohexyl-1,2,5,6,7-pentadeoxy-6-C-methyl-, tris(trifluoroacetate) (salt) or βVal-Phe-His-X (Formula C-5: X is 1-cyclohexyl-2S,6-diamino-3R,4S-dihydroxy-6-methylheptane; Cbz is benzyloxycarbonyl.) Refer to Chart C.

A solution of 79 mg of the title product of Preparation 11 in 1.0 mL of 1:1 trifluoroacetic acid-dichloromethane is allowed to stir for one hour and is dripped slowly into a rapidly stirred 1:2 ether-hexane solution. The precipitated solid is spun down, washed trice with 1:2 ether-hexane and dried under both house and high vacuum to afford 95 mg of the title product as a white solid.

Physical characteristics are as follows:
FAB-HRMS: (m+H)=642.4363.
HPLC (20/80 A/B; 225 nm) k'=5.21.
IC₅₀(M): 2.4 x 10⁻⁸.

### Preparation 12 Boc-N-Me-His(TS)-X(Cbz) (Formula D-1: X is 1-cyclohexyl-2S,6-diamino-3R,4S-dihydroxy-6-methylheptane;Cbz is benzyloxcarbonyl.) Refer to Chart D.

To a stirred solution of 470 mg of H-X(Cbz) and 800 mg of Boc-N-Me-His(Ts)-OH in 10 mL of dichloromethane is added 0.38 mL of diisopropylethylamine, followed by 0.29 mL of diethylphosphoryl cyanide. The reaction mixture is stirred overnight, then flash chromatographed on silica gel with 25% to 50% ethyl acetate in dichloromethane to afford 685 mg of the title compound as a pale tan solid.

Physical characteristics are as follows:
FAB-HRMS: (m+H)=798.4094.

### Preparation 13 Boc-Phe-N-Me-His(Ts)-X(Cbz) (Formula D-2: X is 1-cyclohexyl-2S,6-diamino-3R,4S-dihydroxy-6-methylheptane; Cbz is benzyloxycarbonyl.) Refer to Chart D.

To a solution of 685 mg of the title product of Preparation 12 in 5 mL of dichloromethane is added 5 mL of trifluoroacetic acid. The solution is allowed to stir for one hour. The reaction mixture is diluted with dichloromethane and then added to a stirring solution of saturated aqueous sodium bicarbonate. The resulting mixture is separated and the aqueous layer extracted with several portions of dichloromethane. The combined organic phase is dried (magnesium sulfate) and then concentrated to give 610 mg of the free amine.

To a stirred solution of 610 mg of H-N-Me-His(Ts)-X(Cbz) and 330 mg of Boc-Phe-OH in 9 mL of dichloromethane is added 0.27 mL of diisopropylethylamine, followed by 320 mg of bis(2-oxo-3-oxazolidinyl)phosphinic choride (BOPCl). The reaction mixture is stirred overnight and then flash chromatographed on silica gel with 0% to 5% methanol in dichloromethane to afford 690 mg of the title compound as a pale yellow solid.

Physical characteristics are as follows:
FAB-HRMS: (m+H)=945.4805.

### Preparation 14 Boc-βVal-Phe-N-Me-His(Ts)-X(Cbz) (Formula D-3: X is 1-cyclohexyl-2S,6-diamino-3R,4S-dihydroxy-6-methylheptane; Cbz is benzyloxycarbonyl.) Refer to Chart D.

To a solution of 690 mg of the title product of Preparation 13 in 4 mL of dichloromethane is added 4 mL of trifluoroacetic acid. The solution is allowed to stir for 1.5 hours. The reaction mixture is diluted with dichloromethane and then added to a stirring solution of saturated aqueous sodium bicarbonate. The resulting mixture is separated and the aqueous layer extracted with several portions of dichloromethane. The combined organic phase is dried (magnesium sulfate) and then concentrated to give 593 mg of the free amine.

To a stirred solution of 200 mg of H-Phe-N-Me-His(Ts)-X(Cbz) and 75 mg of Boc-βVal-OH in 3 mL of dichloromethane is added 75 µL of diisopropylethylamine, followed by 55 µL of diethylphosphoryl cyanide. The reaction mixture is stirred overnight, then flash chromatographed on silica with 20% to 50% ethyl acetate in hexane to afford 181 mg of the title compound as a tan solid.

Physical characteristics are as follows:
FAB-HRMS: (m+H)=1044.551.

### Preparation 15 Boc-βVal-Phe-N-Me-His-X(Cbz) (Formula D-4: X is 1-cyclohexyl-2S,6-diamino-3R,4S-dihydroxy-6-methylheptane; Cbz is benzyloxycarbonyl.) Refer to Chart D.

A solution of 181 mg of the title product of Preparation 14 and 150 mg of 1-hydroxybenzotriazole hydrate in 2 mL of methanol is allowed to stir for 3.5 days and is then concentrated under reduced pressure. Chromatography of the residue on silica with 5% to 7% methanol (saturated with ammonia) in dichloromethane affords 128 mg of the title peptide as a white solid.

Physical characteristics are as follows:
FAB-HRMS: (m+H)=890.5379.

### Preparation 16 Boc-βVal-Phe-N-Me-His-X (Formula D-5: X is 1-cyclohexyl-2S,6-diamino-3R,4S-dihydroxy-6-methylheptane;Cbz is benzyloxycarbonyl.) Refer to Chart D.

To a flask containing 128 mg of the title product of Preparation 15, 75 mg of 5% Pd/C, and 210 mg of ammonium formate is added 1.5 mL of dimethylformamide. After stirring 2 days under an argon atmosphere, the reaction mixture is diluted with dichloromethane and then filtered through Celite/cotton plug with dichloromethane washings of the filter cake. The filtrate is concentrated and dimethylformamide removed on high vac. The residue is chromatographed on silica gel with 10% to 20% methanol (saturated with ammonia) in dichloromethane to afford 74 mg of the title product as a white solid.

Physical characteristics are as follows:
FAB-HRMS: (m+H)=756.5021.

### Example 4 L-Arabino-heptitol, 6-amino-2-[[N-[N-(3-amino-3-methyl-1-oxobutyl)-L-phenylalanyl]-N-methyl-L-histidyl]amino]-1,2,5,6,7-pentadeoxy-1-cyclohexyl-6-methyl-, tris (trifluoroacetate) (salt) or βVal-Phe-N-Me-His-X (Formula D-6: X is 1-cyclohexyl-2S,6-diamino-3R,4S-dihydroxy-6-methylheptane; Cbz is benzyloxycarbonyl.) Refer to Chart D.

A solution of 74 mg of the title product of Preparation 16 in 1 mL of 1:1 tetrahydrofuran-dichloromethane is allowed to stir for one hour and is then dripped slowly into 90 mL of rapidly stirred 1:2 ether-hexane. The solution immediately begins to turn cloudy and the flocculent white product is allowed to form over 0.5 hour. The precipitated solid is spun down, washed twice with 1:2 ether-hexane, and carefully dried under both house and high vacuum to afford 90 mg of the title product as a white solid.

Physical characteristics are as follows:
FAB-HRMS: (m+H)=656.4495.
HPLC (20/80 A/B; 225 nm) k'=7.35.
IC₅₀(M): 1.5 x 10⁻⁷.

### Preparation 17 Boc-Pro-Phe-N-Me-His(Ts)-X(Cbz) (Formula E-2: X is 1-cyclohexyl-2S,6-diamino-3R,4S-dihydroxy-6-methylheptane; Cbz is benzyloxycarbonyl.) Refer to Chart E.

To a stirred solution of 200 mg of the deprotected title product of formula E-1, i.e., H-Phe-N-Me-His(Ts)-X-(Cbz), prepared as described in the first paragraph of Preparation 13, and 75 mg of Boc-Pro-OH in 3 mL of dichloromethane is added 75 µL of diisopropylethylamine, followed by 55 µL of diethylphosphoryl cyanide. The reaction mixture is stirred for 20 hours, then flash chromatographed on silica with 0% to 5% methanol in dichloromethane to afford 240 mg of the title product.

Physical characteristics are as follows:
FAB-HRMS: (m+H)=1042.537.

### Preparation 18 Boc-γGlu(Boc)-Pro-Phe-N-Me-His(Ts)-X(Cbz) (Formula E-3: X is 1-cyclohexyl-2S,6-diamino-3R,4S-dihydroxy-6-methylheptane; Cbz is benzyloxycarbonyl.) Refer to Chart E.

A solution of 240 mg of the title product of Preparation 17 in 1.5 mL of dichloromethane is added 1.5 mL of trifluoroacetic acid and then allowed to stir at room temperature. After two hours, the reaction mixture is diluted with dichloromethane and then slowly to a saturated aqueous solution of sodium bicarbonate. The resulting mixture is separated and the aqueous layer extracted with several portions of dichloromethane. The combined organic phase is dried (magnesium sulfate) and then concentrated to give the free amine.

To a stirred solution of 204 mg of H-Pro-Phe-N-Me-His(Ts)-X(Cbz) and 160 mg of Boc-τGlu(Boc)-OH dicyclohexylamine salt in 3 mL of dichloromethane is added 70 µL of diisopropylethylamine, followed by 50 µL of diethylphosphoryl cyanide. The reaction mixture is stirred for 3.5 days and then flash chromatographed on silica gel with 0% to 5% methanol in dichloromethane to afford 246 mg of the title product as a pale orange foam.

Physical Characteristics are as follows:
FAB-HRMS: (m+H)=1227.636

### Preparation 19 Boc-γGlu(Boc)-Pro-Phe-N-Me-His-X(Cbz) (Formula E-4: X is 1-cyclohexyl-2S,6-diamino-3R,4S-dihydroxy-6-methylheptane; Cbz is benzyloxycarbonyl.) Refer to Chart E.

To a flask containing 246 mg of the title product of Preparation 18 and 123 mg of 1-hydroxybenzotriazole hydrate is added 2 mL of methanol. After dissolution, the flask is allowed to stir for 24 hours and is then concentrated under reduced pressure. Chromatography of the residue on silica with 5% to 10% methanol (saturated with ammonia) in dichloromethane affords 163 mg of the title product as a glassy white solid.

Physical Characteristics are as follows:
FAB-HRMS: (m+H)=1073.638.

### Preparation 20 Boc-γGlu(Boc)-Pro-Phe-N-Me-His-X (Formula E-5: X is 1-cyclohexyl-2S,6-diamino-3R,4S-dihydroxy-6-methylheptane; Cbz is benzyloxycarbonyl.) Refer to Chart E.

To a flask containing 163 mg of the title product of Preparation 19, 100 mg of 5% Pd/C, 150 mg of ammonium formate is added 2 mL of dimethylformamide. After stirring for 5 days, the reaction mixture is diluted with dichloromethane and filtered through Celite. The filter cake is washed with methanol/methylene chloride. The combined filtrates are concentrated and dimethylformamide removed under high vacuum. The residue is chromatographed on silica gel with 10% to 20% methanol (saturated with ammonia) in dichloromethane to afford 69 mg of the title product.

Physical Characteristics are as follows:
FAB-HRMS: (m+H)=939.5910.

### Example 5 L-Arabino-heptitol6-amino-1,2,5,6,7-pentadeoxy-1-cyclohexyl-2-[[N-[N-(1-L-γ-glutamyl-L-prolyl)-L-phenylalanyl]-N-methyl-L-histidyl]amino]-6-methyl-tris(trifluoroacetate) (salt) or γGlu-Pro-Phe-N-Me-His-X (Formula E-6: X is 1-cyclohexyl-2S,6-diamino-3R,4S-dihydroxy-6-methylheptane; Cbz is benzyloxycarbonyl.) Refer to Chart E.

To a flask containing 69 mg of the title product of Preparation 20 is added 1 mL of trifluoroacetic acid. After one hour the reaction mixture is slowly dripped into 90 mL of a rapidly stirred 1:2 ether-hexane. After 0.5 hour the precipitated solid is spun down, washed twice with 1:2 ether-hexane, and dried under both house and high vacuum to afford 74 mg of the title product as a creme-white solid.

Physical Characteristics are as follows:
FAB-HRMS: (m+H)=783.4758;
HPLC (20/80 A/B; 225 nm) k'=7.35.
IC₅₀(M):2.6 x 10⁻⁸.

### Preparation 21 Boc-O-MeTyr-His(Ts)-X(Cbz) (Formula F-2: X is 1-cyclohexyl-2S,6-diamino-3R,4S-dihydroxy-6-methylheptane; Cbz is benzyloxycarbonyl.) Refer to Chart F.

To a flask containing 90 mg of the deprotected compound of formula F-1 of Preparation 6, i.e., H-His(Ts)-X(Cbz) and 80 mg of Boc-Tyr(OMe)-OH dicyclohexylamine salt is added 2 mL of dichloromethane. After dissolution of the solids, 35 µL of diisopropylethylamine and 25 µL of diethylphosphoryl cyanide are added. The reaction mixture is stirred overnight and then the solvent is removed at reduced pressue. The residue is chromatographed on silica gel with 40% ethyl acetate in hexane to afford 104 mg of the title product as a light brown solid.

Physical Characteristics are as follows:
FAB-HRMS: (m+H)=961.4742.

### Preparation 22 Boc-O-MeTyr-His-X(Cbz) (Formula F-3: X is 1-cyclohexyl-2S,6-diamino-3R,4S-dihydroxy-6-methylheptane;Cbz is benzyloxycarbonyl.) Refer to Chart F.

To a flask containing 104 mg of the title product of Preparation 21 and 66 mg of 1-hydroxybenzotriazole hydrate is added 1.5 mL of methanol. After dissolution, the flask is allowed to stir for 15 hours and is then concentrated under reduced pressure. Chromatography of the residue on silica with 5% to 7% methanol (saturated with ammonia) in dichloromethane affords 54 mg of the title product as a white solid.

Physical Characteristics are as follows:
FAB-HRMS: (m+H)=807.4640.

### Example 6 L-Arabino-heptitol6-amino-1-cyclohexyl-1,2,5,6,7-pentadeoxy-2-[[N-[N-[(1,1-dimethylethoxy)carbonyl]-0-methyl-L-tyrosyl]-L-histidyl]amino-6-methyl, or Boc-O-MeTyr-His-X (Formula F-4: X is 1-cyclohexyl-2S,6-diamino-3R,4S-dihydroxy-6-methylheptane; Cbz is benzyloxycarbonyl.) Refer to Chart F.

To a Parr pressure test tube containing 40 mg of 5% pallidium on carbon is added 54 mg of the title product of Preparation 22 as a solution in 2 mL of absolute ethanol. The resulting black slurry is placed on a Parr shaker at a hydrogen gas pressure of @55 psi. After shaking for 5 hours, the reaction mixture is filtered through Celite. The filter cake is washed with absolute ethanol and the combined filtrates concentrated at reduced pressure. Further concentration of the reaction mixture under high vacuum affords 40 mg of the title product as a white solid.

Physical Characteristics are as follows:
FAB-HRMS: (m+H)=673.4293;
HPLC (60/40 A/B; 225 nm) k'=10.07.
IC₅₀(M): 3.4 x 10⁻⁹.

### Preparation 23 Boc-Pro-O-MeTyr-His(Ts)-X(Cbz) (Formula G-2: X is 1-cyclohexyl-2S,6-diamino-3R,4S-dihydroxy-6-methyleptane; Cbz is benzyloxycarbonyl.) Refer to Chart G.

A solution of 118 mg of Boc-O-MeTyr-His(Ts)-X(Cbz) of formula G-1 in 1 mL of dichloromethane is added 1 mL of trifluoroacetic acid and then allowed to stir at room temperature. After one hour, the reaction mixture is diluted with dichloromethane and then added slowly to a saturated aqueous solution of sodium bicarbonate. The resulting mixture is separated and the aqueous layer extracted with several portions of dichloromethane. The combined organic phase is dried (magnesium sulfate) and then concentrated to give 102 mg of the free amine.

To a stirred solution of 51 mg of H-O-MeTyr-His(Ts)-X(Cbz) and 18 mg of Boc-Pro-OH in 1 mL of dichloromethane is added 19 µL of diisopropylethylamine, followed by 13 µL of diethylphosphoryl cyanide. The reaction mixture is stirred overnight and then flask chromatographed on silica gel with 5% methanol in dichloromethane to afford 63 mg of the title product.

Physical Characteristics are as follows:
FAB-HRMS: (m+H)=1058.528.

### Preparation 24 Boc-γGlu(Boc)-Pro-O-MeTyr-His(Ts)-X(Cbz) (Formula G-3: X is 1-cyclohexyl-2S,6-diamino-3R,4S-dihydroxy-6-methylheptane; Cbz is benzyloxycarbonyl.) Refer to Chart G.

A solution of 63 mg of the title product of Preparation 23 in 0.5 mL of dichloromethane is added 0.5 mL of trifluoroacetic acid and then allowed to stir at room temperature. After one hour, the reaction mixture is diluted with dichloromethane and then added slowly to a saturated aqueous solution of sodium bicarbonate. The resulting mixture is separated and the aqueous layer extracted with several portions of dichloromethane. The combined organic phase is dried (magnesium sulfate) and then concentrated to give the free amine.

To a stirred solution of 52 mg of H-Pro-O-MeTyr-His(Ts)-X(Cbz) and 34 mg of Boc-γGlu(Boc)-OH dicyclohexylamine salt in 2 mL of dichloromethane is added 15 µL of diisopropylethylamine, followed by 11 µL of diethyphosphoryl cyanide. The reaction mixture is stirred overnight and then flash chromatographed on silica gel with 5% methanol in dichloromethane to afford 49 mg of the title product.

Physical Characteristics are as follows:
FAB-HRMS: (m+H)=1243.629.

### Preparation 25 Boc-γGlu(Boc)-Pro-O-MeTyr-His-X(Cbz) (Formula G-4: X is 1-cyclohexyl-2S,6-diamino-3R,4S-dihydroxy-6-methylheptane; Cbz is benzyloxycarbonyl.) Refer to Chart G.

To a flask containing 49 mg of the title product of Preparation 24 and 24 mg of 1-hydroxy-benzotriazole hydrate is added 2 mL of methanol. After dissolution, the flask is allowed to stir for 24 hours and is then concentrated under reduced pressure. Chromatography of the residue on silica with 5% methanol (saturated with ammonia) in dichloromethane affords 36 mg of the title product as a glassy white solid.

Physical Characteristics are as follows:
FAB-HRMS: (m+H)=1089.625.

### Preparation 26 Boc-γGlu(Boc)-Pro-O-MeTyr-His-X (Formula G-5: X is 1-cyclohexyl-2S,6-diamino-3R,4S-dihydroxy-6-methylheptane; Cbz is benzyloxycarbonyl.) Refer to Chart G.

To a flask containing 36 mg of the title product of Preparation 25, 20 mg of 5% Pd/C, 31 mg of ammonium formate is added 2 mL of dimethylformamide. After stirring for one day, the reaction mixture is diluted with dichloromethane and filtered through Celite. The filter cake is washed with methanol/methylene chloride. The combined filtrates are concentrated and dimethylformamide removed under high vacuum. The residue is chromatographed on silica gel with 10% to 15% methanol (saturated with ammonia) in dichloromethane to afford 24 mg of the title product .

Physical Characteristics are as follows:
FAB-HRMS: (m+H)=955.5872.

### Example 7 L-Arabino-heptitol6-amino-1,2,5,6,7-pentadeoxy-1-cyclohexyl-2-[[N-[N-(1-L-γ-glutamyl-L-prolyl)-O-methyl-L-tyrosyl]-L-histidyl]-amino]-6-methyl-, tris(trisluoroacetate) salt, or γGlu-Pro-O-MeTyr-His-X (Formula G-6: X is 1-cyclohexyl-2S,6-diamino-3R,4S-dihydroxy-6-methylheptane; Cbz is benzyloxycarbonyl.) Refer to Chart G .

To a flask containing 24 mg of the title product of Preparation 26 is added 1 mL of trifluoroacetic acid. After one hour the reaction mixture is slowly dripped into 50 mL of a rapidly stirred 1:2 ether-hexane. After 0.5 hour the precipitated solid is spun down, washed twice with 1:2 ether-hexane, and dried under high vacuum to afford 28 mg of the title product as a white solid.

Physical Characteristics are as follows:
FAB HRMS: (m+H)=799.4731.
HPLC (20/80 A/B; 225 nm) k'=7.82.
IC₅₀(M): 34% inhibition at 10⁻⁸M.

### Preparation 27 Glc(perAc)-Pro-O-MeTyr-His(Ts)-X(Cbz) (Formula H-2: X is 1-cyclohexyl-2S,6-diamino-3R,4S-dihydroxy-6-methylheptane; Cbz is benzyloxycarbonyl.) Refer to Chart H.

To a stirred solution of 51 mg of deprotected Boc-O-MeTyr-His(Ts)-X(Cbz) of formula H-1, i.e., H-O-MeTyr-His(Ts)-X(Cbz) prepared as described in the first paragraph of Preparation 23, and 41 mg of Glc(perAc)-Pro-OH of formula I-4, prepared as described in Chart I, in 2 mL of dichloromethane is added 19 µL of diisopropylethylamine, followed by 13 µL of diethylphosphoryl cyanide. The reaction mixture is stirred overnight and then chromatographed on silica gel with 5% methanol in dichloromethane to afford 29 mg of the title product.

Physical characteristics are as follows:

The proposed structure is supported by ¹H-NMR.

### Preparation 28 Glc-Pro-O-MeTyr-His-X(Cbz) (Formula H-3: X is 1-cyclohexyl-2S,6-diamino-3R,4S-dihydroxy-6-methylheptane; Cbz is benzyloxycarbonyl.) Refer to Chart H.

A solution of 29 mg of the title product of Preparation 27 is allowed to stand in 1 mL of saturated ammonia in methanol overnight. The mixture is concentrated and the residue chromatographed on silica gel with 10% to 20% methanol (saturated with ammonia) in dichloromethane to give 14 mg of the title product.

Physical Characteristics are as follows:
FAB-HRMS: (m+H)=1009.527.

### Example 8 L-Arabino-heptitol,6-amino-1,2,5,6,7-pentadeoxy-1-cyclohexyl-2-[[N,-[N-(2-deoxy-glucopyranose-2-aminocarbonyl-L-prolyl)-O-methyl-L-tyrosyl]-L-histidyl]amino]-6-methyl, tris(trifluoroacetate) salt, or Glc-Pro-O-MeTyr-His-X (Formula H-4: X is 1-cyclohexyl-2S,6-diamino-3R,4S-dihydroxy-6-methylheptane; Cbz is benzyloxycarbonyl.) Refer to Chart H.

To a flask containing 14 mg of the title product of Preparation 28, 10 mg of 5% Pd/C, 15 mg of ammonium formate is added 1 mL of dimethylformamide. After stirring for overnight, the reaction mixture is diluted with dichloromethane and filtered through Celite. The filter cake is washed with methanol/methylene chloride. The combined filtrates are concentrated and dimethylformamide removed under high vacuum. The residue is treated with 1 mL of acetic acid/methylene chloride which is then added to ether. The mixture is spun down to afford 11 mg of the title product.

Physical Characteristics are as follows:
FAB-HRMS: (m+H)=875.4895.
HPLC (10/90 A/B; 225 nm) k'=8.16.

### Preparation 29 1,3,4,6-Tetra-acetyl-2-deoxy-glucopyranose-2-amino-carbonyl-L-prolyl-benzyl ester (Formula I-3: Ac is acetyl; Bzl is benzyl.) Refer to Chart I.

A solution of 0.935 g of the compound of formula I-1, 0.484 g of proline benzyl ester hydrochloride of formula I-2 and 0.7 mL of diisopropylethylamine in 2 mL of dioxane is stirred in an oil bath at 70°C for three hours. The reaction mixture is chromatographed on silica gel with 50%-70% ethyl acetate in hexane to give 0.84 g of the title product.

Physical Characteristics are as follows:
¹H-NMR spectrum is consistent with the structure.

### Preparation 30 1,3,4,6-Tetra-acetyl-2-deoxy-glucopyranose-2-amino-carbonyl-L-proline (Formula I-4: Ac is acetyl.) Refer to Chart I.

A mixture of 0.55 g of the title product of Preparation 29 and 50 mg of 5% palladium on charcoal in 10 mL of ethyl acetate is shaken under 50 psi of hydrogen for three hours. The mixture is then filtered through Celite with ethyl acetate washings. The filtrate is concentrated to give 0.34 g of the title product.

Physical characteristics are as follows:
¹H-NMR spectrum is consistent with the structure.

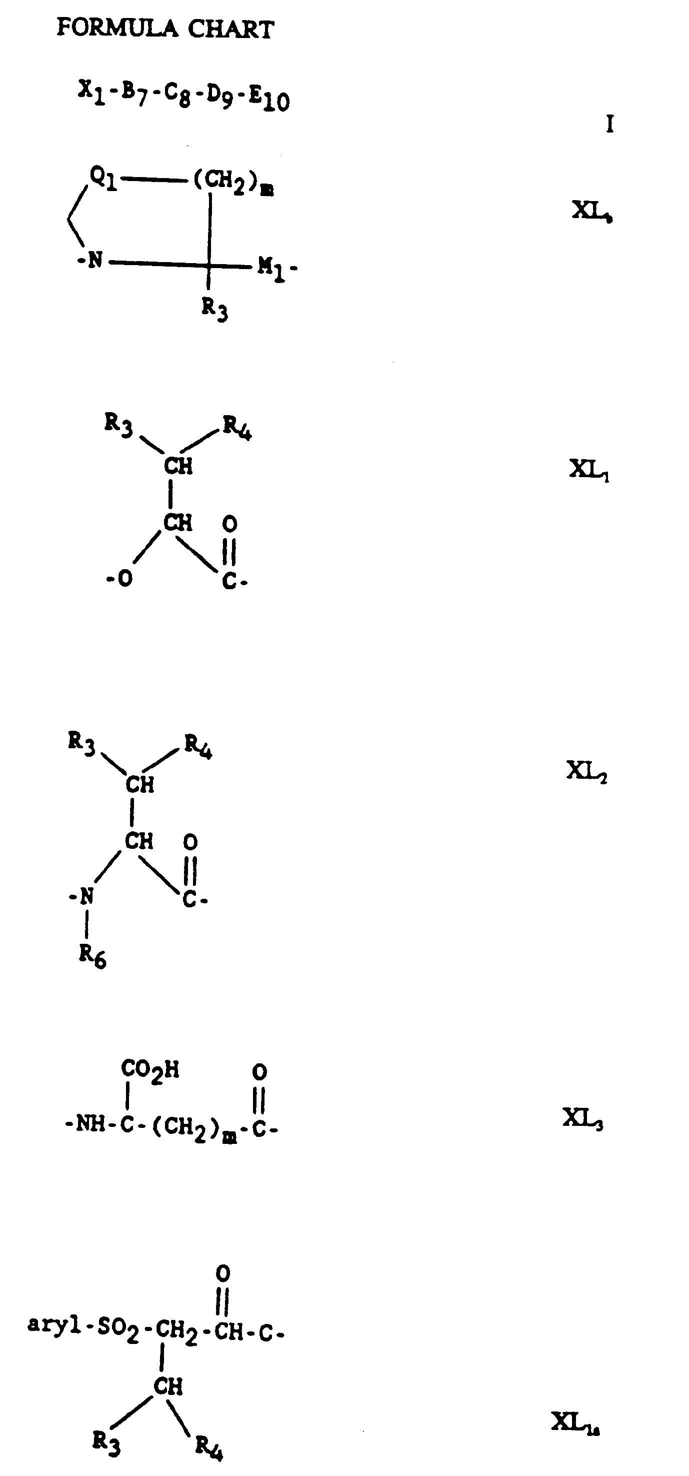

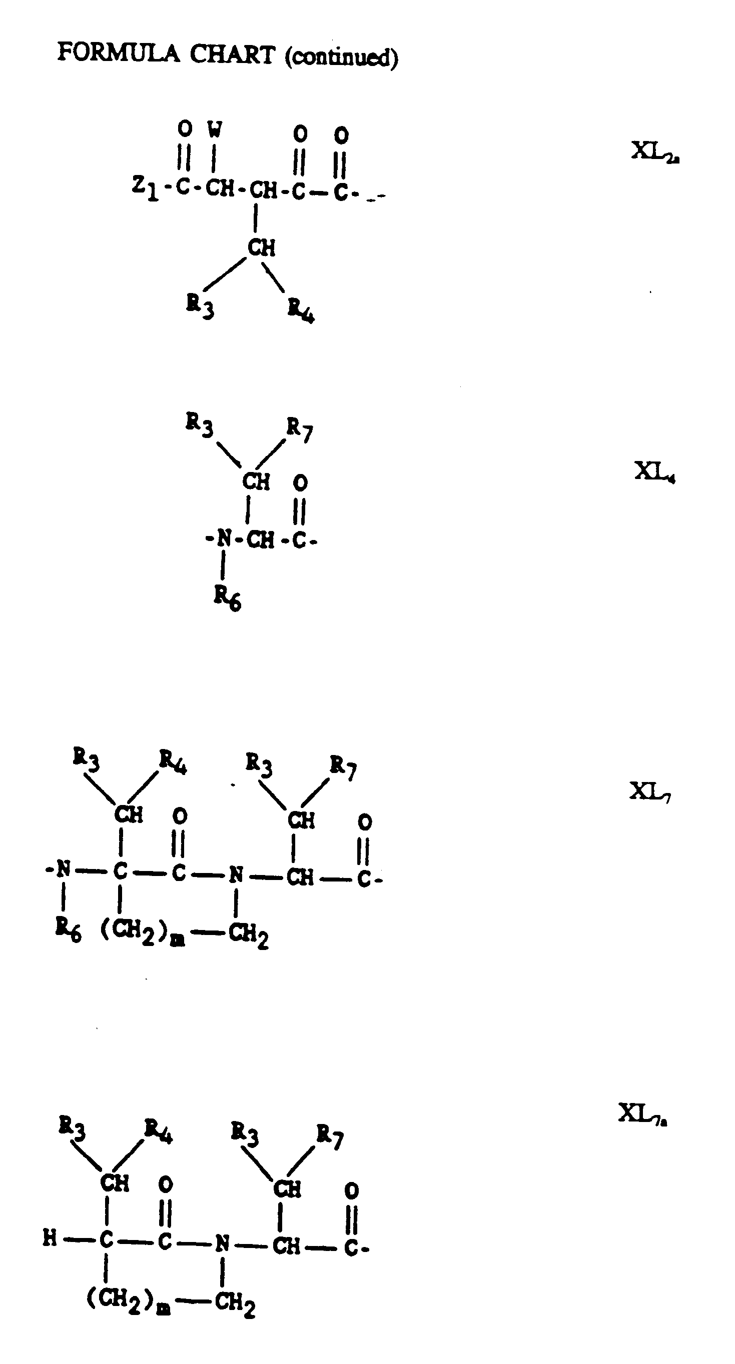

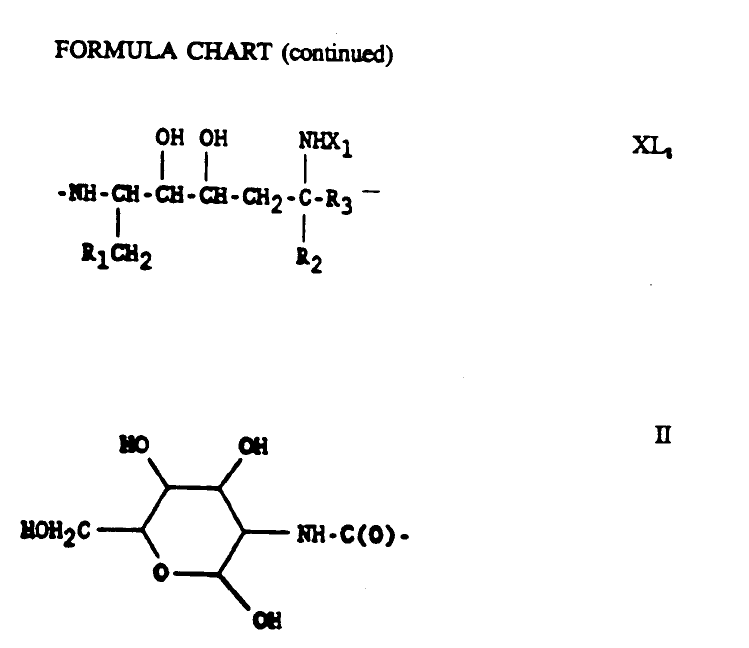

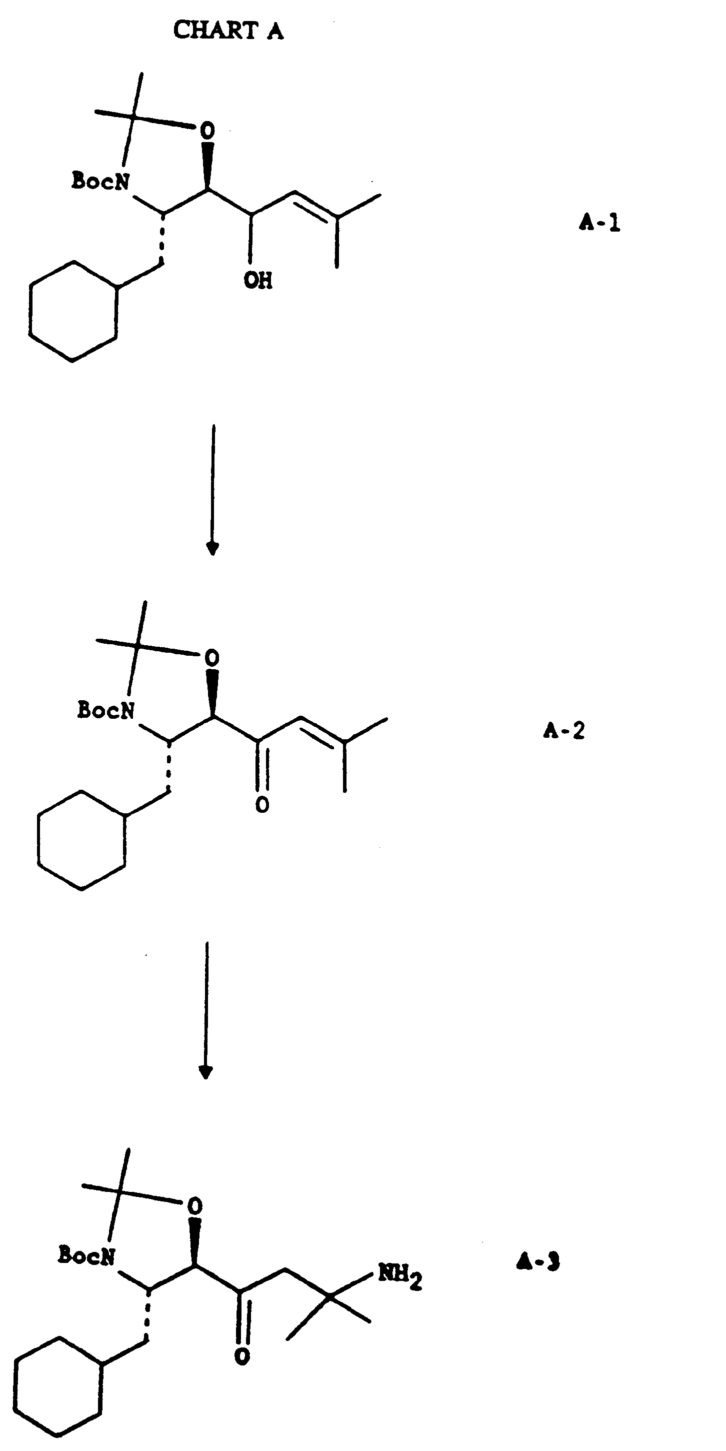

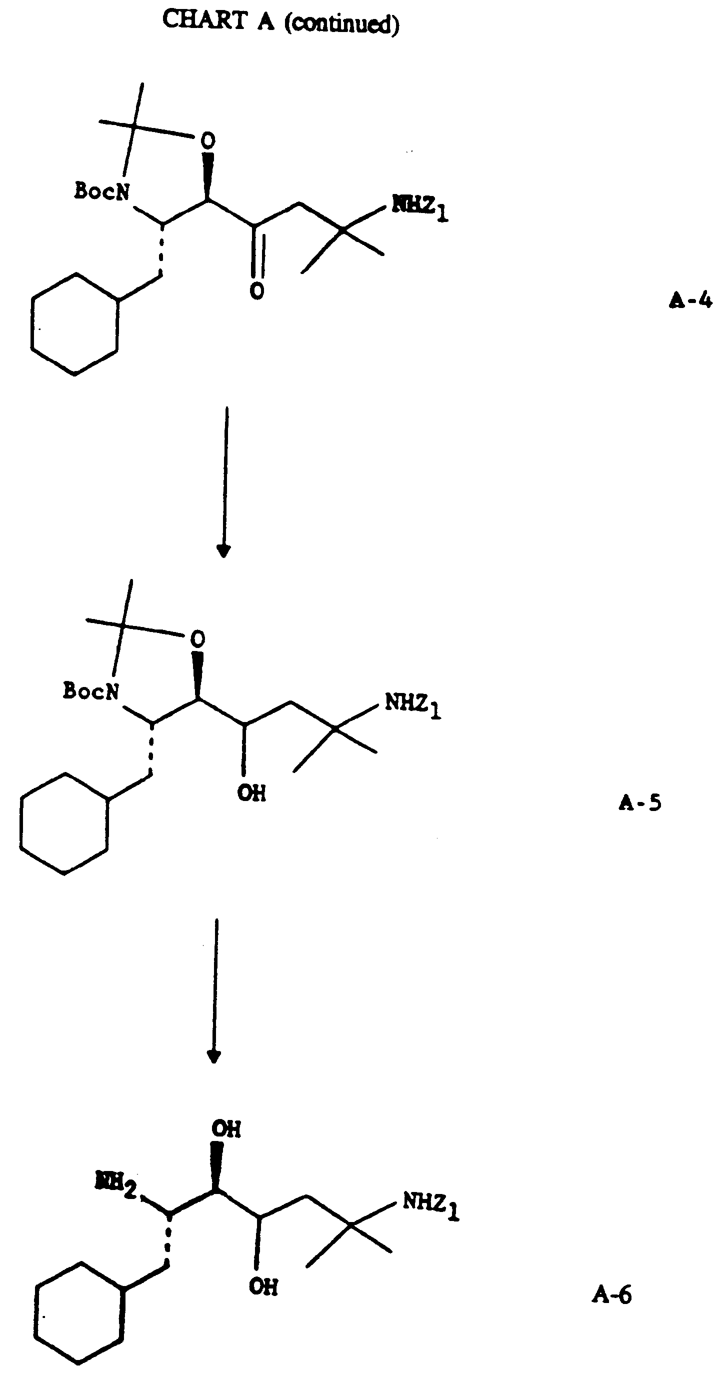

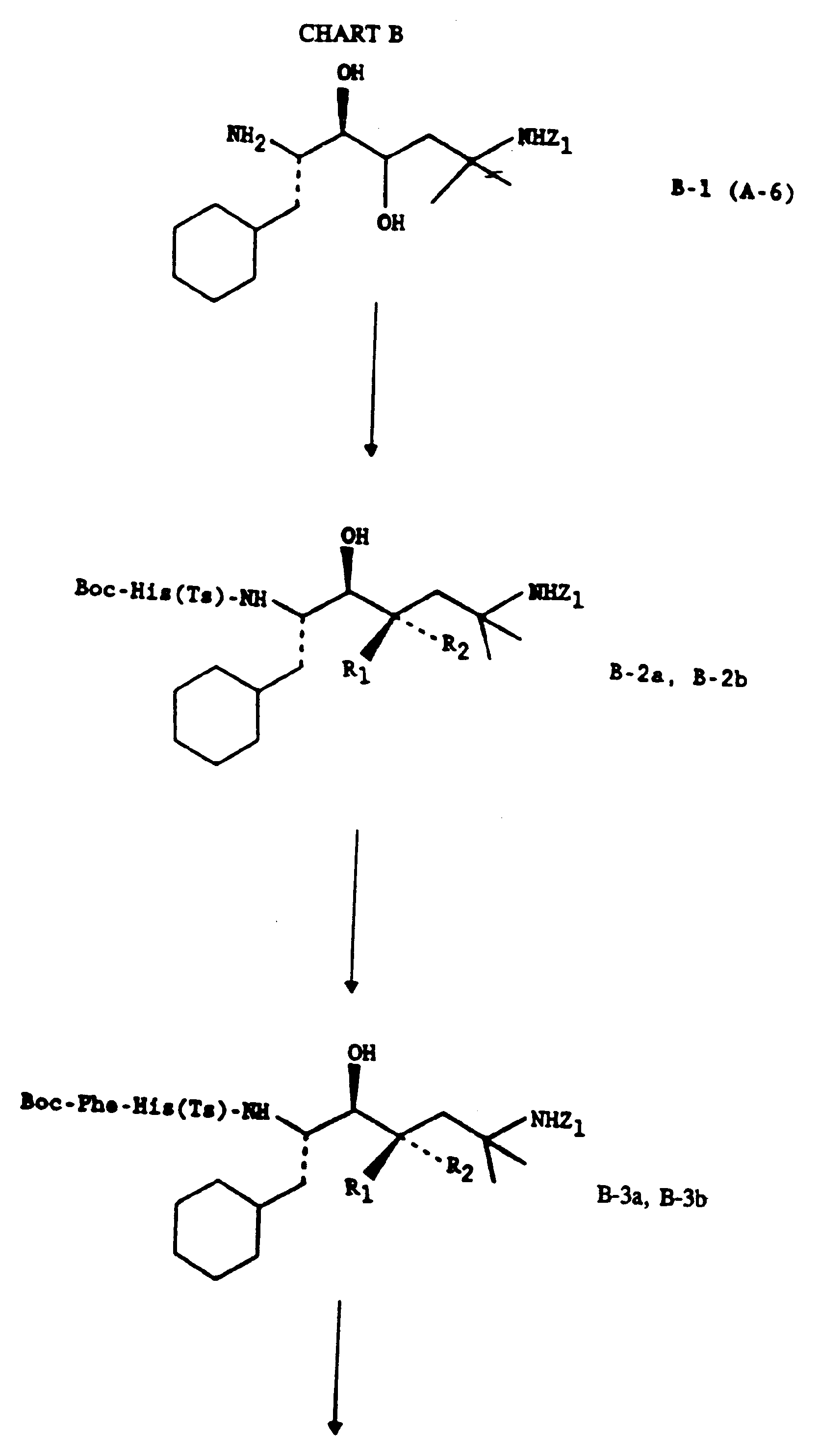

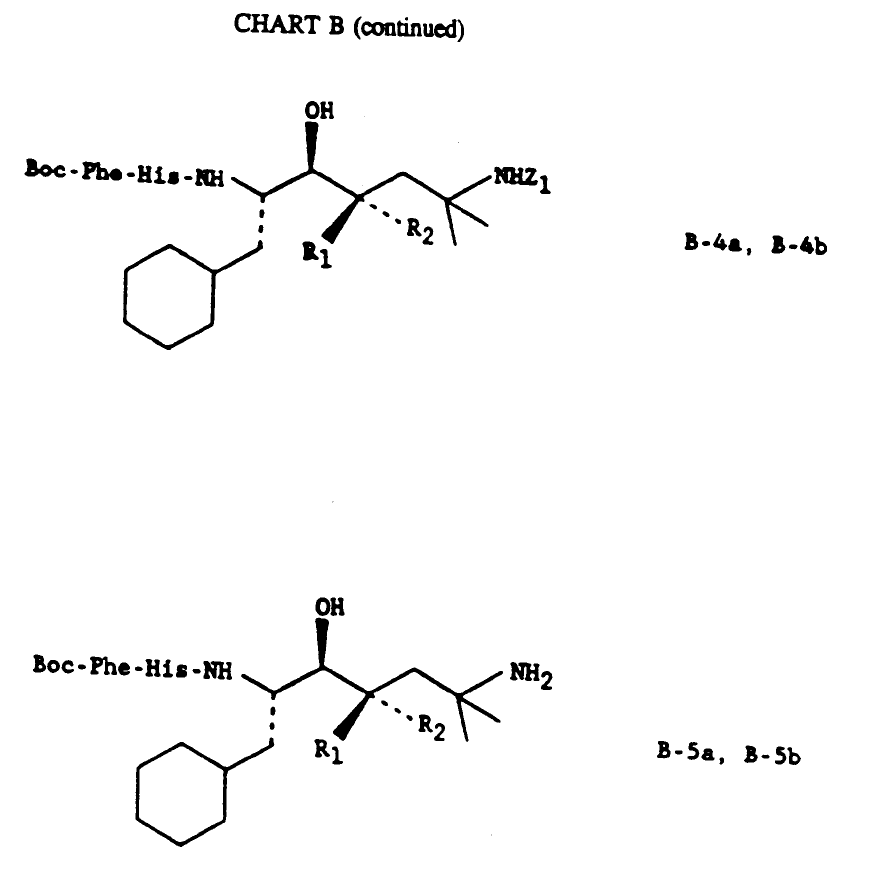

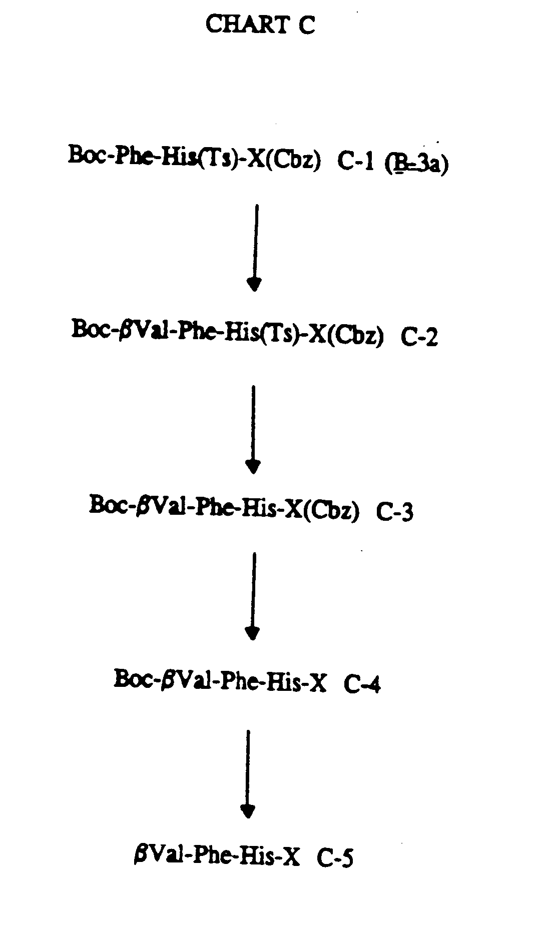

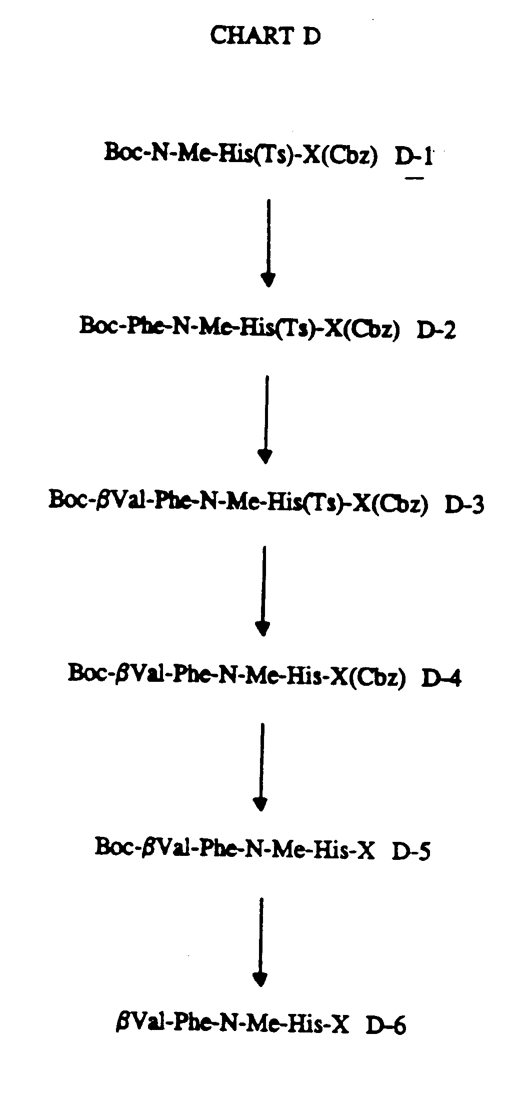

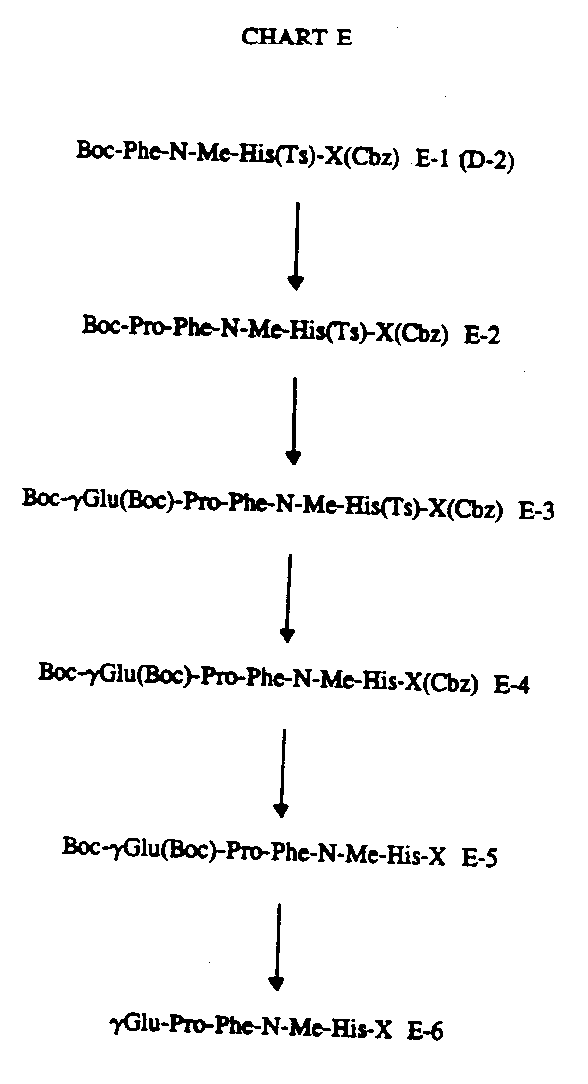

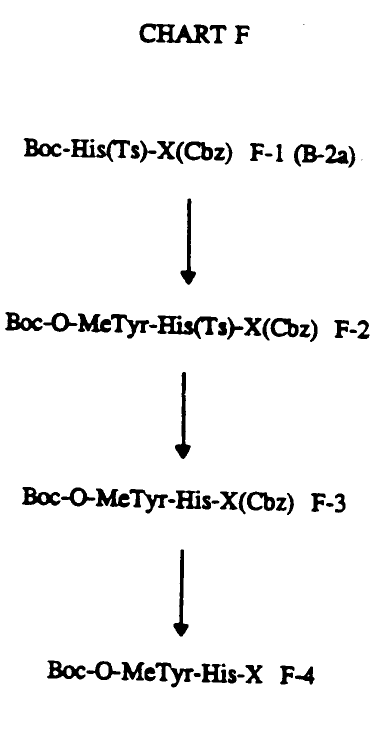

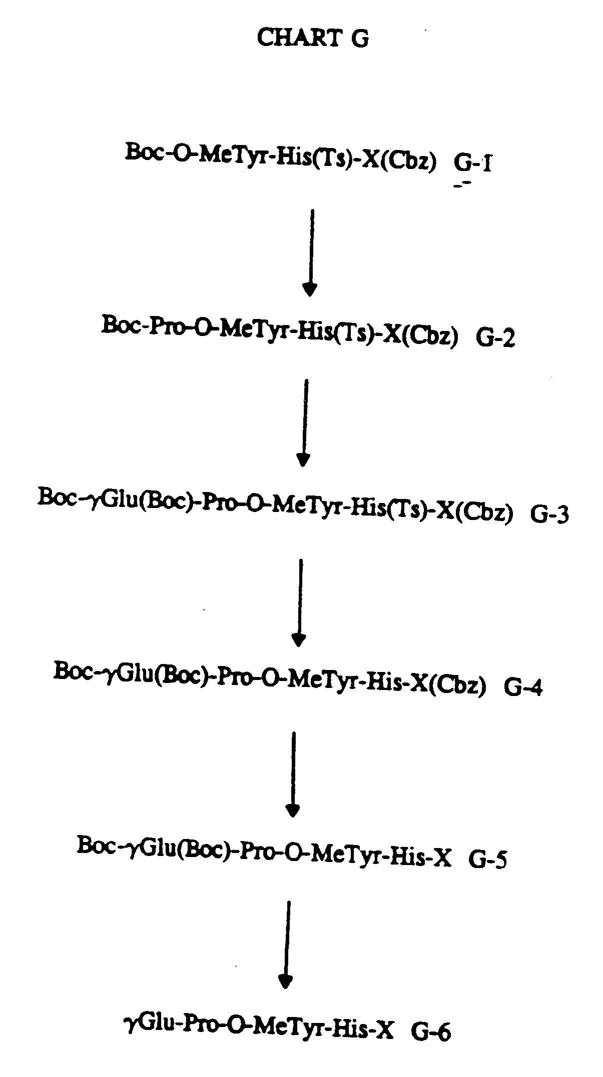

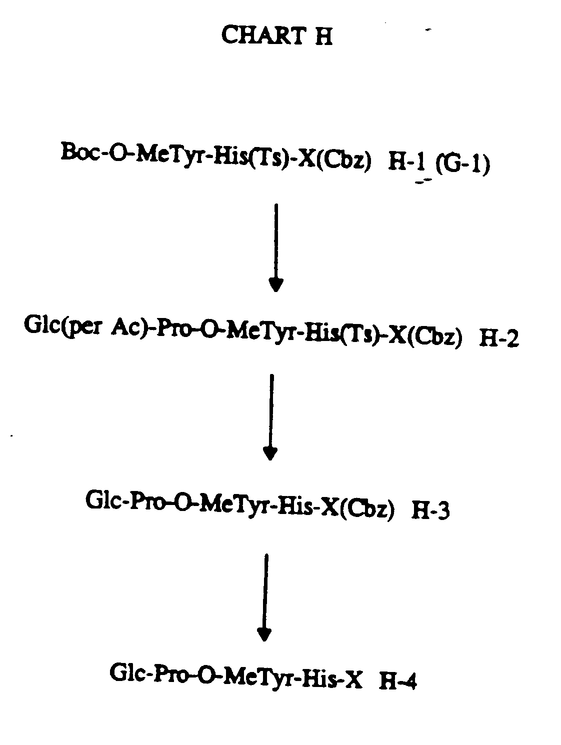

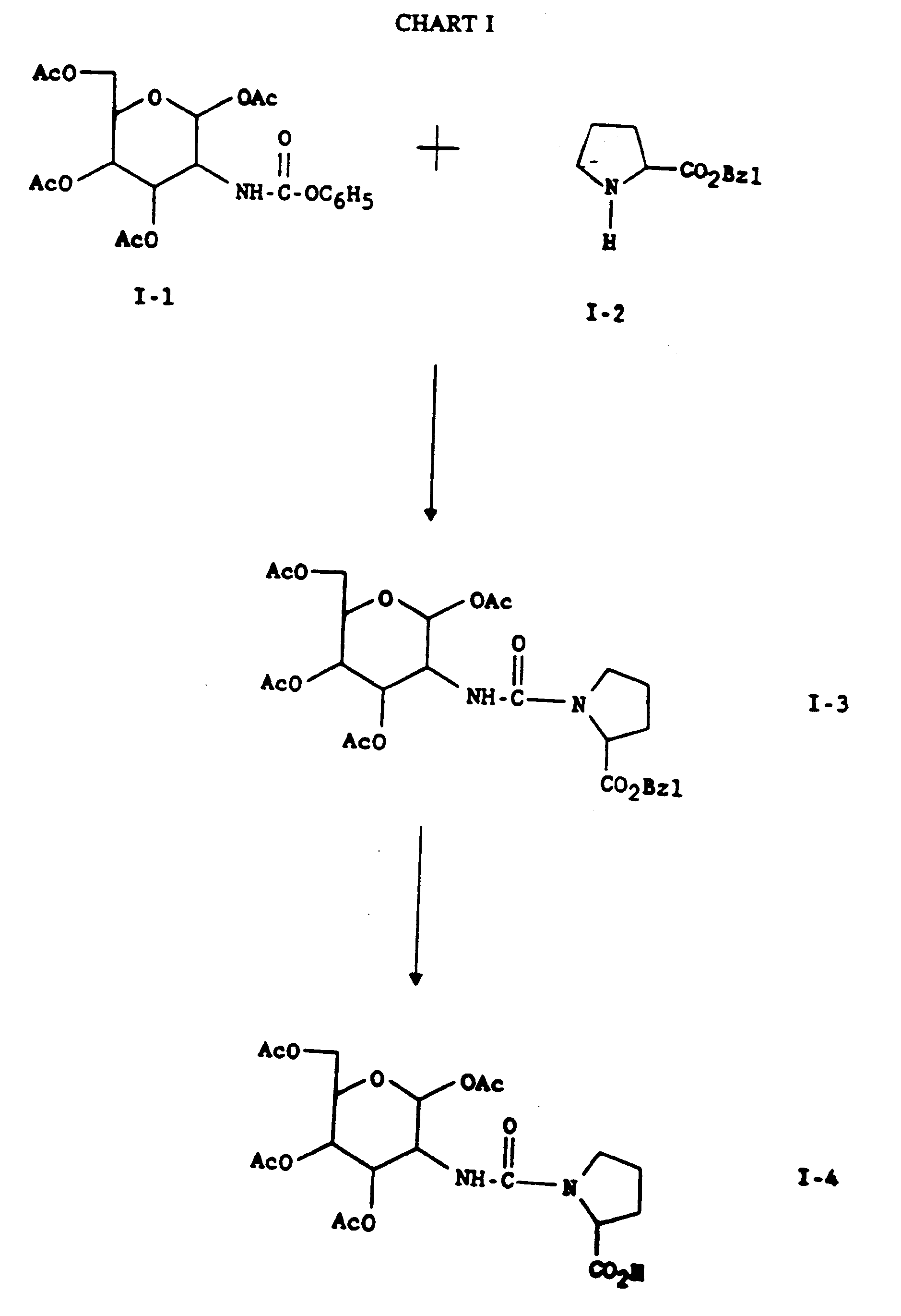

## Claims (Claims for the following Contracting State(s): AT, BE, CH, LI, DE, DK, FR, GB, IT, LU, NL, SE)

1. A peptide having a non-cleavable transition state insert corresponding to the 10,11-position of a renin substrate (angiotensinogen) of the formula XL₈ 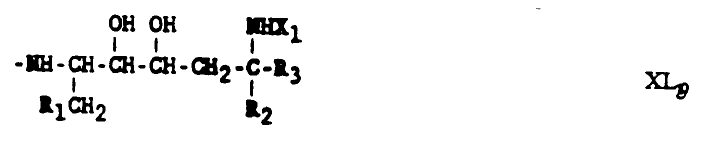 wherein X₁ is
(a) hydrogen,
(b) Y₁-(CH₂)ₐ-C(O)-,
(c) (HOCH₂)₃C-NH-C(O)-,
(d) (HOCH₂)₂CH-NH-C(O)-,
(e) 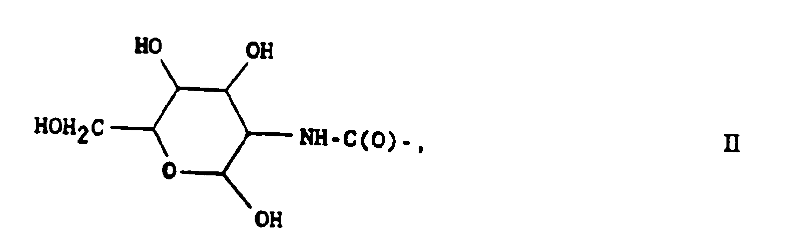
(f) HOH₂C(CHOH)ₐCH(CH₂OH)-NH-C(O)-,
(g) NaO₃S(CH₂)₂N(CH₃)C(O)(CH₂)₆C(O)-,
(h) R₂C(O)-,
(i) R₂-O-C(O)- or
(j) Z₁-C(O)-;
wherein Y₁ is
(a) -NH₂,
(b) -CO₂H,
(c) -C(NH₂)(CH₃)₂,
(d) -CH(NH₂)(CO₂H), or
(e) -OP(O)(OH)₂;
wherein Z₁ is -Het bonded through a nitrogen;
wherein R₁ is
(a) hydrogen,
(b) C₁-C₈ alkyl,
(c) aryl,
(d) C₃-C₇ cycloalkyl,
(e) Het,
(f) C₁-C₃ alkoxy, or
(g) C₁-C₃ alkylthio;
wherein R₂ is
(a) C₁-C₁₀ alkyl,
(b) C₃-C₇ cycloalkyl,
(c) aryl,
(d) Het,
(e) aryl C₁-C₅ alkyl, or
(f) Het C₁-C₅ alkyl;
wherein R₃ is
(a) C₁-C₁₀ alkyl,
(b) -(CH₂)ₚ-aryl,
(c) -(CH₂)ₚ-Het,
(d) -(CH₂)ₚ-C₃-C₇ cycloalkyl, or
(e) -1- or 2-adamantyl;
wherein n is 0 to five inclusive;
wherein p is 0 to two inclusive;
wherein aryl is phenyl or naphthyl substituted by zero to three of the following:
(a) C₁-C₃ alkyl,
(b) hydroxy,
(c) C₁-C₃ alkoxy,
(d) halo,
(e) amino,
(f) mono- or di-C₁-C₃ alkylamino,
(g) -CHO,
(h) -COOH,
(i) -CONH₂,
(j) -CONH(C₁-C₅ alkyl),
(k) -nitro,
(l) mercapto,
(m) C₁-C₃ alkylthio,
(n) C₁-C₃ alkylsulfinyl,
(o) C₁-C₃ alkylsulfonyl,
(p) -N(R₆)-C₁-C₃ alkylsulfonyl,
(q) SO₃H,
(r) SO₂NH₂,
(s) -CN, or
(t) -CH₂NH₂;
wherein -Het is a 5- or 6-membered saturated or unsaturated ring containing from one to three heteroatoms selected from the group consisting of nitrogen, oxygen, and sulfur; and including any bicyclic group in which any of the above heterocyclic rings is rused to a benzene ring, which heterocyclic moiety is substituted with zero to three of the following:
(i) (C₁-C₆ alkyl,
(ii) hydroxy,
(iii) trifluoromethyl,
(iv) C₁-C₄ alkoxy,
(v) halo,
(vi) aryl,
(vii) aryl C₁-C₄ alkyl-,
(viii) amino,
(ix) mono- or di-(C₁-C₄ alkyl) amino, or
(x) C₁-C₅ alkanoyl;
wherein R₆ is
(a) hydrogen,
(b) C₁-C₅ alkyl,
(c) -(CH₂)ₚ-aryl, or
(d) -(CH₂)ₚ-Het.

2. The peptide of claim 1, of formula I
X₁-B₇-C₈-D₉-E₁₀ I
wherein X₁ is as defined in claim 1 and E₁₀ is of formula XL₈ as defined in claim 1;
wherein B₇ is absent or a divalent moiety of the formula XL_{b} 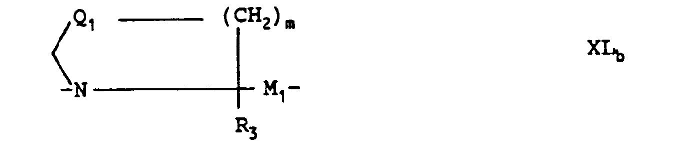 wherein C₂ is absent or a divalent moiety of the formula XL₁, XL₂ or XL₃; 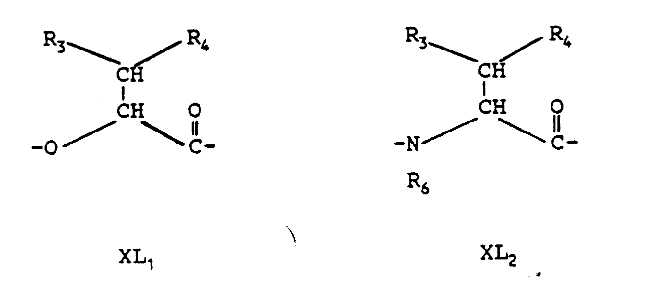 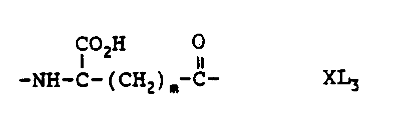 or wherein C₈ is a monovalent moiety of the formula XL₁ₐ or XL₂ₐ when X and B₇ are absent 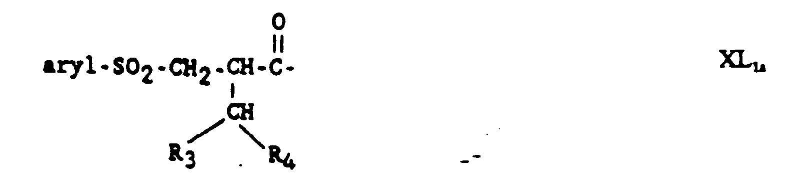 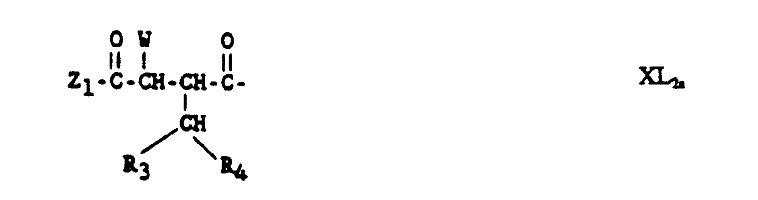 wherein D₉ is absent or a divalent moiety of the formula XL₄; 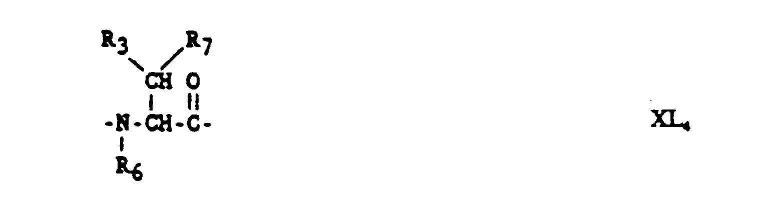 or wherein C₈-D₉ is XL₇, or when X, B₇ are absent, XL₇ₐ; 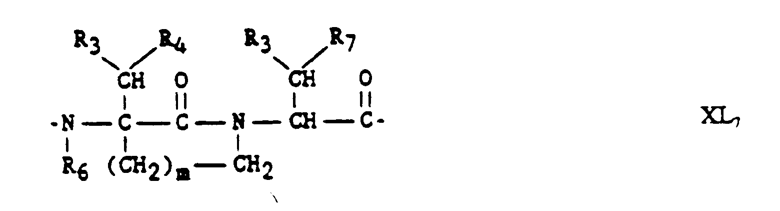 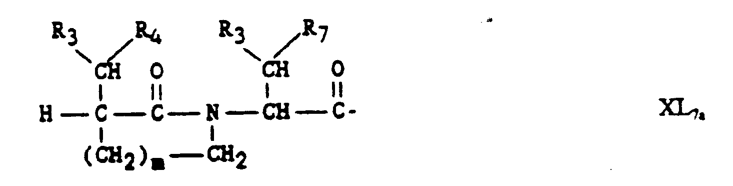 wherein Q is
(a) -CH₂-,
(b) -CH(OH)-,
(c) -O-, or
(d) -S-;
wherein M is -C(O)- or -CH₂-;
wherein m is 1 or 2;
wherein n is 0 to 5 inclusive;
wherein p is 0 to 2 inclusive;
wherein W is H or OH;
wherein Z₁, R₃ and R₆ are as defined in claim 1;
wherein R₄ is
(a) hydrogen,
(b) C₁-C₁₀ alkyl,
(c) -(CH₂)ₚ-aryl,
(d) -(CH₂)ₚ-Het, or
(e) -(CH₂)ₚ-C₃-C₇ cycloalkyl;
wherein R₅ is
(a) hydrogen,
(b) C₁-C₁₀ alkyl,
(c) hydroxy,
(d) amino C₁-C₄ alkyl,
(e) guanidinyl C₁-C₄ alkyl,
(f) aryl,
(g) Het,
(h) methylthio,
(i) -(CH₂)ₚ-C₃-C₇ cycloalkyl, or
(j) amino;
wherein R₇ is
(a) hydrogen,
(b) C₁-C₁₀ alkyl,
(c) hydroxy,
(d) amino C₁-C₄ alkyl,
(e) guanidinyl C₁-C₄ alkyl,
(f) aryl,
(g) Het,
(h) methylthio,
(i) -(CH₂)ₚ-C₃-C₇ cycloalkyl, or
(j) amino,
wherein aryl and Het are as defined in claim 1, or a carboxy, amino or other reactive group protected form thereof;
or a pharmaceutically acceptable acid or base addition salts thereof.

3. A peptide of claim 2
wherein X₁ at N-terminal is t-butyloxycarbonyl, β-valyl, τ-glutamyl, or 2-deoxy-glucopyranose-2-aminocarbonyl;
wherein B₇ is absent, Pro or a derivative thereof;
wherein C₈ is Phe, OMe-Tyr or a derivative thereof;
wherein D₉ is His, N(Me)His or a derivative thereof;
wherein E is 1-cyclohexyl-2S,6-diamino-3R,4S-dihydroxy-6-methylheptane;
or a pharmaceutically acceptable salt thereof.

4. A peptide of claim 3 selected from
L-Histidinamide,N-[(1,1-dimethylethoxy)carbonyl]-L-phenylalanyl-N-[5-amino-1-(cyclohexylmethyl)-2,3-dihydroxy-5-methylhexyl]-, or N-tert-Butyloxycarbonyl-L-phenylalanyl-L-histidyl-2S,6-diamino-1-cyclohexyl-3R,4R-dihydroxy-6-methylheptane;
L-Histidinamide,N-[(1,1-dimethylethoxy)carbonyl]-L-phenylalanyl-N-[5-amino-1-(cyclohexylmethyl)-2,3-dihydroxy-5-methylhexyl]-, or N-tert-Butyloxycarbonyl-L-phenylalanyl-L-histidyl-2S,6-diamino-1-cyclohexyl-3R,4S-dihydroxy-6-methylheptane;
L-Arabino-heptitol, 6-amino-2-[[N-[N-(3-amino-3-methyl-1-oxobutyl)-L-phenylalanyl]-L-histidyl]amino]-1-cyclohexyl-1,2,5,6,7-pentadeoxy-6-C-methyl, tris(trifluoroacetate) (salt), or βVal-Phe-His-X;
L-Arabino-heptitol, 6-amino-2-[[N-[N-(3-amino-3-methyl-1-oxobutyl)-L-phenyhlalanyl]-N-methyl-L-histidyl]amino]1,2,5,6,7-pentadeoxy-1-cyclohexyl-6-methyl-, tris(trifluoroacetate) (salt), or βVal-Phe-N-Me-His-X;
L-Arabino-heptitol, 6-amino-1,2,5,6,7-pentadeoxy-1-cyclohexyl-2-[[N-[N-(1-L-γglutamyl-L-prolyl)-L-phenylalanyl]-N-methyl-L-histidyl]amino]-6-methyl-,tris(trifluoroacetate) (salt), or γGlu-Pro-Phe-N-Me-His-X;
L-Arabino-heptitol, 6-amino-1-cyclohexyl-1,2,5,6,7-pentadeoxy-2-[[N-[N[(1,1-dimethylethoxy)carbonyl]-0-methyl-L-tyrosyl]-L-histidyl]amino]-6-methyl-, or Boc-O-MeTyr-His-X;
L-Arabino-heptitol,6-amino-1,2,5,6,7-pentadeoxy-1-cyclohexyl-2-[[N-[N-(1-L-γ-glutamyl-L-prolyl)-O-methyl-L-tyrosyl]-L-histidyl]-amino]-6-methyl-, tris(trifluoroacetate) salt, or γGlu-Pro-O-MeTyr-His-X; and
L-Arabino-heptitol, 6-amino-1,2,5,6,7-pentadeoxy-1-cyclohexyl-2-[[N-[N-(2-deoxy-glucopyranose-2-aminocarbonyl-L-prolyl)-O-methyl-L-tyrosyl]-L-histidyl]-amino]-6-methyl-, tris(trifluoroacetate) salt, or Glc-Pro-O-MeTyr-His-X.

## Claims (Claims for the following Contracting State(s): ES)

1. A process for preparing a peptide having a non-cleavable transition state insert corresponding to the 10,11-position of a renin substrate (angiotensinogen) of the formula XL₈ 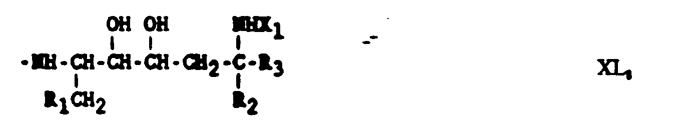 wherein X₁ is
(a) hydrogen,
(b) Y₁-(CH₂)ₐ-C(O)-,
(c) (HOCH₂)₃C-NH-C(O)-,
(d) (HOCH₂)₂CH-NH-C(O)-,
(e) 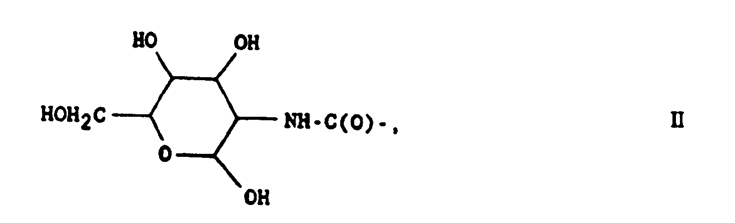
(f) HOH₂C(CHOH)ₐCH(CH₂OH)-NH-C(O)-,
(g) NaO₃S(CH₂)₂N(CH₃)C(O)(CH₂)₆C(O)-,
(h) R₂C(O)-,
(i) R₂-O-C(O)- or
(j) Z₁-C(O)-;
wherein Y₁ is
(a) -NH₂,
(b) -CO₂H,
(c) -C(NH₂)(CH₃)₂,
(d) -CH(NH₂)(CO₂H), or
(e) -OP(O)(OH)₂;
wherein Z₁ is -Het bonded through a nitrogen;
wherein R₁ is
(a) hydrogen,
(b) C₁-C₈ alkyl,
(c) aryl,
(d) C₃-C₇ cycloalkyl,
(e) Het,
(f) C₁-C₃ alkoxy, or
(g) C₁-C₃ alkylthio;
wherein R₂ is
(a) C₁-C₁₀ alkyl,
(b) C₃-C₇ cycloalkyl,
(c) aryl,
(d) Het,
(e) aryl C₁-C₅ alkyl, or
(f) Het C₁-C₅ alkyl;
wherein R₃ is
(a) C₁-C₁₀ alkyl,
(b) -(CH₂)ₚ-aryl,
(c) -(CH₂)ₚ-Het,
(d) -(CH₂)ₚ-C₃-C₇ cycloalkyl, or
(e) -1- or 2-adamantyl;
wherein n is 0 to five inclusive;
wherein p is 0 to two inclusive;
wherein aryl is phenyl or naphthyl substituted by zero to three of the following:
(a) C₁-C₃ alkyl,
(b) hydroxy,
(c) C₁-C₃ alkoxy,
(d) halo,
(e) amino,
(f) mono- or di C₁-C₃ alkylamino,
(g) -CHO,
(h) -COOH,
(i) -CONH₂,
(j) -CONH(C₁-C₅ alkyl),
(k) -nitro,
(l) mercapto,
(m) C₁-C₃ alkylthio,
(n) C₁-C₃ alkylsulfinyl,
(o) C₁-C₃ alkylsulfonyl,
(p) -N(R₆)-C₁-C₃ alkylsulfonyl,
(q) SO₃H,
(r) SO₂NH₂,
(s) -CN, or
(t) -CH₂NH₂;
wherein -Het is a 5- or 6-membered saturated or unsaturated ring containing from one to three heteroatoms selected from the group consisting of nitrogen, oxygen, and sulfur; and including any bicyclic group in which any of the above heterocyclic rings is rused to a benzene ring, which heterocyclic moiety is substituted with zero to three of the following:
(i) (C₁-C₆ alkyl,
(ii) hydroxy,
(iii) trifluoromethyl,
(iv) C₁-C₄ alkoxy,
(v) halo,
(vi) aryl,
(vii) aryl C₁-C₄ alkyl-,
(viii) amino,
(ix) mono- or di-(C₁-C₄ alkyl) amino, or
(x) C₁-C₅ alkanoyl;
wherein R₆ is
(a) hydrogen,
(b) C₁-C₅ alkyl,
(c) -(CH₂)ₚ-aryl, or
(d) -(CH₂)ₚ-Het,
which comprises successively reacting the appropriate acyl derivatives of amino-acid residues or analogues thereof, singly or as units of two or more residues, until the said peptide is prepared.

2. A process according to claim 1, wherein the said peptide is of formula I
X₁-B₇-C₈-D₉-E₁₀ I
wherein X₁ is as defined in claim 1 and E₁₀ is of formula XL₈ as defined in claim 1;
wherein B₇ is absent or a divalent moiety of the formula XL_{b} 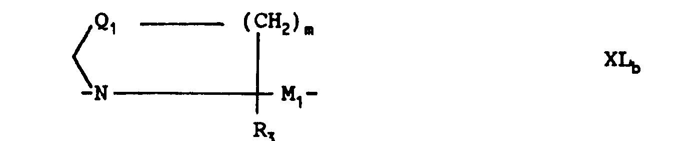 wherein C₂ is absent or a divalent moiety of the formula XL₁, XL₂ or XL₃; 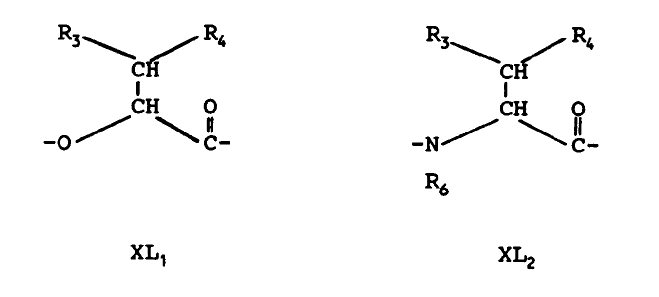 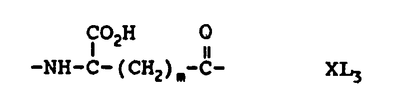 or wherein C₈ is a monovalent moiety of the formula XL₁ₐ or XL₂ₐ when X and B₇ are absent 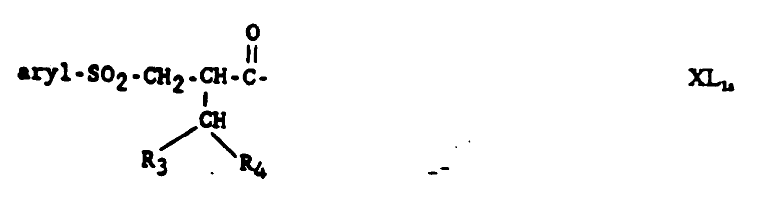 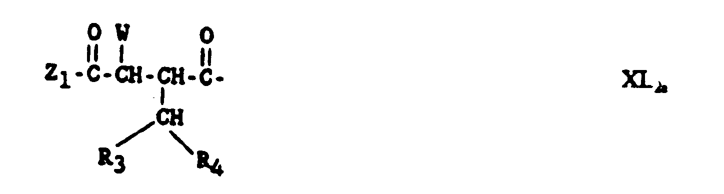 wherein D₉ is absent or a divalent moiety of the formula XL₄; 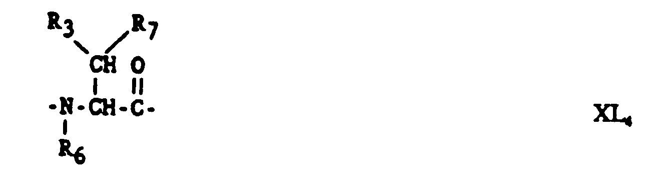 or wherein C₈-D₉ is XL₇, or when X, B₇ are absent, XL₇ₐ; 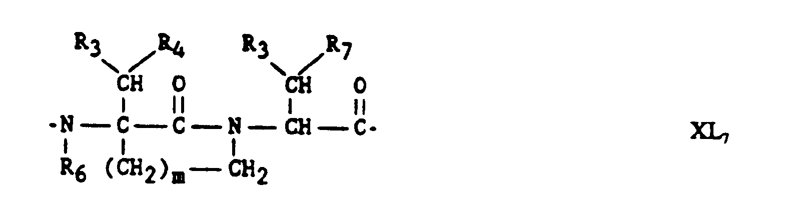 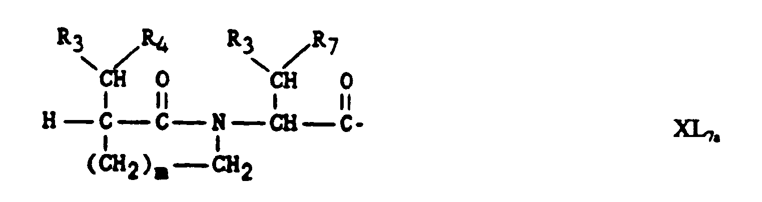 wherein Q is
(a) -CH₂-,
(b) -CH(OH)-,
(c) -O-, or
(d) -S-;
wherein M is -C(O)- or -CH₂-;
wherein m is 1 or 2;
wherein n is 0 to 5 inclusive;
wherein p is 0 to 2 inclusive;
wherein W is H or OH;
wherein Z₁, R₃ and R₆ are as defined in claim 1;
wherein R₄ is
(a) hydrogen,
(b) C₁-C₁₀ alkyl,
(c) -(CH₂)ₚ-aryl,
(d) -(CH₂)ₚ-Het, or
(e) -(CH₂)ₚ-C₃-C₇ cycloalkyl;
wherein R₅ is
(a) hydrogen,
(b) C₁-C₁₀ alkyl,
(c) hydroxy,
(d) amino C₁-C₄ alkyl,
(e) guanidinyl C₁-C₄ alkyl,
(f) aryl,
(g) Het,
(h) methylthio,
(i) -(CH₂)ₚ-C₃-C₇ cycloalkyl, or
(j) amino;
wherein R₇ is
(a) hydrogen,
(b) C₁-C₁₀ alkyl,
(c) hydroxy,
(d) amino C₁-C₄ alkyl,
(e) guanidinyl C₁-C₄ alkyl,
(f) aryl,
(g) Het,
(h) methylthio,
(i) -(CH₂)ₚ-C₃-C₇ cycloalkyl, or
(j) amino,
wherein aryl and Het are as defined in claim 1, or a carboxy, amino or other reactive group protected form thereof;
or a pharmaceutically acceptable acid or base addition salts thereof.

3. A process according to claim 2, wherein, in the said peptide,
X₁ at N-terminal is t-butyloxycarbonyl, β-valyl, τ-glutamyl or 2-deoxyglucopyranose-2-aminocarbonyl;
B₇ is absent, Pro or a derivative thereof;
C₈ is Phe, OMe-Tye or a derivative thereof;
D₉ is His, N(Me)His or a derivative thereof;
E is 1-cyclohexyl-2S,6-diamino-3R,4S-dihydroxy-6-methylheptane;
or a pharmaceutically-acceptable salt thereof.

4. A process according to claim 3, wherein the said peptide is selected from
L-Histidinamide,N-[(1,1-dimethylethoxy)carbonyl]-L-phenylalanyl-N-[5-amino-1-(cyclohexylmethyl)-2,3-dihydroxy-5-methylhexyl]-, or N-tert-Butyloxycarbonyl-L-phenylalanyl-L-histidyl-2S,6-diamino-1-cyclohexyl-3R,4R-dihydroxy-6-methylheptane;
L-Histidinamide,N-[(1,1-dimethylethoxy)carbonyl]-L-phenylalanyl-N-[5-amino-1-(cyclohexylmethyl)-2,3-dihydroxy-5-methylhexyl]-, or N-tert-Butyloxycarbonyl-L-phenylalanyl-L-histidyl-2S,6-diamino-1-cyclohexyl-3R,4S-dihydroxy-6-methylheptane;
L-Arabino-heptitol, 6-amino-2-[[N-[N-(3-amino-3-methyl-1-oxobutyl)-L-phenylalanyl]-L-histidyl]amino]-1-cyclohexyl-1,2,5,6,7-pentadeoxy-6-C-methyl, tris(trifluoroacetate) (salt), or βVal-Phe-His-X;
L-Arabino-heptitol, 6-amino-2-[[N-[N-(3-amino-3-methyl-1-oxobutyl)-L-phenyhlalanyl]-N-methyl-L-histidyl]amino]1,2,5,6,7-pentadeoxy-1-cyclohexyl-6-methyl-, tris(trifluoroacetate) (salt), or βVal-Phe-N-Me-His-X;
L-Arabino-heptitol, 6-amino-1,2,5,6,7-pentadeoxy-1-cyclohexyl-2-[[N-[N-(1-L-γglutamyl-L-prolyl)-L-phenylalanyl]-N-methyl-L-histidyl]amino]-6-methyl-, tris(trifluoroacetate) (salt), or γGlu-Pro-Phe-N-Me-His-X;
L-Arabino-heptitol, 6-amino-1-cyclohexyl-1,2,5,6,7-pentadeoxy-2-[[N-[N-[(1,1-dimethylethoxy)carbonyl]-0-methyl-L-tyrosyl]-L-histidyl]amino]-6-methyl-, or Boc-O-MeTyr-His-X;
L-Arabino-heptitol,6-amino-1,2,5,6,7-pentadeoxy-1-cyclohexyl-2-[[N-[N-(1-L-γ-glutamyl-L-prolyl)-O-methyl-L-tyrosyl]-L-histidyl]-amino]-6-methyl-, tris(trifluoroacetate) salt, or γGlu-Pro-O-MeTyr-His-X; and
L-Arabino-heptitol, 6-amino-1,2,5,6,7-pentadeoxy-1-cyclohexyl-2-[[N-[N-(2-deoxy-glucopyranose-2-aminocarbonyl-L-prolyl)-O-methyl-L-tyrosyl]-L-histidyl]-amino]-6-methyl-, tris(trifluoroacetate) salt, or Glc-Pro-O-MeTyr-His-X.

## Patentansprüche (Patentansprüche für folgende(n) Vertragsstaat(en): AT, BE, CH, DE, DK, FR, GB, IT, LI, LU, NL, SE)

1. Peptid mit einem nicht-spaltbaren Übergangszustandinsert entsprechend der 10,11-Stellung eines Reninsubstrats (Angiotensinogen) der Formel XL₈ 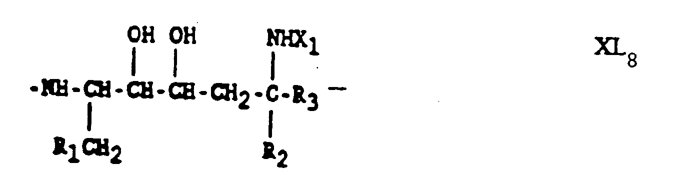 worin bedeuten:
(a) Wasserstoff,
(b) Y₁-(CH₂)ₐ-C(O)-,
(c) (HOCH₂)₃C-NH-C(O)-,
(d) (HOCH₂)₂CH-NH-C(O)-,
(e) 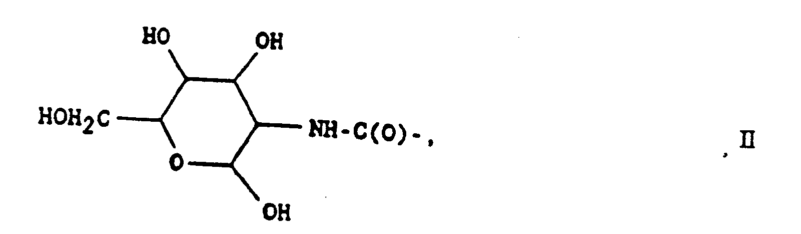
(f) HOH₂C(CHOH)ₐCH(CH₂OH)-NH-C(O)-,
(g) NaO₃S(CH₂)₂N(CH₃)C(O)(CH₂)₆C(O)-,
(h) R₂C(O)-,
(i) R₂-O-C(O)- oder
(j) Z₁-C(O)-;
mit Y₁ gleich
(a) -NH₂,
(b) -CO₂H,
(c) -C(NH₂)(CH₃)₂,
(d) -CH(NH₂(CO₂H), oder
(e) -OP(O)(OH)₂;
Z₁ gleich über einen Stickstoff gebundenes -Het,
R₁
(a) Wasserstoff,
(b) C₁-C₈ Alkyl,
(c) Aryl,
(d) C₃-C₇ Cycloalkyl,
(e) Het,
(f) C₁-C₃ Alkoxy oder
(g) C₁-C₃ Alkylthio;
R₂
(a) C₁-C₁₀ Alkyl,
(b) C₃-C₇ Cycloalkyl,
(c) Aryl,
(d) Het,
(e) Aryl C₁-C₅ alkyl oder
(f) Het C₁-C₅ alkyl;
R₃
(a) C₁-C₁₀ Alkyl,
(b) -(CH₂)ₚ-Aryl,
(c) -(CH₂)ₚ-Het,
(d) -(CH₂)ₚ-C₃-C₇ Cycloalkyl oder
(e) -1 oder 2-Adamantyl;
n = 0 bis einschließlich 5;
p = 0 bis einschließlich 2
mit Aryl gleich Phenyl oder Naphthyl, substituiert durch 0 bis 3 der folgenden Substituenten:
(a) C₁-C₃ Alkyl,
(b) Hydroxy,
(c) C₁-C₃ Alkoxy,
(d) Halogen,
(e) Amino,
(f) mono- oder di-C₁-C₃ Alkylamino,
(g) -CHO,
(h) -COOH,
(i) -CONH₂,
(j) -CONH(C₁-C₅ Alkyl)
(k) -Nitro,
(l) Mercapto,
(m) C₁-C₃ Alkylthio
(n) C₁-C₃ Alkylsulfinyl,
(o) C₁-C₃ Alkylsulfonyl,
(p) -N(R₆)-C₁-C₃ Alkylsulfonyl,
(q) SO₃H,
(r) SO₂NH₂,
(s) -CN oder
(t) -CH₂NH₂;
mit -Het gleich einem 5- oder 6-gliedrigen gesättigten oder ungesättigten Ring mit 1 bis 3 Heteroatom(en) aus der Gruppe Stickstoff, Sauerstoff und Schwefel, einschließlich irgendwelcher bicyclischer Gruppen, bei denen einer der genannten heterocyclischen Ringe an einen Benzolring ankondensiert ist, wobei die heterocyclische Einheit mit 0 bis 3 der folgenden Substituenten substituiert ist:
(i) (C₁-C₆ Alkyl,
(ii) Hydroxy,
(iii) Trifluormethyl,
(iv) C₁-C₄ Alkoxy,
(v) Halogen,
(vi) Aryl,
(vii) Aryl C₁-C₄ alkyl-,
(viii) Amino,
(ix) mono- oder di-(C₁-C₄ Alkyl) amino, oder
(x) C₁-C₅ Alkanoyl;
mit R₆ gleich
(a) Wasserstoff,
(b) C₁-C₅ Alkyl,
(c) -(CH₂)ₚ-Aryl oder
(d) -(CH₂)ₚ-Het.

2. Peptid nach Anspruch 1 der Formel I
X₁-B₇-C₈-D₉-E₁₀ I
worin X₁ die in Anspruch 1 angegebene Bedeutung besitzt und E₁₀ der Formel XL₈ gemäß Anspruch 1 entspricht, B₇ abwesend ist oder für eine zweiwertige Einheit der Formel XL_{b} 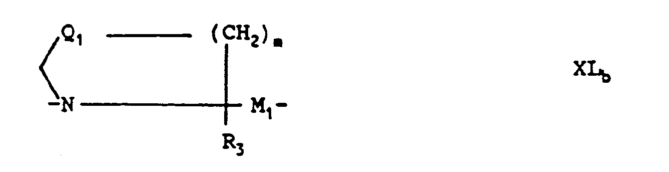 steht, worin C₈ abwesend ist oder für eine zweiwertige Einheit der Formel XL₁, XL₂ oder XL₃ 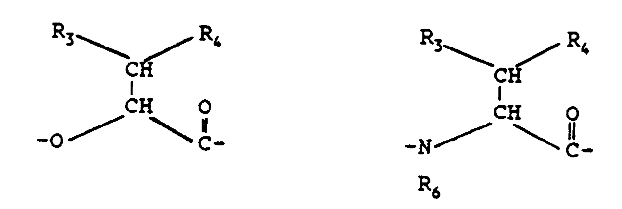 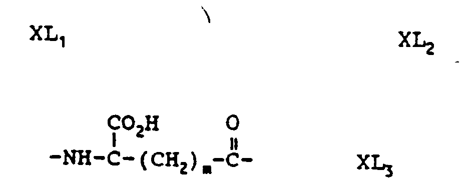 steht oder worin C₈ für eine einwertige Einheit der Formel XL₁ₐ oder XL₂ₐ steht, wenn X und B₇ fehlen 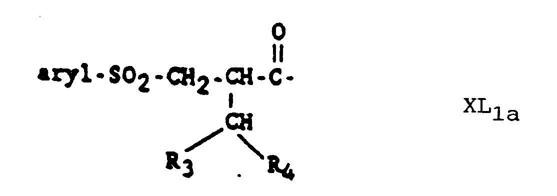 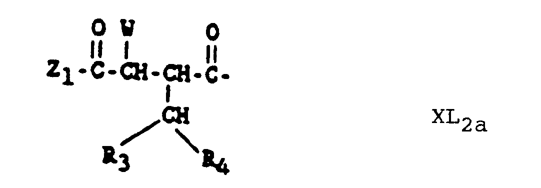 worin D₉ fehlt oder eine zweiwertige Einheit der Formel XL₄ darstellt: 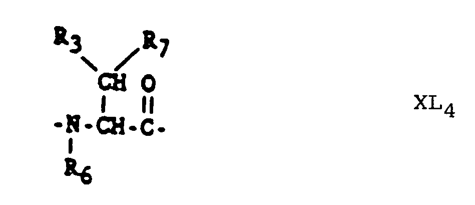 oder worin C₈-D₉ XL₇, oder bei Fehlen von X und B₇, XL₇ₐ ist, 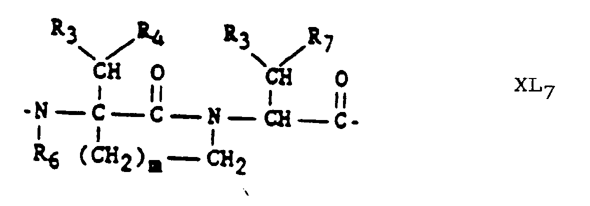 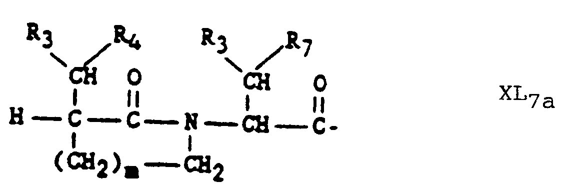 worin Q =
(a) -CH₂-,
(b) -CH(OH)-,
(c) -O- oder
(d) -S-;
worin M für -C(O)- oder -CH₂- steht;
worin m = 1 oder 2;
worin n = 0 bis einschließlich 5;
worin p = 0 bis einschließlich 2;
worin W = H oder OH;
worin Z₁, R₃ und R₆ die in Anspruch 1 angegebene Bedeutung besitzen,
worin R₄ =
(a) Wasserstoff,
(b) C₁-C₁₀ Alkyl,
(c) -(CH₂)ₚ-Aryl,
(d) -(CH₂)ₚ-Het oder
(e) -(CH₂)ₚ-C₃-C₇ Cycloalkyl;
worin R₅ =
(a) Wasserstoff,
(b) C₁-C₁₀ Alkyl,
(c) Hydroxy,
(d) Amino C₁-C₄ alkyl,
(e) Guanidinyl C₁-C₄ alkyl,
(f) Aryl,
(g) Het,
(h) Methylthio,
(i) -(CH₂)ₚ-C₃-C₇ Cycloalkyl oder
(j) Amino;
worin R₇ =
(a) Wasserstoff,
(b) C₁-C₁₀ Alkyl,
(c) Hydroxy,
(d) Amino C₁-C₄ alkyl,
(e) Guanidinyl C₁-C₄ alkyl,
(f) Aryl,
(g) Het,
(h) Methylthio,
(i) -(CH₂)ₚ-C₃-C₇ Cycloalkyl oder
(j) Amino,
worin Aryl und Het die in Anspruch 1 angegebene Bedeutung besitzen, oder eine Carboxy-, Amino- oder sonstige reaktivgruppengeschützte Form hiervon; oder ein pharmazeutisch akzeptables Säure- oder Baseadditionssalz hiervon.

3. Peptid nach Anspruch 2, worin X₁ am N-Terminus für tert.- Butyloxycarbonyl, β-Valyl, τ-Glutamyl oder 2-Desoxyglucopyranose-2-aminocarbonyl steht, B₇ fehlt oder Pro oder ein Derivat desselben ist, C₈ für Phe, OMe-Tyr oder ein Derivat derselben steht, D₉ His, N(Me)His oder ein Derivat derselben darstellt und E 1-Cyclohexyl-2S,6-diamino-3R,4S-dihydroxy-6-methylheptan bedeutet; oder ein pharmazeutisch akzeptables Salz desselben.

4. Peptid nach Anspruch 3, ausgewählt aus
L-Histidinamid,N-[(1,1-dimethylethoxy)carbonyl]-L-phenylalanyl-N-[5-amino-1-(cyclohexylmethyl)-2,3-dihydroxy-5-methylhexyl]-, oder N-tert.-Butyloxycarbonyl-L-phenylalanyl-L-histidyl-2S,6-diamino-1-cyclohexyl-3R,4R-dihydroxy-6-methylheptan;
L-Histidinamid,N-[(1,1-dimethylethoxy)carbonyl]-L-phenylalanyl-N-[5-amino-1-(cyclohexylmethyl)-2,3-dihydroxy-5-methylhexyl]-, oder N-tert.-Butyloxycarbonyl-L-phenylalanyl-L-histidyl-2S,6-diamino-1-cyclohexyl-3R,4S-dihydroxy-6-methylheptan;
L-Arabino-heptit, 6-amino-2-[[N-[N-(3-amino-3-methyl-1-oxobutyl)-L-phenylalanyl]-L-histidyl]amino]-1-cyclohexyl-1,2,5,6,7-pentadeoxy-6-C-methyl, tris(trifluoracetat) (Salz) oder βVal-Phe-His-X;
L-Arabino-heptit, 6-amino-2-[[N-[N-(3-amino-3-methyl-1-oxobutyl)-L-phenylalanyl]-N-methyl-L-histidyl]amino]1,2,5,6,7-pentadeoxy-1-cyclohexyl-6-methyl-, tris(trifluoracetat) (Salz) oder βVal-Phe-N-Me-His-X;
L-Arabino-hepit, 6-amino-1,2,5,6,7-pentadeoxy-1-cyclohexyl-2-[[N-[N-(1-L-γglutamyl-L-prolyl)-L-phenylalanyl]-N-methyl-L-histidyl]amino]-6-methyl-, tris(trifluoracetat) (Salz) oder
γGlu-Pro-Phe-N-Me-His-X;
L-Arabino-heptit, 6-amino-1-cyclohexyl-1,2,5,6,7-pentadeoxy-2-[[N-[N-[(1,1-dimethylethoxy)carbonyl]-0-methyl-L-tyrosyl]-L-histidyl]amino]-6-methyl-, oder Boc-O-MeTyr-His-X;
L-Arabino-heptit,6-amino-1,2,5,6,7-pentadeoxy-1-cyclohexyl-2-[[N-[N-(1-L-γ-glutamyl-L-prolyl)-O-methyl-L-tyrosyl]-L-histidyl]-amino]-6-methyl-,
tris(trifluoracetat) Salz oder γGlu-Pro-O-MeTyr-His-X; und
L-Arabino-heptit, 6-amino-1,2,5,6,7-Pentadeoxy-1-cyclohexyl-2-[[N-[N-(2-deoxy-glucopyranose-2-aminocarbonyl-L-prolyl)-O-methyl-L-tyrosyl]-L-histidyl]-amino]-6-methyl-, tris(trifluoracetat) Salz oder Glc-Pro-O-MeTyr-His-X.

## Patentansprüche (Patentansprüche für folgende(n) Vertragsstaat(en): ES)

1. Verfahren zur Herstellung eines Peptids mit einem nichtspaltbaren Übergangszustandinsert entsprechend der 10,11-Stellung eines Reninsubstrats (Angiotensinogen) der Formel XL₈ 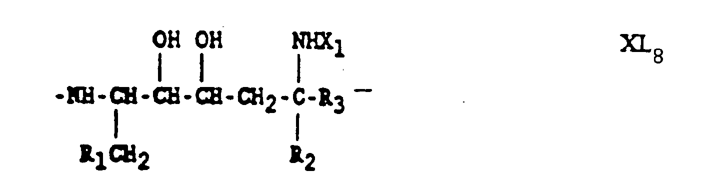 worin bedeuten:
(a) Wasserstoff,
(b) Y₁-(CH₂)ₐ-C(O)-,
(c) (HOCH₂)₃C-NH-C(O)-,
(d) (HOCH₂)₂CH-NH-C(O)-,
(e) 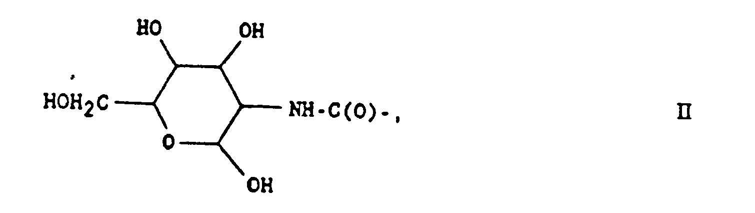
(f) HOH₂C(CHOH)ₐCH(CH₂OH)-NH-C(O)-,
(g) NaO₃S(CH₂)₂N(CH₃)C(O)(CH₂)₆C(O)-,
(h) R₂C(O)-,
(i) R₂-O-C(O)- oder
(j) Z₁-C(O)-;
mit Y₁ gleich
(a) -NH₂,
(b) -CO₂H,
(c) -C(NH₂)(CH₃)₂,
(d) -CH(NH₂)(CO₂H), oder
(e) -OP(O)(OH)₂;
Z₁ gleich über einen Stickstoff gebundenes -Het,
R₁
(a) Wasserstoff,
(b) C₁-C₈ Alkyl,
(c) Aryl,
(d) C₃-C₇ Cycloalkyl,
(e) Het,
(f) C₁-C₃ Alkoxy oder
(g) C₁-C₃ Alkylthio;
R₂
(a) C₁-C₁₀ Alkyl,
(b) C₃-C₇ Cycloalkyl,
(c) Aryl,
(d) Het,
(e) Aryl C₁-C₅ alkyl oder
(f) Het C₁-C₅ alkyl;
R₃
(a) C₁-C₁₀ Alkyl,
(b) -(CH₂)ₚ-Aryl,
(c) -(CH₂)ₚ-Het,
(d) -(CH₂)ₚ-C₃-C₇ Cycloalkyl oder
(e) -1 oder 2-Adamantyl;
n = 0 bis einschließlich 5;
p = 0 bis einschließlich 2
mit Aryl gleich Phenyl oder Naphthyl, substituiert durch 0 bis 3 der folgenden Substituenten:
(a) C₁-C₃ Alkyl,
(b) Hydroxy,
(c) C₁-C₃ Alkoxy,
(d) Halogen,
(e) Amino,
(f) mono- oder di-C₁-C₃ Alkylamino,
(g) -CHO,
(h) -COOH,
(i) -CONH₂,
(j) -CONH(C₁-C₅ Alkyl)
(k) -Nitro,
(l) Mercapto,
(m) C₁-C₃ Alkylthio
(n) C₁-C₃ Alkylsulfinyl,
(o) C₁-C₃ Alkylsulfonyl,
(p) -N(R₆)-C₁-C₃ Alkylsulfonyl,
(q) SO₃H,
(r) SO₂NH₂,
(s) -CN oder
(t) -CH₂NH₂;
mit -Het gleich einem 5- oder 6-gliedrigen gesättigten oder ungesättigten Ring mit 1 bis 3 Heteroatom(en) aus der Gruppe Stickstoff, Sauerstoff und Schwefel, einschließlich irgendwelcher bicyclischer Gruppen, bei denen einer der genannten heterocyclischen Ringe an einen Benzolring ankondensiert ist, wobei die heterocyclische Einheit mit 0 bis 3 der folgenden Substituenten substituiert ist:
(i) (C₁-C₆ Alkyl,
(ii) Hydroxy,
(iii) Trifluormethyl,
(iv) C₁-C₄ Alkoxy,
(v) Halogen,
(vi) Aryl,
(vii) Aryl C₁-C₄ alkyl-,
(viii) Amino,
(ix) mono- oder di-(C₁-C₄ Alkyl) amino, oder
(x) C₁-C₅ Alkanoyl;
mit R₆ gleich
(a) Wasserstoff,
(b) C₁-C₅ Alkyl,
(c) -(CH₂)ₚ-Aryl oder
(d) -(CH₂)ₚ-Het,
durch sukzessives Umsetzen der geeigneten Acylderivate von Aminosäureresten oder deren Analogen einzeln oder als Einheiten aus zwei oder mehreren Resten bis zum Erhalt des Peptids.

2. Verfahren nach Anspruch 1, wobei das Peptid der Formel I
X₁-B₇-C₈-D₉-E₁₀ I
entspricht,
worin X₁ die in Anspruch 1 angegebene Bedeutung besitzt und E₁₀ der Formel XL₈ gemäß Anspruch 1 entspricht,
B₇ abwesend ist oder für eine zweiwertige Einheit der Formel XL_{b} 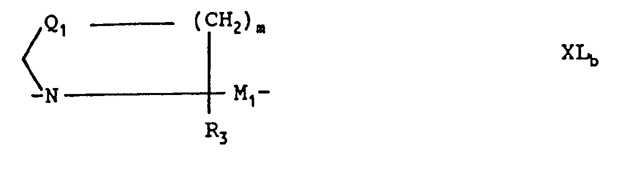 steht, worin C₈ abwesend ist oder für eine zweiwertige Einheit der Formel XL₁, XL₂ oder XL₃ 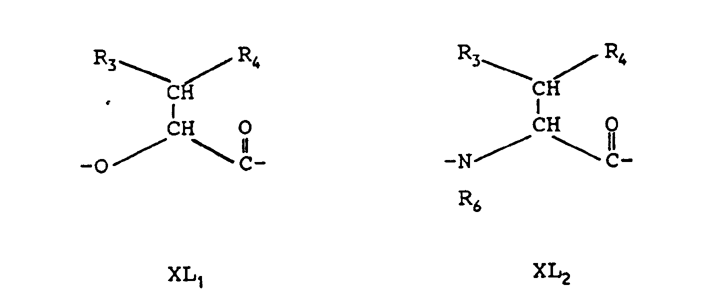 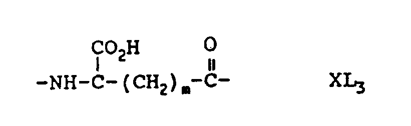 steht oder worin C₈ für eine einwertige Einheit der Formel XL₁ₐ oder XL₂ₐ steht, wenn X und B7 fehlen 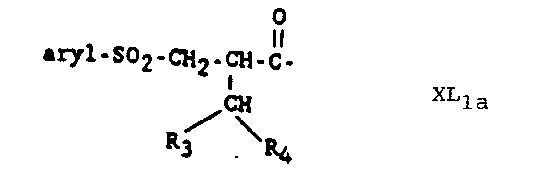 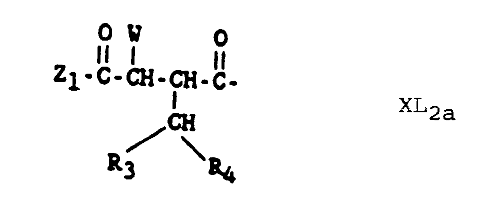 worin D₉ fehlt oder eine zweiwertige Einheit der Formel XL₄ darstellt: 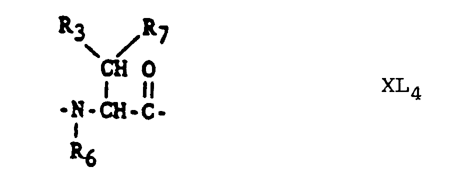 oder worin C₈-D₉ XL₇, oder bei Fehlen von X und B₇, XL₇ₐ ist, 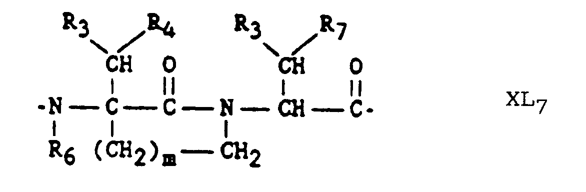 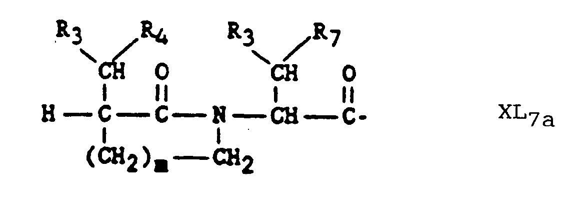 worin Q =
(a) -CH₂-,
(b) -CH(OH)-,
(c) -O- oder
(d) -S-;
worin M für -C(O)- oder -CH₂- steht;
worin m = 1 oder 2;
worin n = 0 bis einschließlich 5;
worin p = 0 bis einschließlich 2;
worin W = H oder OH;
worin Z₁, R₃ und R₆ die in Anspruch 1 angegebene Bedeutung besitzen,
worin R₄ =
(a) Wasserstoff,
(b) C₁-C₁₀ Alkyl,
(c) -(CH₂)ₚ-Aryl,
(d) -(CH₂)ₚ-Het oder
(e) -(CH₂)ₚ-C₃-C₇ Cycloalkyl;
worin R₅ =
(a) Wasserstoff,
(b) C₁-C₁₀ Alkyl,
(c) Hydroxy,
(d) Amino C₁-C₄ alkyl,
(e) Guanidinyl C₁-C₄ alkyl,
(f) Aryl,
(g) Het,
(h) Methylthio,
(i) -(CH₂)ₚ-C₃-C₇ Cycloalkyl oder
(j) Amino;
worin R₇ =
(a) Wasserstoff,
(b) C₁-C₁₀ Alkyl,
(c) Hydroxy,
(d) Amino C₁-C₄ alkyl,
(e) Guanidinyl C₁-C₄ alkyl,
(f) Aryl,
(g) Het,
(h) Methylthio,
(i) -(CH₂)ₚ-C₃-C₇ Cycloalkyl oder
(j) Amino,
worin Aryl und Het die in Anspruch 1 angegebene Bedeutung besitzen, oder eine Carboxy-, Amino- oder sonstige reaktivgruppengeschützte Form hiervon; oder ein pharmazeutisch akzeptables Säure- oder Baseadditionssalz hiervon.

3. Verfahren nach Anspruch 2, wobei in dem Peptid X₁ am N-Terminus für tert.-Butyloxycarbonyl, β-Valyl, τ-Glutamyl oder 2-Desoxyglucopyranose-2-aminocarbonyl steht, B₇ fehlt oder Pro oder ein Derivat desselben ist, C₈ für Phe, OMe-Tyr oder ein Derivat derselben steht, D₉ His, N(Me)His oder ein Derivat derselben darstellt und E 1-Cyclohexyl-2S,6-diamino-3R,4S-dihydroxy-6-methylheptan bedeutet;
oder ein pharmazeutisch akzeptables Salz desselben.

4. Verfahren nach Anspruch 3, wobei das Peptid ausgewählt ist aus:
L-Histidinamid,N-[(1,1-dimethylethoxy)carbonyl]-L-phenylalanyl-N-[5-amino-1-(cyclohexylmethyl)-2,3-dihydroxy-5-methylhexyl]-, oder N-tert.-Butyloxycarbonyl-L-phenylalanyl-L-histidyl-2S,6-diamino-1-cyclohexyl-3R,4R-dihydroxy-6-methylheptan;
L-Histidinamid,N-[(1,1-dimethylethoxy)carbonyl]-L-phenylalanyl-N-[5-amino-1-(cyclohexylmethyl)-2,3-dihydroxy-5-methylhexyl]-, oder N-tert.-Butyloxycarbonyl-L-phenylalanyl-L-histidyl-2S,6-diamino-1-cyclohexyl-3R,4S-dihydroxy-6-methylheptan;
L-Arabino-heptit, 6-amino-2-[[N-[N-(3-amino-3-methyl-1-oxobutyl)-L-phenylalanyl]-L-histidyl]amino]-1-cyclohexyl-1,2,5,6,7-pentadeoxy-6-C-methyl, tris(trifluoracetat) (Salz) oder βVal-Phe-His-X;
L-Arabino-heptit, 6-amino-2-[[N-[N-(3-amino-3-methyl-1-oxobutyl)-L-phenylalanyl]-N-methyl-L-histidyl]amino]1,2,5,6,7-pentadeoxy-1-cyclohexyl-6-methyl-, tris(trifluoracetat) (Salz) oder βVal-Phe-N-Me-His-X;
L-Arabino-hepit, 6-amino-1,2,5,6,7-pentadeoxy-1-cyclohexyl-2-[[N-[N-(1-L-γglutamyl-L-prolyl)-L-phenylalanyl]-N-methyl-L-histidyl]amino]-6-methyl-, tris(trifluoracetat) (Salz) oder
γGlu-Pro-Phe-N-Me-His-X;
L-Arabino-heptit, 6-amino-1-cyclohexyl-1,2,5,6,7-pentadeoxy-2-[[N-[N-[(1,1-dimethylethoxy)carbonyl]-0-methyl-L-tyrosyl]-L-histidyl]amino]-6-methyl-, oder Boc-O-MeTyr-His-X;
L-Arabino-heptit,6-amino-1,2,5,6,7-pentadeoxy-1-cyclohexyl-2-[[N-[N-(1-L-γ-glutamyl-L-prolyl)-O-methyl-L-tyrosyl]-L-histidyl]-amino]-6-methyl-,
tris(trifluoracetat) Salz oder γGlu-Pro-O-MeTyr-His-X; und
L-Arabino-heptit, 6-amino-1,2,5,6,7-Pentadeoxy-1-cyclohexyl-2-[[N-(N-(2-deoxy-glucopyranose-2-aminocarbonyl-L-prolyl)-O-methyl-L-tyrosyl]-L-histidyl]-amino]-6-methyl-, tris(trifluoracetat) Salz oder Glc-Pro-O-MeTyr-His-X.

## Revendications (Revendications pour l'(les) Etat(s) contractant(s) suivant(s): AT, BE, CH, DE, DK, FR, GB, IT, LI, LU, NL, SE)

1. Peptide possédant un groupement d'insertion d'état de transition non clivable correspondant à la position 10,11 d'un substrat de rénine (angiotensinogène), de formule XL₈ 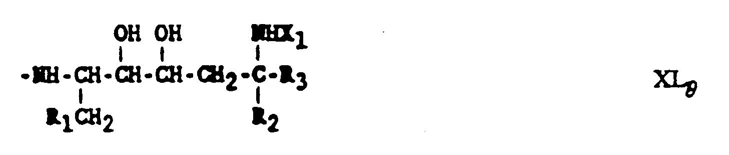 dans laquelle X₁ représente
(a) l'hydrogène,
(b) un groupe Y₁-(CH₂)ₐ-C(O)-,
(c) un groupe (HOCH₂)₃C-NH-C(O)-,
(d) un groupe (HOCH₂)₂CH-NH-C(O)-,
(e) 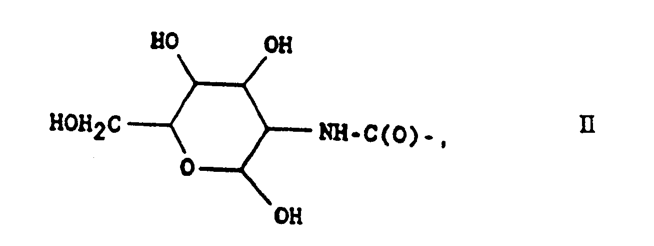
(f) un groupe HOH₂C(CHOH)ₐCH(CH₂OH)-NH-C(O)-,
(g) un groupe NaO₃S(CH₂)₂N(CH₃)C(O)(CH₂)₆C(O)-,
(h) un groupe R₂C(O)-,
(i) un groupe R₂-O-C(O)-, ou
(j) un groupe Z₁-C(O)- ;
Y₁ représente
(a) un groupe -NH₂-,
(b) un groupe -CO₂H,
(c) un groupe -C(NH₂)(CH₃)₂,
(d) un groupe -CH(NH₂)(CO₂H), ou
(e) un groupe -OP(O)(OH)₂ ;
Z₁ représente un groupe -Het lié par l'intermédiaire d'un atome d'azote ;
R₁ représente
(a) l'hydrogène,
(b) un groupe alkyle en C₁ à C₈,
(c) un groupe aryle,
(d) un groupe cycloalkyle en C₃ à C₇,
(e) un groupe Het,
(f) un groupe alkoxy en C₁ à C₃, ou
(g) un groupe alkylthio en C₁ à C₃ ;
R₂ représente
(a) un groupe alkyle en C₁ à C₁₀,
(b) un groupe cycloalkyle en C₃ à C₇,
(c) un groupe aryle,
(d) un groupe Het,
(e) un groupe aryl-(alkyle en C₁ à C₅), ou
(f) un groupe Het-(alkyle en C₁ à C₅) ;
R₃ représente
(a) un groupe alkyle en C₁ à C₁₀,
(b) un groupe -(CH₂)ₚ-aryle,
(c) un groupe -(CH₂)ₚ-Het,
(d) un groupe -(CH₂)ₚ-(cycloalkyle en C₃ à C₇),
ou
(e) un groupe -1- ou 2-adamantyle ;
n a une valeur de 0 à 5 inclus ;
p a une valeur de 0 à 2 inclus ;
le groupe aryle est un groupe phényle ou naphtyle portant 0 à 3 des substituants suivants :
(a) alkyle en C₁ à C₃,
(b) hydroxy,
(c) alkoxy en C₁ à C₃,
(d) halogéno,
(e) amino,
(f) mono- ou di-(alkyle en C₁ à C₃)amino,
(g) -CHO,
(h) -COOH,
(i) -CONH₂,
(j) -CONH(alkyle en C₁ à C₅),
(k) -nitro,
(l) mercapto,
(m) alkylthio en C₁ à C₃,
(n) alkylsulfinyle en C₁ à C₃,
(o) alkylsulfonyle en C₁ à C₃,
(p) -N(R₆)-(alkylsulfonyle en C₁ à C₃),
(q) SO₃H,
(r) SO₂NH₂,
(s) -CN, ou
(t) -CH₂NH₂ ;
-Het représente un noyau penta- ou hexagonal saturé ou insaturé contenant 1 à 3 hétéroatomes choisis dans le groupe consistant en azote, oxygène et soufre ; et comprenant n'importe quel groupe bicyclique dans lequel l'un quelconque des noyaux hétérocycliques précités est condensé à un noyau benzénique, groupement hétérocyclique qui porte 0 à 3 des substituants suivants :
(i) alkyle en C₁ à C₆,
(ii) hydroxy,
(iii) trifluorométhyle,
(iv) alkoxy en C₁ à C₄,
(v) halogéno,
(vi) aryle,
(vii) aryl-(alkyle en C₁ à C₄),
(viii) amino,
(ix) mono- ou di-(alkyle en C₁ à C₄)amino, ou
(x) alcanoyle en C₁ à C₅ ;
R₆ représente
(a) l'hydrogène,
(b) un groupe alkyle en C₁ à C₅,
(c) un groupe -(CH₂)ₚ-aryle, ou
(d) un groupe -(CH₂)ₚ-Het.

2. Peptide suivant la revendication 1, répondant à la formule I
X₁-B₇-C₈-D₉-E₁₀ (I)
dans laquelle X₁ répond à la définition suivant la revendication 1 et E₁₀ répond à la formule XL₈ telle qu'elle est définie dans la revendication 1 ;
B₇ est absent ou représente un groupement divalent de formule XL_{b} 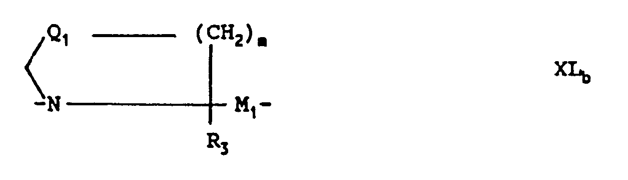 C₈ est absent ou représente un groupement divalent de formule XL₁, XL₂ ou XL₃ ; 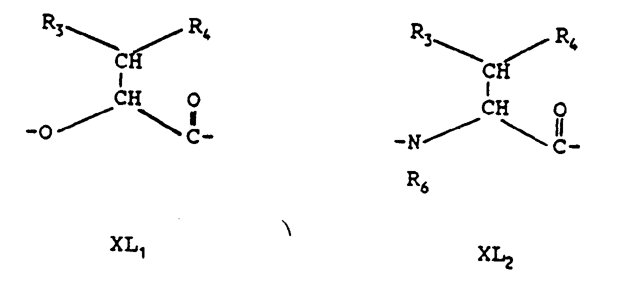 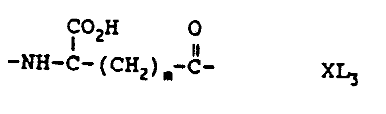 ou bien C₈ représente un groupement monovalent de formule XL₁ₐ ou XL₂ₐ lorsque X et B₇ sont absents. 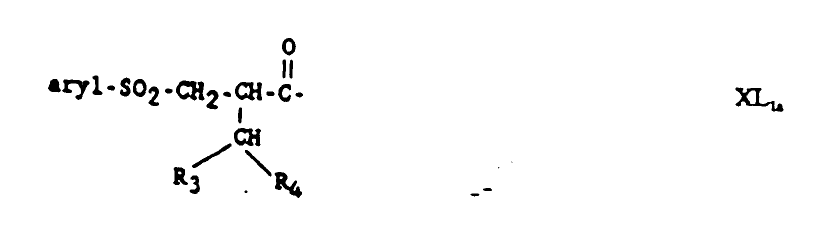 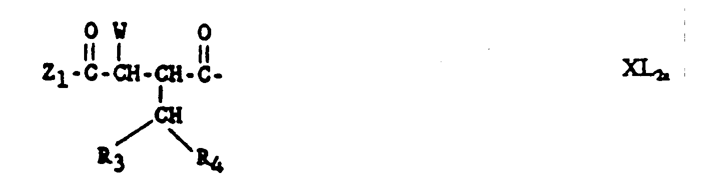 D₉ est absent ou représente un groupement divalent de formule XL₄ 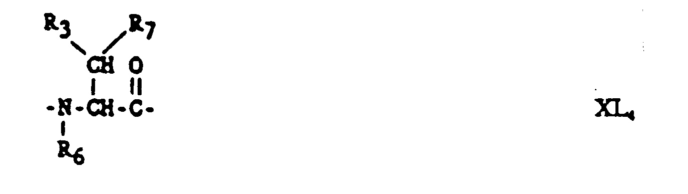 ou bien C₈-D₉ représente un groupe XL₇ ou, lorsque X₉ et B₇ sont absents, représente un groupe XL₇ₐ : 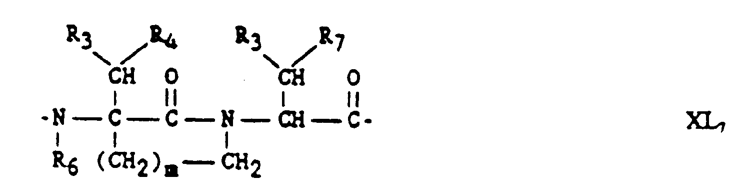 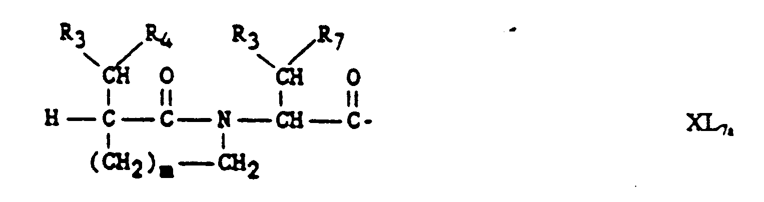 Q représente
(a) un groupe -CH₂-,
(b) un groupe -CH(OH)-,
(c) un groupe -O-, ou
(d) un groupe -S- ;
M représente un groupe -C(O)- ou -CH₂- ;
m est égal à 1 ou 2 ;
n a une valeur de 0 à 5 inclus ;
p a une valeur de 0 à 2 inclus ;
W représente H ou un groupe OH ;
Z₁, R₃ et R₆ répondent aux définitions suivant la revendication 1 ;
R₄ représente
(a) l'hydrogène,
(b) un groupe alkyle en C₁ à C₁₀,
(c) un groupe -(CH₂)ₚ-aryle,
(d) un groupe -(CH₂)ₚ-Het, ou
(e) un groupe -(CH₂)ₚ-(cycloalkyle en C₃ à C₇) ;
R₅ représente
(a) l'hydrogène,
(b) un groupe alkyle en C₁ à C₁₀,
(c) un groupe hydroxy,
(d) un groupe amino-(alkyle en C₁ à C₄),
(e) un groupe guanidinyl-(alkyle en C₁ à C₄),
(f) un groupe aryle,
(g) un groupe Het,
(h) un groupe méthylthio,
(i) un groupe -(CH₂)ₚ-(cycloalkyle en C₃ à C₇),
ou
(j) un groupe amino ;
R₇ représente
(a) l'hydrogène,
(b) un groupe alkyle en C₁ à C₁₀,
(c) un groupe hydroxy,
(d) un groupe amino-(alkyle en C₁ à C₄),
(e) un groupe guanidinyl-(alkyle en C₁ à C₄),
(f) un groupe aryle,
(g) un groupe Het,
(h) un groupe méthylthio,
(i) un groupe -(CH₂)ₚ-(cycloalkyle en C₃ à C₇),
ou
(j) un groupe amino,
les groupes aryle et Het répondant aux définitions suivant la revendication 1,
ou une forme de ce peptide protégée au niveau des groupes carboxy, amino ou d'autres groupes réactifs ;
ou un de ses sels d'addition d'acides ou de bases pharmaceutiquement acceptables.

3. Peptide suivant la revendication 2, dans lequel X₁ à l'extrémité N-terminale représente un groupe t-butyloxycarbonyle, β-valyle, γ-glutamyle ou 2-désoxyglucopyrannose-2-aminocarbonyle ;
B₇ est absent ou représente un groupe Pro ou un de ses dérivés ;
C₈ représente un groupe Phe, OMe-Tyr ou un de ses dérivés ;
D₉ représente un groupe His, N(Me)His ou un de ses dérivés ;
E représente un groupe 1-cyclohexyl-2S,6-diamino-3R,4S-dihydroxy-6-méthylheptane ;
ou un de ses sels pharmaceutiquement acceptables.

4. Peptide suivant la revendication 3, choisi entre
le N-[(1,1-diméthyléthoxy)carbonyl]-L-phénylalanyl-N-[5-amino-1-(cyclohexylméthyl)-2,3-dihydroxy-5-méthylhexyl-L-histidinamide, ou le N-tert-butyloxycarbonyl-L-phénylalanyl-L-histidyl-2S,6-diamino-1-cyclohexyl-3R,4R-dihydroxy-6-méthylheptane ;
le N-[(1,1-diméthyléthoxy)carbonyl]-L-phénylalanyl-N-[5-amino-1-(cyclohexylméthyl)-2,3-dihydroxy-5-méthylhexyl]-L-histidinamide, ou le N-tert-butyloxycarbonyll-L-phénylalanyl-L-histidyl-2S,6-diamino-1-cyclohexyl-3R,4S-dihydroxy-6-méthylheptane ;
le 6-amino-2-[[N-[N-(3-amino-3-méthyl-1-oxobutyl)-L-phénylalanyl]-L-histidyl]amino]-1-cyclohexyl-1,2,5,6,7-pentadésoxy-6-C-méthyl-L-arabino-heptitol, tris-(trifluoracétate) (sel), ou βVal-Phe-His-X ;
le 6-amino-2-[[N-[N-(3-amino-3-méthyl-1-oxobutyl)-L-phénylalanyl]-N-méthyl-L-histidyl]amino]1,2,5,6,7-pentadésoxy-1-cyclohexyl-6-méthyl-L-arabino-heptitol, tris(trifluoracétate) (sel), ou βVal-Phe-N-Me-His-X ;
le 6-amino-1,2,5,6,7-pentadésoxy-1-cyclohexyl-2-[[N-[N-(1-L-γglutamyl-L-prolyl)-L-phénylalanyl]-N-méthyl-L-histidyl]amino]-6-méthyl-L-arabino-heptitol, tris(trifluoracétate) (sel) ou γGlu-Pro-Phe-N-Me-His-X ;
le 6-amino-1-cyclohexyl-1,2,5,6,7-pentadésoxy-2-[[N-[N-[(1,1-diméthyléthoxy)carbonyl]-O-méthyl-L-tyrosyl]-L-histidyl]amino]-6-méthyl-L-arabino-heptitol, ou Boc-O-MeTyr-His-X ;
le 6-amino-1,2,5,6,7-pentadésoxy-1-cyclohexyl-2-[[N-[N-(1-L-γglutamyl-L-prolyl)-O-méthyl-L-tyrosyl]-L-histidyl]-amino]-6-méthyl-L-arabino-heptitol,tris(trifluoracétate) (sel) ou yGlu-Pro-O-MeTyr-His-X ; et
le 6-amino-1,2,5,6,7-pentadésoxy-1-cyclohexyl-2-[[N-[N-(2-désoxy-glucopyrannose-2-aminocarbonyl-L-prolyl)-O-méthyl-L-tyrozyl]-L-histidyl]-amino]-6-méthyl-L-arabinoheptitol, tris(trifluoracétate) (sel) ou Glc-Pro-O-MeTyr-His-X.

## Revendications (Revendications pour l'(les) Etat(s) contractant(s) suivant(s): ES)

1. Procédé de préparation d'un peptide possédant un groupement d'insertion d'état de transition non clivable correspondant à la position 10,11 d'un substrat de rénine (angiotensinogène), répondant à la formule XL₈ 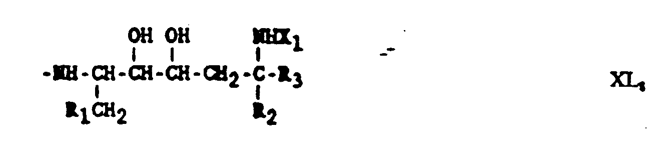 dans laquelle X₁ représente
(a) l'hydrogène,
(b) un groupe Y₁-(CH₂)ₐ-C(O)-,
(c) un groupe (HOCH₂)₃C-NH-C(O)-,
(d) un groupe (HOCH₂)₂CH-NH-C(O)-,
(e) 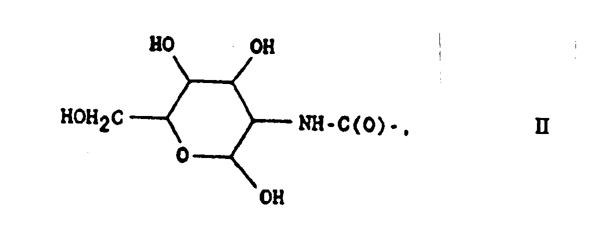
(f) un groupe HOH₂C(CHOH)ₐCH(CH₂OH)-NH-C(O)-,
(g) un groupe NaO₃S(CH₂)₂N(CH₃)C(O)(CH₂)₆C(O)-,
(h) un groupe R₂C(O)-,
(i) un groupe R₂-O-C(O)-, ou
(j) un groupe Z₁-C(O)- ;
Y₁ représente
(a) un groupe -NH₂-,
(b) un groupe -CO₂H,
(c) un groupe -C(NH₂)(CH₃)₂,
(d) un groupe -CH(NH₂)(CO₂H), ou
(e) un groupe -OP(O)(OH)₂ ;
Z₁ représente un groupe -Het lié par l'intermédiaire d'un atome d'azote ;
R₁ représente
(a) l'hydrogène,
(b) un groupe alkyle en C₁ à C₈,
(c) un groupe aryle,
(d) un groupe cycloalkyle en C₃ à C₇,
(e) un groupe Het,
(f) un groupe alkoxy en C₁ à C₃, ou
(g) un groupe alkylthio en C₁ à C₃ ;
R₂ représente
(a) un groupe alkyle en C₁ à C₁₀,
(b) un groupe cycloalkyle en C₃ à C₇,
(c) un groupe aryle,
(d) un groupe Het,
(e) un groupe aryl-(alkyle en C₁ à C₅), ou
(f) un groupe Het-(alkyle en C₁ à C₅) ;
R₃ représente
(a) un groupe alkyle en C₁ à C₁₀,
(b) un groupe -(CH₂)ₚ-aryle,
(c) un groupe -(CH₂)ₚ-Het,
(d) un groupe -(CH₂)ₚ-(cycloalkyle en C₃ à C₇),
ou
(e) un groupe -1- ou 2-adamantyle ;
n a une valeur de 0 à 5 inclus ;
p a une valeur de 0 à 2 inclus ;
le groupe aryle est un groupe phényle ou naphtyle portant 0 à 3 des substituants suivants :
(a) alkyle en C₁ à C₃,
(b) hydroxy,
(c) alkoxy en C₁ à C₃,
(d) halogéno,
(e) amino,
(f) mono- ou di-(alkyle en C₁ à C₃)amino,
(g) -CHO,
(h) -COOH,
(i) -CONH₂,
(j) -CONH(alkyle en C₁ à C₅),
(k) -nitro,
(l) mercapto,
(m) alkylthio en C₁ à C₃,
(n) alkylsulfinyle en C₁ à C₃,
(o) alkylsulfonyle en C₁ à C₃,
(p) -N(R₆)-(alkylsulfonyle en C₁ à C₃),
(q) SO₃H,
(r) SO₂NH₂,
(s) -CN, ou
(t) -CH₂NH₂ ;
-Het représente un noyau penta- ou hexagonal saturé ou insaturé contenant 1 à 3 hétéroatomes choisis dans le groupe consistant en azote, oxygène et soufre ; et comprenant n'importe quel groupe bicyclique dans lequel l'un quelconque des noyaux hétérocycliques précités est condensé à un noyau benzénique, groupement hétérocyclique qui porte 0 à 3 des substituants suivants :
(i) alkyle en C₁ à C₆,
(ii) hydroxy,
(iii) trifluorométhyle,
(iv) alkoxy en C₁ à C₄,
(v) halogéno,
(vi) aryle,
(vii) aryl-(alkyle en C₁ à C₄),
(viii) amino,
(ix) mono- ou di-(alkyle en C₁ à C₄)amino, ou
(x) alcanoyle en C₁ à C₅ ;
R₆ représente
(a) l'hydrogène,
(b) un groupe alkyle en C₁ à C₅,
(c) un groupe -(CH₂)ₚ-aryle, ou
(d) un groupe -(CH₂)ₚ-Het,
qui consiste à faire réagir successivement les dérivés acylés appropriés de résidus d'aminoacides ou de leurs analogues, seuls ou sous forme de motifs de deux ou plus de deux résidus, jusqu'à ce que ledit peptide soit préparé.

2. Procédé suivant la revendication 1, dans lequel le peptide répond à la formule I
X₁-B₇-C₈-D₉-E₁₀ I
dans laquelle X₁ répond à la définition suivant la revendication 1 et E₁₀ répond à la formule XL₈ telle qu'elle est définie dans la revendication 1 ;
B₇ est absent ou bien représente un groupement divalent de formule XL_{b} 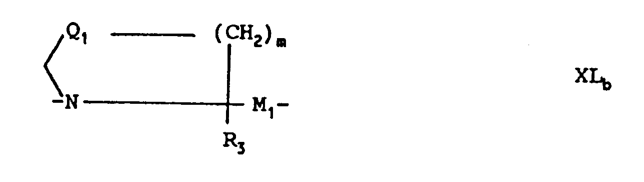 C₈ est absent ou bien représente un groupement divalent de formule XL₁, XL₂ ou XL₃ ; 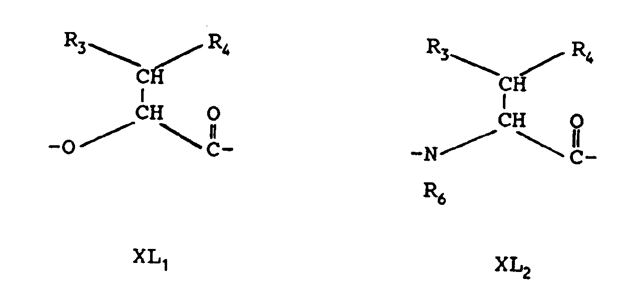 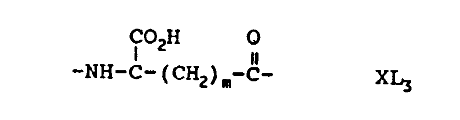 ou bien C₈ représente un groupement monovalent de formule XL₁ₐ ou XL₂ₐ lorsque X et B₇ sont absents 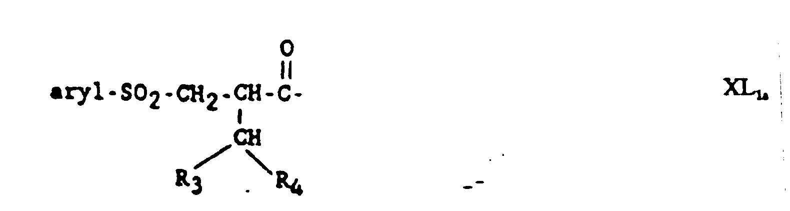 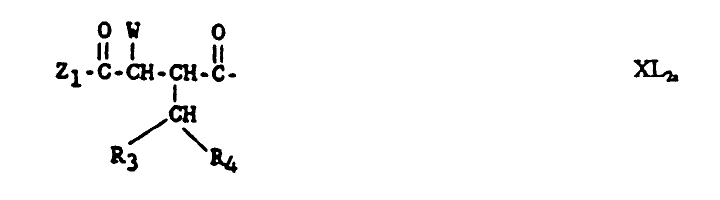 D₉ est absent ou représente un groupement divalent de formule XL₄ : 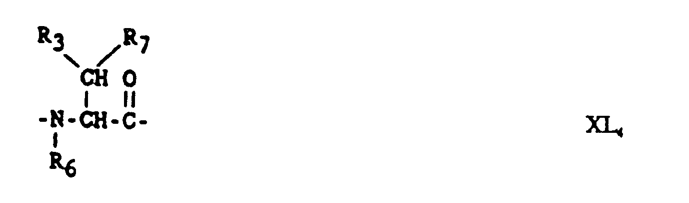 ou bien C₈-D₉ représente un groupe XL₇ ou, lorsque X et B₇ sont absents, représente un groupe XL₇ₐ 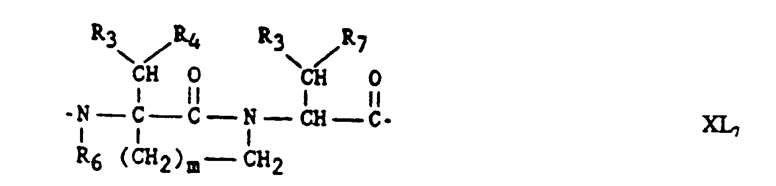 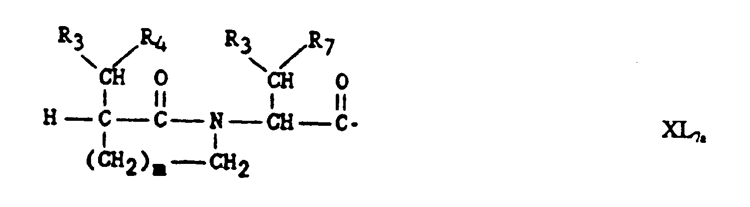 Q représente
(a) un groupe -CH₂-,
(b) un groupe -CH(OH)-,
(c) un groupe -O-, ou
(d) un groupe -S- ;
M représente un groupe -C(O)- ou -CH₂- ;
m est égal à 1 ou 2 ;
n a une valeur de 0 à 5 inclus ;
p a une valeur de 0 à 2 inclus ;
W représente H ou un groupe OH ;
Z₁, R₃ et R₆ répondent aux définitions suivant la revendication 1 ;
R₄ représente
(a) l'hydrogène,
(b) un groupe alkyle en C₁ à C₁₀,
(c) un groupe -(CH₂)ₚ-aryle,
(d) un groupe -(CH₂)ₚ-Het, ou
(e) un groupe -(CH₂)ₚ-(cycloalkyle en C₃ à C₇) ;
R₅ représente
(a) l'hydrogène,
(b) un groupe alkyle en C₁ à C₁₀,
(c) un groupe hydroxy,
(d) un groupe amino-(alkyle en C₁ à C₄),
(e) un groupe guanidinyl-(alkyle en C₁ à C₄),
(f) un groupe aryle,
(g) un groupe Het,
(h) un groupe méthylthio,
(i) un groupe -(CH₂)ₚ-(cycloalkyle en C₃ à C₇),
ou
(j) un groupe amino ;
R₇ représente
(a) l'hydrogène,
(b) un groupe alkyle en C₁ à C₁₀,
(c) un groupe hydroxy,
(d) un groupe amino-(alkyle en C₁ à C₄),
(e) un groupe guanidinyl-(alkyle en C₁ à C₄),
(f) un groupe aryle,
(g) un groupe Het,
(h) un groupe méthylthio,
(i) un groupe -(CH₂)ₚ-(cycloalkyle en C₃ à C₇),
ou
(j) un groupe amino,
les groupes aryle et Het répondant aux définitions suivant la revendication 1,
ou est une forme d'un tel peptide à groupes carboxy, amino ou d'autres groupes réactifs protégés ;
ou est un de ses sels d'addition d'acides ou de bases pharmaceutiquement acceptables.

3. Procédé suivant la revendication 2, dans lequel, dans le peptide,
X₁ à l'extrémité N-terminale représente un groupe t-butyloxycarbonyle, β-valyle, y-glutamyle ou 2-désoxyglucopyrannose-2-aminocarbonyle ;
B₇ est absent ou bien représente un groupe Pro ou un de ses dérivés ;
C₈ représente un groupe Phe, Ome-Tyr ou un de ses dérivés ;
D₉ représente un groupe His, N(Me)His ou un de ses dérivés ;
E représente un groupe 1-cyclohexyl-2S,6-diamino-3R,4S-dihydroxy-6-méthylheptane ;
ou un de ses sels pharmaceutiquement acceptables.

4. Procédé suivant la revendication 3, dans lequel le peptide est choisi entre
le N-[(1,1-diméthyléthoxy)carbonyl]-L-phénylalanyl-N-[5-amino-1-(cyclohexylméthyl)-2,3-dihydroxy-5-méthylhexyl-L-histidinamide, ou le N-tert-butyloxycarbonyl-L-phénylalanyl-L-histidyl-2S,6-diamino-1-cyclohexyl-3R,4R-dihydroxy-6-méthylheptane ;
le N-[(1,1-diméthyléthoxy)carbonyl]-L-phénylalanyl-N-[5-amino-1-(cyclohexylméthyl)-2,3-dihydroxy-5-méthylhexyl]-L-histidinamide, ou le N-tert-butyloxycarbonyll-L-phénylalanyl-L-histidyl-2S,6-diamino-1-cyclohexyl-3R,4S-dihydroxy-6-méthylheptane ;
le 6-amino-2-[[N-[N-(3-amino-3-méthyl-1-oxobutyl)-L-phénylalanyl]-L-histidyl]amino]-1-cyclohexyl-1,2,5,6,7-pentadésoxy-6-C-méthyl-L-arabino-heptitol, tris-(trifluoracétate) (sel), ou βVal-Phe-His-X ;
le 6-amino-2-[[N-[N-(3-amino-3-méthyl-1-oxobutyl)-L-phénylalanyl]-N-méthyl-L-histidyl]amino]1,2,5,6,7-pentadésoxy-1-cyclohexyl-6-méthyl-L-arabino-heptitol, tris(trifluoracétate) (sel), ou βVal-Phe-N-Me-His-X ;
le 6-amino-1,2,5,6,7-pentadésoxy-1-cyclohexyl-2-[[N-[N-(1-L-γglutamyl-L-prolyl)-L-phénylalanyl]-N-méthyl-L-histidyl]amino]-6-méthyl-L-arabino-heptitol, tris(trifluoracétate) (sel) ou γGlu-Pro-Phe-N-Me-His-X ;
le 6-amino-1-cyclohexyl-1,2,5,6,7-pentadésoxy-2-[[N-[N-[(1,1-diméthyléthoxy)carbonyl]-O-méthyl-L-tyrosyl]-L-histidyl]amino]-6-méthyl-L-arabino-heptitol, ou Boc-O-MeTyr-His-X ;
le 6-amino-1,2,5,6,7-pentadésoxy-1-cyclohexyl-2-[[N-[N-(1-L-γglutamyl-L-prolyl)-O-méthyl-L-tyrosyl]-L-histidyl]-amino]-6-méthyl-L-arabino-heptitol,tris(trifluoracétate) (sel) ou γGlu-Pro-O-MeTyr-His-X ; et
le 6-amino-1,2,5,6,7-pentadésoxy-1-cyclohexyl-2-[[N-[N-(2-désoxy-glucopyrannose-2-aminocarbonyl-L-prolyl)-O-méthyl-L-tyrozyl]-L-histidyl]-amino]-6-méthyl-L-arabino-heptitol, tris(trifluoracétate) (sel) ou Glc-Pro-O-MeTyr-His-X.
